# EUROPEAN PATENT SPECIFICATION

(11) **EP 4 562 003 B1**
(45) Date of publication and mention of the grant of the patent: **07.10.2026**
(21) Application number: 23748060.3
(22) Date of filing: 26.07.2023
(51) Int. Cl.: C07D 403/14, C07D 413/14, A61P 25/00, A61K 31/437

(54) **NOVEL 7-SUBSTITUTED INDOLE SULFONAMIDE DERIVATIVES**
NEUARTIGE 7-SUBSTITUIERTE INDOLSULFONAMIDDERIVATE
NOUVEAUX DÉRIVÉS D'INDOLE SULFONAMIDE 7-SUBSTITUÉS

(30) Priority: 28.07.2022 EP 22187363
(43) Date of publication of application: 04.06.2025
(73) Proprietor: F. Hoffmann-La Roche AG, 4070 Basel (CH)
(72) Inventor: GALLEY, Guido, 4070 Basel (CH); GOBBI, Luca Claudio, 4070 Basel (CH); GUBA, Wolfgang, 4070 Basel (CH); MAZUNIN, Dmitry, 4070 Basel (CH); PINARD, Emmanuel, 4070 Basel (CH)
(74) Representative: Jochnowitz, Evan
(86) International application number: PCT/EP2023/070657
(87) International publication number: WO 2024/023128

(56) References cited:
- WO-A1-2019/243398
- WO-A1-2022/180136

## Description

The present invention relates to organic compounds useful for therapy and/or prophylaxis in a mammal, and in particular to compounds that modulate GPR17 activity.

The present invention provides novel compounds of formula I 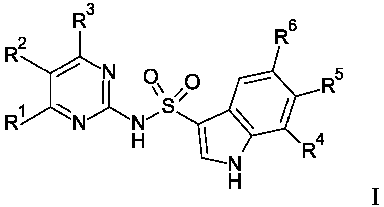 wherein,
R¹ is alkoxy or haloalkoxy;
R² is halo, alkyl, alkoxy, haloalkyl, haloalkoxy, cyanoalkyl, cyanoalkoxy or cyclopropyl optional substituted with up to two substituents independently selected from cyano and halo;
R³ is H, alkoxy or haloalkoxy;
R⁵ is H, halo, alkyl, or haloalkyl;
R⁶ is H or halo;
R⁴ is a C-linked aryl or a C-linked heteroaryl, wherein the C-linked aryl or C-linked heteroaryl is optionally substituted by one or more substituents independently selected from alkyl, halo, and haloalkyl;
and pharmaceutically acceptable salts.

Furthermore, the invention includes all racemic mixtures, all their corresponding enantiomers and/or optical isomers.

### Background of the Invention

Myelination is a process that occurs robustly during development and despite the abundant presence of oligodendrocyte precursor cells (OPCs) throughout the adult CNS, the transition to myelinating oligodendrocytes and the production of restorative myelin sheaths around denuded axons is impaired in chronic demyelinating diseases. During development, myelination proceeds in a very orderly manner, with OPCs, characterized by expression of markers such as neural/glial antigen 2 (NG2) and platelet-derived growth factor alpha (PDGFRα), differentiating into oligodendrocytes which lose NG2 and PDGFRα expression and gain the expression of markers such as myelin basic protein (MBP) and myelin oligodendrocyte glycoprotein (MOG). The production of myelin by oligodendrocytes is a very tightly regulated process and in the CNS, this can be controlled by interactions with axons, well-understood in the peripheral but not in the central nervous system (Macklin, W.B. (2010). Sci. Signal. 3, pe32-pe32, "The myelin brake: When Enough Is Enough"). Myelination can also be controlled by internal brakes within oligodendrocytes themselves, through the transcription factor EB (TFEB)-PUMA axis or through GPR17 antagonism (Chen, Y., et al. (2009). Nat Neurosci 12, 1398-1406, "The oligodendrocyte-specific G protein-coupled receptor GPR17 is a cell-intrinsic timer of myelination") (Sun, L.O., et al. (2018). Cell 175, 1811-1826.e21, "Spatiotemporal Control of CNS Myelination by Oligodendrocyte Programmed Cell Death through the TFEB-PUMA Axis"). Myelin serves not only to protect axons and facilitate neuronal transmission, but oligodendrocytes have also been shown to play an important role in metabolism of axons as well as in maintaining the electrolyte balance around axons (Schirmer, L., et al. (2014). Ann Neurol 75, 810-828, "Differential loss of KIR4.1 immunoreactivity in multiple sclerosis lesions") (Simons, M., and Nave, K.-A. (2015). Cold Spring Harb Perspect Biol. 22, "Oligodendrocytes: Myelination and Axonal Support").

GPR17 is a Class A orphan G protein-coupled receptor (GPCR). GPCRs are 7 domain transmembrane proteins that couple extracellular ligands with intracellular signaling via their intracellular association with small, heterotrimeric G-protein complexes consisting of G_{α}, G_{β}, 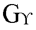 subunits. It is the coupling of the GPCR to the G_{α} subunit that confers results in downstream intracellular signaling pathways. GPR17 is known to be coupled directly to G_{α i/o}, which leads to inhibition of adenylate cyclase activity, resulting in a reduction in cyclic AMP production (cAMP). GPR17 has also been shown to couple to G_{q/11}, that targets phospholipase C. Activation of phospholipase C leads to the cleavage of phosphatidylinositol 4,5-bisphosphate which produces inositol triphosphate (IP₃) and diacylglycerol (DAG). IP₃ consequently binds to the IP₃ receptor on the endoplasmic reticulum and causes an increase in intracellular calcium levels (Hanlon, C.D., and Andrew, D.J. (2015). J Cell Sci. 128, 3533-3542, "Outside-in signaling-a brief review of GPCR signaling with a focus on the Drosophila GPCR family") (Inoue, A., et al. (2019), Cell 177, 1933-1947.e25, "Illuminating G-Protein-Coupling Selectivity of GPCRs").

The role of GPR17 in myelination was first identified in a screen of the optic nerves of *Olig1* knockout mice to identify genes regulating myelination. GPR17 expression was found to be expressed only in the myelinating cells of the CNS and absent from the Schwann cells, the peripheral nervous system's myelinating cells. The expression of GPR17 was found to be exclusively expressed in the oligodendrocyte lineage cells and was downregulated in myelinating oligodendrocyte (Chen, Y., et al. (2009)). Specifically, GPR17 expression is found to be present at low levels early on in the OPC and increases in the pre-myelinating oligodendrocyte before the expression is downregulated in the mature, myelinating oligodendrocyte (Boda, E., et al. (2011), Glia 59, 1958-1973, "The GPR17 receptor in NG2 expressing cells: Focus on in vivocell maturation and participation in acute trauma and chronic damage") (Dziedzic, A., et al. (2020). Int. J. Mol. Sci. 21, 1852, "The gpr17 receptor-a promising goal for therapy and a potential marker of the neurodegenerative process in multiple sclerosis") (Fumagalli, M. et al. (2011), J Biol Chem 286, 10593-10604, "Phenotypic changes, signaling pathway, and functional correlates of GPR17-expressing neural precursor cells during oligodendrocyte differentiation"). GPR17 knockout animals were shown to exhibit precocious myelination throughout the CNS and conversely, transgenic mice overexpressing *GPR17* in oligodendrocytes with the CNP-Cre (2', 3' - cyclic-nucleotide 3'-phosphodiesterase) promoter exhibited myelinogenesis defects, in line with what is to be expected of a cell-intrinsic brake on the myelination process (Chen, Y., et al. (2009)). Furthermore, loss of GPR17 enhances remyelination following demyelination with lysophosphatidylcholine-induced demyelination (Lu, C., Dong, et al. (2018), Sci. Rep. 8, 4502, "G-Protein-Coupled Receptor Gpr17 Regulates Oligodendrocyte Differentiation in Response to Lysolecithin-Induced Demyelination"). As such, antagonism of GPR17 that promotes the differentiation of oligodendrocyte lineage cells into mature, myelinating oligodendrocytes would lead to increase in myelination following demyelination.

Multiple sclerosis (MS) is a chronic neurodegenerative disease that is characterized by the loss of myelin, the protective fatty lipid layer surrounding axons, in the central nervous system (CNS). Prevention of myelin loss or remyelination of denuded axons is thought to prevent axonal degeneration and thus prevent progression of the disease (Franklin, R.J. (2002), Nat Rev Neurosci 3, 705-714, "Why does remyelination fail in multiple sclerosis?"). Due to the restorative impact that myelin repair has on the central nervous system, such a treatment will benefit all types of MS namely relapse-remitting, secondary progressive, primary progressive and progressive relapsing MS. Reparation of lost myelin will alleviate neurological symptoms associated with MS due to the neuroprotective effect of preserving axons.

Due to the essential role that myelination plays in functioning of the nervous system, facilitating OPC to oligodendrocyte differentiation has the potential to impact multiple diseases where white matter defects/irregularities due to either loss of myelinating oligodendrocytes or hampered differentiation of OPCs to oligodendrocytes have been observed, due to the disease itself or inflammation. This is in addition to the diseases where GPR17 expression itself is altered.

The diseases that GPR17 antagonism can be thus used to yield a positive disease outcome include, but are not limited to:
Direct damage to myelin sheaths:
- Metabolic conditions that lead to destruction of central myelin such as central pontine myelinolysis, extra-pontine myelinolysis due to overly-rapid correction of hyponatremia in conditions for instance, but not limited to, alcoholism, liver disease, immunosuppression after transplantation
- Carbon monoxide poisoning where oligodendrocyte dysfunction and failure to regenerate has been reported in the deep white matter layers of the brain
- Nutritional deficiency that results in myelin loss or failure to properly generate myelin during development
- Virus-induced demyelination

Primary demyelinating disorders
- Multiple Sclerosis (relapse-remitting, secondary progressive, primary progressive and progressive relapsing MS)
- Acute and multiphasic disseminated encephalomyelitis
- Neuromyelitis optica spectrum disorders including optic neuritis
- Transverse myelitis
- Leukodystrophies such as adrenoleukodystrophy, adrenomyeloneuropathy and other inherited leukodystrophies that result in myelin loss

CNS disorders with associated myelin loss:
- Alzheimer's Disease
- Schizophrenia
- Parkinson's Disease
- Huntington's disease
- Amyotrophic lateral
- Ischemia due to stroke

Other diseases:
- Inflammation in the CNS for instance following encephalitis, primary angiitis, meningitis

The compounds of formula I bind to and modulates GPR17 activity.

The compounds of formula I are therefore particularly useful in the treatment of diseases related to GPR17 antagonism.

The compounds of formula I are particularly useful in the treatment or prophylaxis of multiple sclerosis (MS), conditions related to direct damage to myelin sheaths such as carbon monoxide poisoning or virus induced demyelination, primary demyelinating disorders such as acute and multiphasic disseminated encephalomyelitis, and other CNS disorders associated with myelin loss such as Alzheimer's disease, schizophrenia, Parkinson's disease and Huntington's disease.

WO2019/243398 A1 discloses pyridin-2-yl-1H-indole-3-sulfonamide type GPR17 antagonists.

### Summary of the Invention

The present invention provides novel compounds of formula I 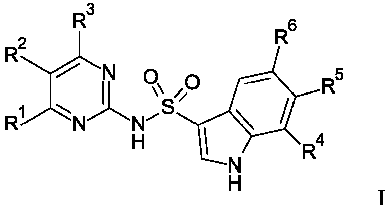 wherein
R¹ is alkoxy or haloalkoxy;
R² is halo, alkyl, alkoxy, haloalkyl, haloalkoxy, cyanoalkyl, cyanoalkoxy or cyclopropyl optional substituted with up to two substituents independently selected from cyano and halo;
R³ is H, alkoxy or haloalkoxy;
R⁵ is H, halo, alkyl, or haloalkyl;
R⁶ is H or halo;
R⁴ is a C-linked aryl or a C-linked heteroaryl, wherein the C-linked aryl or C-linked heteroaryl is optionally substituted by one or more substituents independently selected from alkyl, halo, and haloalkyl;
and pharmaceutically acceptable salts.

The term "alkyl" denotes a monovalent linear or branched saturated hydrocarbon group of 1 to 6 carbon atoms. In some embodiments, if not otherwise described, alkyl comprises 1 to 6 carbon atoms (C₁₋₆-alkyl), or 1 to 4 carbon atoms (C₁₋₄-alkyl). Examples of C₁₋₆-alkyl include methyl, ethyl, propyl, isopropyl, n-butyl, iso-butyl, sec-butyl, tert-butyl and pentyl. Particular alkyl group is methyl. When an alkyl residue having a specific number of carbons is named, all geometric isomers having that number of carbons may be encompassed. Thus, for example, "butyl" can include n-butyl, sec-butyl, isobutyl and t-butyl, and "propyl" can include n-propyl and isopropyl. Particular example of alkyl is methyl.

The term "alkoxy" denotes a group of the formula -O-R', wherein R' is a C₁₋₆-alkyl group. Examples of C₁₋₆-alkoxy groups include methoxy, ethoxy, n-propoxy, isopropoxy, n-butoxy, isobutoxy and tert-butoxy. Particular example is methoxy.

The term "aryl", alone or in combination with other groups, denotes a monovalent cyclic aromatic hydrocarbon moiety consisting of a mono- or bicyclic aromatic ring. Preferred aryl is phenyl. Aryl may be unsubstituted or substituted as described herein. Particular example of aryl is phenyl.

The term "halogen", "halide" and "halo" are used interchangeably herein and denote fluoro, chloro, bromo or iodo. Particular halogens are fluoro, chloro and bromo.

The term "haloalkyl" denotes a C₁₋₆-alkyl group wherein at least one of the hydrogen atoms of the C₁₋₆-alkyl group has been replaced by the same or different halogen atoms. Particular examples are difluoroethyl and diflouromethyl.

The term "haloalkoxy" denotes a C₁₋₆-alkoxy group wherein at least one of the hydrogen atoms of the C₁₋₆-alkoxy group has been replaced by the same or different halogen atoms. Particular examples fluoroethoxy, difluoroethoxy, and difluoromethoxy.

The term "cyano" denotes a -C≡N group.

"Cyanoalkyl" means a moiety of the formula -R'-R", where R' is alkyl as defined herein and R" is cyano or nitrile. An example of cyanoalkyl is cyanoethyl.

"Cyanoalkoxy" means a moiety of the formula -R'-R", where R' is alkoxy as defined herein and R" is cyano or nitrile. An example of cyanoalkoxy is cyanomethoxy.

The term "heteroaryl" denotes a monovalent aromatic mono- or bicyclic ring system of 4 to 9 ring atoms, comprising 1, 2, 3, or 4 ring heteroatoms selected from N and O, the remaining ring atoms being carbon. Heteroatom may also be S. Bicyclic means consisting of two cycles having one or two ring atoms in common. Example for heteroaryl are pyrimidyl, oxazolyl, pyridazinyl, imidazolyl, pyrazinyl, and pyridyl. Other example for heteroaryl are pyrazolyl, pyrimidinyl, isoazolyl, triazolyl, oxadiazolyl, oxazolyl, pyrrolylimidazolyl, and thiazolyl.

The term "C-linked heteroaryl" means denotes a heteroaryl system up to 9 ring atoms, wherein the heteroaryl system is attached to the rest of the molecule through a C-atom.

The term "pharmaceutically acceptable salts" refers to those salts which retain the biological effectiveness and properties of the free bases or free acids, which are not biologically or otherwise undesirable. The salts are formed with inorganic acids such as hydrochloric acid, hydrobromic acid, sulfuric acid, nitric acid, phosphoric acid, particularly hydrochloric acid, and organic acids such as formic acid, acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid, N-acetylcystein. In addition these salts may be prepared from addition of an inorganic base or an organic base to the free acid. Salts derived from an inorganic base include, but are not limited to, the sodium, potassium, lithium, ammonium, calcium, magnesium salts. Salts derived from organic bases include, but are not limited to salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, lysine, arginine, N-ethylpiperidine, piperidine, polyamine resins. The compound of formula I can also be present in the form of zwitterions. Particularly preferred pharmaceutically acceptable salts of compounds of formula I are the salts formed with formic acid and the salts formed with hydrochloric acid yielding a hydrochloride, dihydrochloride or trihydrochloride salt.

The abbreviation uM means microMolar and is equivalent to the symbol µM.

The abbreviation uL means microliter and is equivalent to the symbol µL.

The abbreviation ug means microgram and is equivalent to the symbol µg.

The compounds of formula I can contain several asymmetric centers and can be present in the form of optically pure enantiomers, mixtures of enantiomers such as, for example, racemates, optically pure diastereoisomers, mixtures of diastereoisomers, diastereoisomeric racemates or mixtures of diastereoisomeric racemates.

According to the Cahn-Ingold-Prelog Convention the asymmetric carbon atom can be of the "R" or "S" configuration.

Also an embodiment of the present invention provides compounds according to formula I as described herein and pharmaceutically acceptable salts or esters thereof, in particular compounds according to formula I as described herein and pharmaceutically acceptable salts thereof, more particularly compounds according to formula I as described herein.

An embodiment of the present invention provides compounds according to formula I as described herein, wherein R¹ is alkoxy.

An embodiment of the present invention provides compounds according to formula I as described herein, wherein R² is halo, alkyl, haloalkyl, haloalkoxy, cyanoalkyl, cyanoalkoxy or cyclopropyl substituted with cyano.

An embodiment of the present invention provides compounds according to formula I as described herein, wherein R² is haloalkyl, haloalkoxy, or cyclopropyl substituted with cyano.

An embodiment of the present invention provides compounds according to formula I as described herein, wherein R² is alkyl, haloalkoxy or haloalkyl.

An embodiment of the present invention provides compounds according to formula I as described herein, wherein R² is haloalkyl or haloalkoxy.

An embodiment of the present invention provides compounds according to formula I as described herein, wherein R³ is H or alkoxy.

An embodiment of the present invention provides compounds according to formula I as described herein, wherein R³ is alkoxy.

An embodiment of the present invention provides compounds according to formula I as described herein, wherein R⁵ is H, halo, or haloalkyl.

An embodiment of the present invention provides compounds according to formula I as described herein, wherein R⁵ is alkyl, H, or halo.

An embodiment of the present invention provides compounds according to formula I as described herein, wherein R⁵ is H or halo.

An embodiment of the present invention provides compounds according to formula I as described herein, wherein R⁶ is H.

An embodiment of the present invention provides compounds according to formula I as described herein, wherein R⁴ is a C-linked aryl or a C-linked heteroaryl, wherein the C-linked aryl or C-linked heteroaryl is optionally substituted by one or more substituents independently selected from alkyl and halo.

An embodiment of the present invention provides compounds according to formula I as described herein, wherein R⁴ is a C-linked aryl or a C-linked heteroaryl, wherein the C-linked aryl or C-linked heteroaryl is optionally substituted by alkyl, halo, or haloalkyl.

An embodiment of the present invention provides compounds according to formula I as described herein, wherein R⁴ is selected from
i. a C-linked 6-member aryl optionally substituted with alkyl, halo or haloalkyl;
ii. a C-linked 6-member heteroaryl comprising 1 to 3 nitrogen heteroatoms optionally substituted with alkyl, halo or haloalkyl;
iii. a C-linked 5-member heteroaryl comprising 1-3 heteroatoms independently selected from N, S and O, optionally substituted with alkyl, halo or haloalkyl; and
iv. a C-linked 8-9 membered bicyclic heteroaryl system comprising 2 N heteroatoms.

An embodiment of the present invention provides compounds according to formula I as described herein, wherein R⁴ is selected from
i. a C-linked 6-member aryl;
ii. a C-linked 6-member heteroaryl comprising 1 to 2 nitrogen heteroatoms optionally substituted with alkyl or halo;
iii. a C-linked 5-member heteroaryl comprising 1-3 heteroatoms independently selected from N, S and O, optionally substituted with 1 to 2 substituents independently selected from alkyl and halo; and
iv. a C-linked 8-membered bicyclic heteroaryl system comprising 2 N heteroatoms.

An embodiment of the present invention provides compounds according to formula I as described herein, wherein R⁴ is selected from
i. a C-linked 6-member heteroaryl comprising 1 to 3 nitrogen heteroatoms optionally substituted with alkyl or halo; and
ii. a C-linked 5-member heteroaryl comprising 1-2 heteroatoms independently selected from N and O.

An embodiment of the present invention provides compounds according to formula I as described herein, wherein R⁴ is selected from 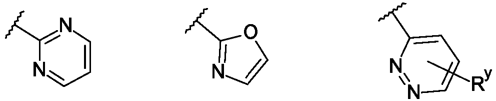 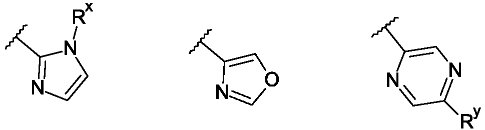 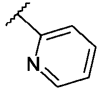 wherein R^{x} is H or alkyl and R^{y} is H, alkyl or halo.

An embodiment of the present invention provides compounds according to formula I as described herein, wherein R⁴ is selected from 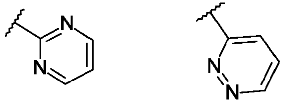 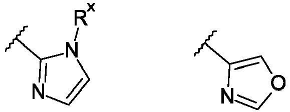 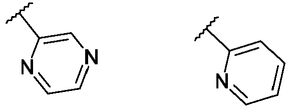 wherein R^{x} is methyl.

An embodiment of the present invention provides compounds according to formula I as described herein, wherein
R¹ is alkoxy;
R² is halo, alkyl, haloalkyl, haloalkoxy, cyanoalkyl, cyanoalkoxy or cyclopropyl substituted with cyano;
R³ is H or alkoxy;
R⁵ is H, halo, alkyl, or haloalkyl;
R⁶ is H or halo;
R⁴ is a C-linked aryl or a C-linked heteroaryl, wherein the C-linked aryl or C-linked heteroaryl is optionally substituted by one or more substituents independently selected from alkyl and halo;
and pharmaceutically acceptable salts.

An embodiment of the present invention provides compounds according to formula I as described herein, wherein
R¹ is alkoxy;
R² is halo, alkyl, haloalkyl, haloalkoxy, cyanoalkyl, cyanoalkoxy or cyclopropyl substituted with cyano;
R³ is H or alkoxy;
R⁵ is H, halo, alkyl, or haloalkyl;
R⁶ is H or halo;
R⁴ is selected from
   i. a C-linked 6-member aryl;
   ii. a C-linked 6-member heteroaryl comprising 1 to 2 nitrogen heteroatoms optionally substituted with alkyl or halo;
   iii. a C-linked 5-member heteroaryl comprising 1-3 heteroatoms independently selected from N, S and O, optionally substituted with 1 to 2 substituents independently selected from alkyl and halo; and
   iv. a C-linked 8-membered bicyclic heteroaryl system comprising 2 N heteroatoms; and pharmaceutically acceptable salts.

An embodiment of the present invention provides compounds according to formula I as described herein, wherein
R¹ is alkoxy or haloalkoxy;
R² is halo, alkyl, alkoxy, haloalkyl, haloalkoxy, cyanoalkyl, cyanoalkoxy or cyclopropyl optional substituted with up to two substituents independently selected from cyano and halo;
R³ is H, alkoxy or haloalkoxy;
R⁵ is H, halo, alkyl, or haloalkyl;
R⁶ is H or halo;
R⁴ is a C-linked aryl or a C-linked heteroaryl, wherein the C-linked aryl or C-linked heteroaryl is optionally substituted by alkyl, halo, or haloalkyl;
and pharmaceutically acceptable salts.

An embodiment of the present invention provides compounds according to formula I as described herein, wherein
R¹ is alkoxy;
R² is haloalkyl, haloalkoxy, or cyclopropyl substituted with cyano;
R³ is alkoxy;
R⁵ is H, halo, or haloalkyl;
R⁶ is H;
R⁴ is selected from 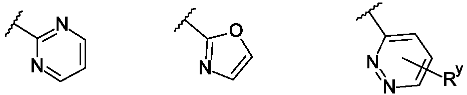 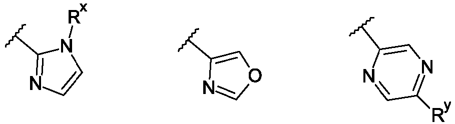 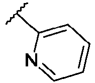 wherein R^{x} is H or alkyl and R^{y} is H, alkyl or halo;
and pharmaceutically acceptable salts.

An embodiment of the present invention provides compounds according to formula I as described herein, wherein
R¹ is alkoxy;
R² is haloalkyl or haloalkoxy;
R³ is alkoxy;
R⁵ is H or halo;
R⁶ is H;
R⁴ is selected from 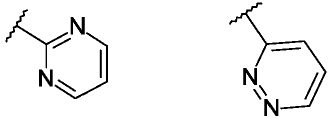 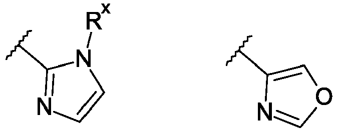 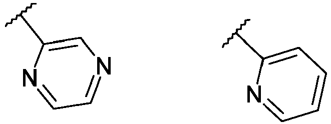 wherein R^{x} is alkyl;
and pharmaceutically acceptable salts.

Particular examples of compounds of formula I as described herein are selected from
6-chloro-N-[5-(2,2-difluoroethoxy)-4,6-dimethoxy-pyrimidin-2-yl]-7-(2-pyrimidyl)-1H-indole-3-sulfonamide;
6-chloro-N-[5-(2,2-difluoroethoxy)-4,6-dimethoxy-pyrimidin-2-yl]-7-oxazol-2-yl-1H-indole-3-sulfonamide;
6-chloro-N-[5-(2,2-difluoroethyl)-4,6-dimethoxy-pyrimidin-2-yl]-7-(2-pyrimidyl)-1H-indole-3-sulfonamide;
6-chloro-N-[5-(2,2-difluoroethoxy)-4,6-dimethoxy-pyrimidin-2-yl]-7-pyridazin-3-yl-1H-indole-3-sulfonamide;
N-[5-(2,2-difluoroethyl)-4,6-dimethoxy-pyrimidin-2-yl]-7-(1-methylimidazol-2-yl)-1H-indole-3-sulfonamide;
6-chloro-N-[5-(2,2-difluoroethyl)-4,6-dimethoxy-pyrimidin-2-yl]-7-pyridazin-3-yl-1H-indole-3-sulfonamide;
N-[5-(2,2-difluoroethoxy)-4,6-dimethoxy-pyrimidin-2-yl]-7-(1-methylimidazol-2-yl)-1H-indole-3-sulfonamide;
6-chloro-N-[5-(2,2-difluoroethoxy)-4,6-dimethoxy-pyrimidin-2-yl]-7-(1-methylimidazol-2-yl)-1H-indole-3-sulfonamide;
N-[5-(2,2-difluoroethoxy)-4,6-dimethoxy-pyrimidin-2-yl]-6-fluoro-7-(1-methylimidazol-2-yl)-1H-indole-3-sulfonamide;
N-[5-(2,2-difluoroethyl)-4,6-dimethoxy-pyrimidin-2-yl]-6-fluoro-7-(1-methylimidazol-2-yl)-1H-indole-3-sulfonamide;
N-[5-(2,2-difluoroethyl)-4,6-dimethoxy-pyrimidin-2-yl]-7-pyridazin-3-yl-1H-indole-3-sulfonamide;
N-[5-(2,2-difluoroethoxy)-4,6-dimethoxy-pyrimidin-2-yl]-7-pyridazin-3-yl-1H-indole-3-sulfonamide;
N-[5-(2,2-difluoroethoxy)-4,6-dimethoxy-pyrimidin-2-yl]-7-(4-methylpyridazin-3-yl)-1H-indole-3-sulfonamide;
6-chloro-N-[5-(2,2-difluoroethoxy)-4,6-dimethoxy-pyrimidin-2-yl]-7-oxazol-4-yl-1H-indole-3-sulfonamide;
6-chloro-N-[5-(2,2-difluoroethyl)-4,6-dimethoxy-pyrimidin-2-yl]-7-pyrazin-2-yl-1H-indole-3-sulfonamide;
6-chloro-N-[5-(2,2-difluoroethoxy)-4,6-dimethoxy-pyrimidin-2-yl]-7-pyrazin-2-yl-1H-indole-3-sulfonamide;
N-[5-(2,2-difluoroethyl)-4,6-dimethoxy-pyrimidin-2-yl]-7-(2-pyrimidyl)-1H-indole-3-sulfonamide;
6-chloro-N-[5-(2,2-difluoroethyl)-4,6-dimethoxy-pyrimidin-2-yl]-7-(2-pyridyl)-1H-indole-3-sulfonamide;
6-chloro-N-[5-(2,2-difluoroethoxy)-4,6-dimethoxy-pyrimidin-2-yl]-7-(2-pyridyl)-1H-indole-3-sulfonamide;
6-chloro-N-[5-(difluoromethoxy)-4,6-dimethoxy-pyrimidin-2-yl]-7-pyrazin-2-yl-1H-indole-3-sulfonamide;
6-chloro-N-[5-(2-fluoroethoxy)-4,6-dimethoxy-pyrimidin-2-yl]-7-pyrazin-2-yl-1H-indole-3-sulfonamide;
6-chloro-N-[5-(2-fluoroethoxy)-4,6-dimethoxy-pyrimidin-2-yl]-7-(2-pyrimidyl)-1H-indole-3-sulfonamide;
6-chloro-N-[5-(difluoromethoxy)-4,6-dimethoxy-pyrimidin-2-yl]-7-(2-pyrimidyl)-1H-indole-3-sulfonamide;
6-chloro-N-[5-(2-cyanocyclopropyl)-4,6-dimethoxy-pyrimidin-2-yl]-7-(2-pyrimidyl)-1H-indole-3-sulfonamide;
N-[5-(2,2-difluoroethyl)-4,6-dimethoxy-pyrimidin-2-yl]-7-pyrazin-2-yl-1H-indole-3-sulfonamide;
N-[5-(2,2-difluoroethoxy)-4,6-dimethoxy-pyrimidin-2-yl]-6-fluoro-7-(2-pyrimidyl)-1H-indole-3-sulfonamide;
N-[5-(2,2-difluoroethyl)-4,6-dimethoxy-pyrimidin-2-yl]-6-fluoro-7-(2-pyrimidyl)-1H-indole-3-sulfonamide;
6-fluoro-N-[5-(2-fluoroethoxy)-4,6-dimethoxy-pyrimidin-2-yl]-7-(2-pyrimidyl)-1H-indole-3-sulfonamide;
N-[5-(difluoromethoxy)-4,6-dimethoxy-pyrimidin-2-yl]-6-fluoro-7-(2-pyrimidyl)-1H-indole-3-sulfonamide;
N-[5-(2,2-difluoroethoxy)-4,6-dimethoxy-pyrimidin-2-yl]-7-pyrazin-2-yl-1H-indole-3-sulfonamide;
N-[5-(2,2-difluoroethyl)-4,6-dimethoxy-pyrimidin-2-yl]-6-fluoro-7-pyrazin-2-yl-1H-indole-3-sulfonamide;
6-fluoro-N-[5-(2-fluoroethoxy)-4,6-dimethoxy-pyrimidin-2-yl]-7-pyrazin-2-yl-1H-indole-3-sulfonamide;
N-[5-(2,2-difluoroethoxy)-4,6-dimethoxy-pyrimidin-2-yl]-6-fluoro-7-pyrazin-2-yl-1H-indole-3-sulfonamide;
N-[5-(difluoromethoxy)-4,6-dimethoxy-pyrimidin-2-yl]-6-fluoro-7-pyrazin-2-yl-1H-indole-3-sulfonamide;
6-bromo-N-[5-(2,2-difluoroethyl)-4,6-dimethoxy-pyrimidin-2-yl]-7-(2-pyrimidyl)-1H-indole-3-sulfonamide;
6-bromo-N-[5-(difluoromethoxy)-4,6-dimethoxy-pyrimidin-2-yl]-7-(2-pyrimidyl)-1H-indole-3-sulfonamide;
6-bromo-N-[5-(2-fluoroethoxy)-4,6-dimethoxy-pyrimidin-2-yl]-7-(2-pyrimidyl)-1H-indole-3-sulfonamide;
6-bromo-N-[5-(2,2-difluoroethoxy)-4,6-dimethoxy-pyrimidin-2-yl]-7-(2-pyrimidyl)-1H-indole-3-sulfonamide;
6-bromo-N-[5-(2,2-difluoroethyl)-4,6-dimethoxy-pyrimidin-2-yl]-7-pyrazin-2-yl-1H-indole-3-sulfonamide;
6-bromo-N-[5-(difluoromethoxy)-4,6-dimethoxy-pyrimidin-2-yl]-7-pyrazin-2-yl-1H-indole-3-sulfonamide;
6-bromo-N-[5-(2,2-difluoroethoxy)-4,6-dimethoxy-pyrimidin-2-yl]-7-pyrazin-2-yl-1H-indole-3-sulfonamide;
6-chloro-N-[5-(2,2-difluoroethoxy)-4,6-dimethoxy-pyrimidin-2-yl]-7-(5-fluoropyrazin-2-yl)-1H-indole-3-sulfonamide;
6-bromo-N-[5-(2,2-difluoroethyl)-4,6-dimethoxy-pyrimidin-2-yl]-7-pyridazin-3-yl-1H-indole-3-sulfonamide;
6-(difluoromethyl)-N-[5-(2-fluoroethoxy)-4,6-dimethoxy-pyrimidin-2-yl]-7-(2-pyrimidyl)-1H-indole-3-sulfonamide;
N-[5-(2,2-difluoroethoxy)-4,6-dimethoxy-pyrimidin-2-yl]-6-fluoro-7-(1H-imidazol-2-yl)-1H-indole-3-sulfonamide; and
pharmaceutically acceptable salts thereof.

Further particular examples of compounds of formula I as described herein are selected from
6-chloro-N-[5-(2,2-difluoroethoxy)-4,6-dimethoxy-pyrimidin-2-yl]-7-(1,5-dimethylimidazol-4-yl)-1H-indole-3-sulfonamide;
6-chloro-N-[5-(2,2-difluoroethoxy)-4,6-dimethoxy-pyrimidin-2-yl]-7-(1-methylimidazol-4-yl)-1H-indole-3-sulfonamide;
N-[5-(2,2-difluoroethoxy)-4,6-dimethoxy-pyrimidin-2-yl]-6-methyl-7-(2-pyrimidyl)-1H-indole-3-sulfonamide;
N-[5-(2,2-difluoroethoxy)-4,6-dimethoxy-pyrimidin-2-yl]-6-(difluoromethyl)-7-(2-pyrimidyl)-1H-indole-3-sulfonamide;
N-[5-(difluoromethoxy)-4,6-dimethoxy-pyrimidin-2-yl]-6-(difluoromethyl)-7-(2-pyrimidyl)-1H-indole-3-sulfonamide;
N-[5-(2,2-difluoroethyl)-4,6-dimethoxy-pyrimidin-2-yl]-6-(difluoromethyl)-7-(2-pyrimidyl)-1H-indole-3-sulfonamide;
N-[5-(2,2-difluoroethyl)-4,6-dimethoxy-pyrimidin-2-yl]-6-methyl-7-(2-pyrimidyl)-1H-indole-3-sulfonamide;
N-[5-(2,2-difluoroethyl)-4,6-dimethoxy-pyrimidin-2-yl]-7-(3-methylpyrazin-2-yl)-1H-indole-3-sulfonamide;
N-[5-(2,2-difluoroethoxy)-4,6-dimethoxy-pyrimidin-2-yl]-7-(3-methylpyrazin-2-yl)-1H-indole-3-sulfonamide;
N-[5-(difluoromethoxy)-4,6-dimethoxy-pyrimidin-2-yl]-6-methyl-7-(2-pyrimidyl)-1H-indole-3-sulfonamide;
N-[5-(2-fluoroethoxy)-4,6-dimethoxy-pyrimidin-2-yl]-6-methyl-7-(2-pyrimidyl)-1H-indole-3-sulfonamide;
N-[5-(2,2-difluoroethoxy)-4,6-dimethoxy-pyrimidin-2-yl]-6-methyl-7-pyridazin-3-yl-1H-indole-3-sulfonamide;
N-[5-(2,2-difluoroethoxy)-4,6-dimethoxy-pyrimidin-2-yl]-6-methyl-7-pyrazin-2-yl-1H-indole-3-sulfonamide;
N-[5-(difluoromethoxy)-4,6-dimethoxy-pyrimidin-2-yl]-6-methyl-7-pyrazin-2-yl-1H-indole-3-sulfonamide;
6-chloro-N-[5-(2,2-difluoroethoxy)-4,6-dimethoxy-pyrimidin-2-yl]-5-fluoro-7-(2-pyrimidyl)-1H-indole-3-sulfonamide;
6-chloro-N-[5-(2,2-difluoroethoxy)-4,6-dimethoxy-pyrimidin-2-yl]-7-(1-methylpyrazol-3-yl)-1H-indole-3-sulfonamide;
6-chloro-N-[5-(2,2-difluoroethoxy)-4,6-dimethoxy-pyrimidin-2-yl]-7-(2-methylpyrimidin-4-yl)-1H-indole-3-sulfonamide;
6-chloro-N-[5-(2,2-difluoroethoxy)-4,6-dimethoxy-pyrimidin-2-yl]-7-(6-methylpyridazin-3-yl)-1H-indole-3-sulfonamide;
6-chloro-7-(3-chloropyrazin-2-yl)-N-[5-(difluoromethoxy)-4,6-dimethoxy-pyrimidin-2-yl]-1H-indole-3-sulfonamide;
6-chloro-7-(3-chloropyrazin-2-yl)-N-[5-(2,2-difluoroethyl)-4,6-dimethoxy-pyrimidin-2-yl]-1H-indole-3-sulfonamide;
6-chloro-7-(3-chloropyrazin-2-yl)-N-[5-(2,2-difluoroethoxy)-4,6-dimethoxy-pyrimidin-2-yl]-1H-indole-3-sulfonamide;
6-chloro-N-[5-(difluoromethoxy)-4,6-dimethoxy-pyrimidin-2-yl]-7-(1-methylpyrazol-3-yl)-1H-indole-3-sulfonamide;
6-chloro-N-[5-(2-fluoroethoxy)-4,6-dimethoxy-pyrimidin-2-yl]-7-(1-methylpyrazol-3-yl)-1H-indole-3-sulfonamide;
6-bromo-N-[5-(2,2-difluoroethoxy)-4,6-dimethoxy-pyrimidin-2-yl]-7-(1-methylpyrazol-4-yl)-1H-indole-3-sulfonamide;
6-chloro-N-[5-(2,2-difluoroethoxy)-4,6-dimethoxy-pyrimidin-2-yl]-7-phenyl-1H-indole-3-sulfonamide;
6-chloro-N-[5-(2,2-difluoroethyl)-4,6-dimethoxy-pyrimidin-2-yl]-7-phenyl-1H-indole-3-sulfonamide;
N-[5-(2,2-difluoroethoxy)-4,6-dimethoxy-pyrimidin-2-yl]-7-(2-pyrimidyl)-1H-indole-3-sulfonamide;
6-chloro-N-[5-(2,2-difluoroethoxy)-4,6-dimethoxy-pyrimidin-2-yl]-7-(3-methylpyrazin-2-yl)-1H-indole-3-sulfonamide;
6-chloro-N-[5-(2,2-difluoroethyl)-4,6-dimethoxy-pyrimidin-2-yl]-7-(3-methylpyrazin-2-yl)-1H-indole-3-sulfonamide;
N-(5-bromo-4,6-dimethoxy-pyrimidin-2-yl)-6-chloro-7-(2-pyrimidyl)-1H-indole-3-sulfonamide;
6-chloro-N-[5-(2,2-difluoroethyl)-4,6-dimethoxy-pyrimidin-2-yl]-7-(4-pyrimidyl)-1H-indole-3-sulfonamide;
6-chloro-N-[5-(2,2-difluoroethoxy)-4,6-dimethoxy-pyrimidin-2-yl]-7-(4-pyrimidyl)-1H-indole-3-sulfonamide;
6-chloro-N-[5-(cyanomethoxy)-4,6-dimethoxy-pyrimidin-2-yl]-7-(2-pyrimidyl)-1H-indole-3-sulfonamide;
6-chloro-N-[5-(2-cyanoethyl)-4,6-dimethoxy-pyrimidin-2-yl]-7-(2-pyrimidyl)-1H-indole-3-sulfonamide;
6-chloro-N-[5-(2-fluoroethoxy)-4-methoxy-pyrimidin-2-yl]-7-(2-pyrimidyl)-1H-indole-3-sulfonamide;
6-chloro-N-[5-(difluoromethoxy)-4,6-dimethoxy-pyrimidin-2-yl]-7-(3-methylpyrazin-2-yl)-1H-indole-3-sulfonamide;
6-chloro-N-[5-(2,2-difluoroethyl)-4,6-dimethoxy-pyrimidin-2-yl]-7-(5-methylpyrazin-2-yl)-1H-indole-3-sulfonamide;
6-chloro-N-[5-(difluoromethoxy)-4,6-dimethoxy-pyrimidin-2-yl]-7-(5-methylpyrazin-2-yl)-1H-indole-3-sulfonamide;
6-chloro-N-[5-(2,2-difluoroethoxy)-4,6-dimethoxy-pyrimidin-2-yl]-7-(5-methylpyrazin-2-yl)-1H-indole-3-sulfonamide;
6-chloro-N-(4,6-dimethoxy-5-methyl-pyrimidin-2-yl)-7-(2-pyrimidyl)-1H-indole-3-sulfonamide;
6-chloro-N-[5-(2,2-difluoroethyl)-4,6-dimethoxy-pyrimidin-2-yl]-7-(6-methylpyrazin-2-yl)-1H-indole-3-sulfonamide;
6-chloro-N-[5-(difluoromethoxy)-4,6-dimethoxy-pyrimidin-2-yl]-7-(6-methylpyrazin-2-yl)-1H-indole-3-sulfonamide;
6-chloro-N-[5-(2,2-difluoroethoxy)-4,6-dimethoxy-pyrimidin-2-yl]-7-(6-methylpyrazin-2-yl)-1H-indole-3-sulfonamide;
6-chloro-N-[5-(2,2-difluoroethoxy)-4,6-dimethoxy-pyrimidin-2-yl]-7-(5-pyrimidyl)-1H-indole-3-sulfonamide;
6-chloro-7-(5-chloropyrazin-2-yl)-N-[5-(2,2-difluoroethoxy)-4,6-dimethoxy-pyrimidin-2-yl]-1H-indole-3-sulfonamide;
6-chloro-7-(5-chloropyrazin-2-yl)-N-[5-(2,2-difluoroethyl)-4,6-dimethoxy-pyrimidin-2-yl]-1H-indole-3-sulfonamide;
6-chloro-N-[5-(2,2-difluoroethoxy)-4,6-dimethoxy-pyrimidin-2-yl]-7-(6-methyl-2-pyridyl)-1H-indole-3-sulfonamide;
6-chloro-N-[5-(2,2-difluoroethyl)-4,6-dimethoxy-pyrimidin-2-yl]-7-(6-methyl-2-pyridyl)-1H-indole-3-sulfonamide;
6-chloro-N-[5-(difluoromethoxy)-4,6-dimethoxy-pyrimidin-2-yl]-7-(6-methyl-2-pyridyl)-1H-indole-3-sulfonamide;
6-chloro-N-[5-(2,2-difluoroethoxy)-4,6-dimethoxy-pyrimidin-2-yl]-7-pyridazin-4-yl-1H-indole-3-sulfonamide;
6-chloro-N-[5-(2,2-difluoroethoxy)-4,6-dimethoxy-pyrimidin-2-yl]-7-(6-methylpyrimidin-4-yl)-1H-indole-3-sulfonamide;
6-chloro-N-[5-(2,2-difluoroethyl)-4,6-dimethoxy-pyrimidin-2-yl]-7-(3-fluoropyrazin-2-yl)-1H-indole-3-sulfonamide;
6-chloro-N-[5-(2,2-difluoroethoxy)-4,6-dimethoxy-pyrimidin-2-yl]-7-(3-fluoropyrazin-2-yl)-1H-indole-3-sulfonamide;
6-chloro-N-[5-(difluoromethoxy)-4,6-dimethoxy-pyrimidin-2-yl]-7-(3-fluoropyrazin-2-yl)-1H-indole-3-sulfonamide;
6-chloro-N-[5-(2,2-difluoroethoxy)-4,6-dimethoxy-pyrimidin-2-yl]-7-(3-methyl-2-pyridyl)-1H-indole-3-sulfonamide;
6-chloro-N-[5-(2,2-difluoroethoxy)-4,6-dimethoxy-pyrimidin-2-yl]-7-(1-methyltriazol-4-yl)-1H-indole-3-sulfonamide;
6-chloro-N-[5-(2,2-difluoroethyl)-4,6-dimethoxy-pyrimidin-2-yl]-7-(3-methyl-2-pyridyl)-1H-indole-3-sulfonamide;
6-chloro-N-[5-(difluoromethoxy)-4,6-dimethoxy-pyrimidin-2-yl]-7-(3-methyl-2-pyridyl)-1H-indole-3-sulfonamide;
6-chloro-N-[5-(2,2-difluoroethoxy)-4,6-dimethoxy-pyrimidin-2-yl]-7-(1-methyl-1,2,4-triazol-3-yl)-1H-indole-3-sulfonamide;
6-chloro-N-[5-(2,2-difluoroethoxy)-4,6-dimethoxy-pyrimidin-2-yl]-7-(5-fluoropyrimidin-2-yl)-1H-indole-3-sulfonamide;
6-chloro-N-[5-(2,2-difluoroethoxy)-4,6-dimethoxy-pyrimidin-2-yl]-7-(2-methyltriazol-4-yl)-1H-indole-3-sulfonamide;
6-chloro-N-[5-(difluoromethoxy)-4,6-dimethoxy-pyrimidin-2-yl]-7-(2-methyltriazol-4-yl)-1H-indole-3-sulfonamide;
6-chloro-N-[5-(2,2-difluoroethyl)-4,6-dimethoxy-pyrimidin-2-yl]-7-(2-methyltriazol-4-yl)-1H-indole-3-sulfonamide;
6-chloro-N-[5-(2-fluoroethoxy)-4,6-dimethoxy-pyrimidin-2-yl]-7-(2-methyltriazol-4-yl)-1H-indole-3-sulfonamide;
6-chloro-N-[5-(2,2-difluoroethoxy)-4,6-dimethoxy-pyrimidin-2-yl]-7-(1,3,4-oxadiazol-2-yl)-1H-indole-3-sulfonamide;
6-chloro-N-[5-(2,2-difluoroethoxy)-4,6-dimethoxy-pyrimidin-2-yl]-7-(1-methylpyrazol-4-yl)-1H-indole-3-sulfonamide;
6-chloro-N-[5-(2-fluoroethoxy)-4,6-dimethoxy-pyrimidin-2-yl]-7-(1-methylpyrazol-4-yl)-1H-indole-3-sulfonamide;
6-chloro-N-[5-(difluoromethoxy)-4,6-dimethoxy-pyrimidin-2-yl]-7-(1-methylpyrazol-4-yl)-1H-indole-3-sulfonamide;
6-chloro-N-[5-(2,2-difluoroethyl)-4,6-dimethoxy-pyrimidin-2-yl]-7-(1-methylpyrazol-4-yl)-1H-indole-3-sulfonamide;
6-chloro-N-[5-(2,2-difluoroethoxy)-4,6-dimethoxy-pyrimidin-2-yl]-7-thiazol-4-yl-1H-indole-3-sulfonamide;
6-chloro-N-[5-(2,2-difluoroethoxy)-4,6-dimethoxy-pyrimidin-2-yl]-7-oxazol-5-yl-1H-indole-3-sulfonamide;
6-chloro-N-[5-(difluoromethoxy)-4,6-dimethoxy-pyrimidin-2-yl]-7-oxazol-5-yl-1H-indole-3-sulfonamide;
6-chloro-N-[5-(2,2-difluoroethoxy)-4,6-dimethoxy-pyrimidin-2-yl]-7-isoxazol-4-yl-1H-indole-3-sulfonamide;
6-chloro-N-[5-(difluoromethoxy)-4,6-dimethoxy-pyrimidin-2-yl]-7-isoxazol-4-yl-1H-indole-3-sulfonamide;
6-chloro-N-[5-(difluoromethoxy)-4,6-dimethoxy-pyrimidin-2-yl]-7-thiazol-4-yl-1H-indole-3-sulfonamide;
N-[5-(2,2-difluoroethyl)-4,6-dimethoxy-pyrimidin-2-yl]-7-(6,7-dihydro-5H-pyrrol[1,2-c]imidazol-3-yl)-1H-indole-3-sulfonamide; and
pharmaceutically acceptable salts thereof.

Preferred examples of compounds of formula I as described herein are selected from
6-chloro-N-[5-(2,2-difluoroethoxy)-4,6-dimethoxy-pyrimidin-2-yl]-7-(2-pyrimidyl)-1H-indole-3-sulfonamide;
6-chloro-N-[5-(2,2-difluoroethyl)-4,6-dimethoxy-pyrimidin-2-yl]-7-(2-pyrimidyl)-1H-indole-3-sulfonamide;
6-chloro-N-[5-(2,2-difluoroethoxy)-4,6-dimethoxy-pyrimidin-2-yl]-7-pyridazin-3-yl-1H-indole-3-sulfonamide;
N-[5-(2,2-difluoroethyl)-4,6-dimethoxy-pyrimidin-2-yl]-7-(1-methylimidazol-2-yl)-1H-indole-3-sulfonamide;
6-chloro-N-[5-(2,2-difluoroethyl)-4,6-dimethoxy-pyrimidin-2-yl]-7-pyridazin-3-yl-1H-indole-3-sulfonamide;
N-[5-(2,2-difluoroethoxy)-4,6-dimethoxy-pyrimidin-2-yl]-7-(1-methylimidazol-2-yl)-1H-indole-3-sulfonamide;
6-chloro-N-[5-(2,2-difluoroethoxy)-4,6-dimethoxy-pyrimidin-2-yl]-7-(1-methylimidazol-2-yl)-1H-indole-3-sulfonamide;
N-[5-(2,2-difluoroethyl)-4,6-dimethoxy-pyrimidin-2-yl]-6-fluoro-7-(1-methylimidazol-2-yl)-1H-indole-3-sulfonamide;
6-chloro-N-[5-(2,2-difluoroethoxy)-4,6-dimethoxy-pyrimidin-2-yl]-7-oxazol-4-yl-1H-indole-3-sulfonamide;
6-chloro-N-[5-(2,2-difluoroethyl)-4,6-dimethoxy-pyrimidin-2-yl]-7-pyrazin-2-yl-1H-indole-3-sulfonamide;
6-chloro-N-[5-(2,2-difluoroethoxy)-4,6-dimethoxy-pyrimidin-2-yl]-7-pyrazin-2-yl-1H-indole-3-sulfonamide;
6-chloro-N-[5-(2,2-difluoroethyl)-4,6-dimethoxy-pyrimidin-2-yl]-7-(2-pyridyl)-1H-indole-3-sulfonamide;
6-chloro-N-[5-(2,2-difluoroethoxy)-4,6-dimethoxy-pyrimidin-2-yl]-7-(2-pyridyl)-1H-indole-3-sulfonamide;
6-chloro-N-[5-(difluoromethoxy)-4,6-dimethoxy-pyrimidin-2-yl]-7-pyrazin-2-yl-1H-indole-3-sulfonamide;
6-chloro-N-[5-(2-fluoroethoxy)-4,6-dimethoxy-pyrimidin-2-yl]-7-pyrazin-2-yl-1H-indole-3-sulfonamide;
6-chloro-N-[5-(difluoromethoxy)-4,6-dimethoxy-pyrimidin-2-yl]-7-(2-pyrimidyl)-1H-indole-3-sulfonamide;
6-bromo-N-[5-(2,2-difluoroethyl)-4,6-dimethoxy-pyrimidin-2-yl]-7-(2-pyrimidyl)-1H-indole-3-sulfonamide;
6-bromo-N-[5-(difluoromethoxy)-4,6-dimethoxy-pyrimidin-2-yl]-7-(2-pyrimidyl)-1H-indole-3-sulfonamide;
6-bromo-N-[5-(2,2-difluoroethyl)-4,6-dimethoxy-pyrimidin-2-yl]-7-pyrazin-2-yl-1H-indole-3-sulfonamide;
6-bromo-N-[5-(difluoromethoxy)-4,6-dimethoxy-pyrimidin-2-yl]-7-pyrazin-2-yl-1H-indole-3-sulfonamide;
6-bromo-N-[5-(2,2-difluoroethoxy)-4,6-dimethoxy-pyrimidin-2-yl]-7-pyrazin-2-yl-1H-indole-3-sulfonamide; and
pharmaceutically acceptable salts thereof.

Further preferred examples of compounds of formula I as described herein are selected from
6-chloro-N-[5-(2,2-difluoroethoxy)-4,6-dimethoxy-pyrimidin-2-yl]-7-(1,5-dimethylimidazol-4-yl)-1H-indole-3-sulfonamide;
6-chloro-N-[5-(2,2-difluoroethoxy)-4,6-dimethoxy-pyrimidin-2-yl]-7-(1-methylimidazol-4-yl)-1H-indole-3-sulfonamide;
N-[5-(2,2-difluoroethyl)-4,6-dimethoxy-pyrimidin-2-yl]-6-methyl-7-(2-pyrimidyl)-1H-indole-3-sulfonamide;
N-[5-(2,2-difluoroethyl)-4,6-dimethoxy-pyrimidin-2-yl]-7-(3-methylpyrazin-2-yl)-1H-indole-3-sulfonamide;
6-chloro-N-[5-(2,2-difluoroethoxy)-4,6-dimethoxy-pyrimidin-2-yl]-7-(1-methylpyrazol-3-yl)-1H-indole-3-sulfonamide;
6-chloro-N-[5-(2,2-difluoroethoxy)-4,6-dimethoxy-pyrimidin-2-yl]-7-(2-methylpyrimidin-4-yl)-1H-indole-3-sulfonamide;
6-chloro-N-[5-(2,2-difluoroethoxy)-4,6-dimethoxy-pyrimidin-2-yl]-7-(6-methylpyridazin-3-yl)-1H-indole-3-sulfonamide;
6-chloro-7-(3-chloropyrazin-2-yl)-N-[5-(difluoromethoxy)-4,6-dimethoxy-pyrimidin-2-yl]-1H-indole-3-sulfonamide;
6-chloro-7-(3-chloropyrazin-2-yl)-N-[5-(2,2-difluoroethyl)-4,6-dimethoxy-pyrimidin-2-yl]-1H-indole-3-sulfonamide;
6-chloro-N-[5-(difluoromethoxy)-4,6-dimethoxy-pyrimidin-2-yl]-7-(1-methylpyrazol-3-yl)-1H-indole-3-sulfonamide;
6-chloro-N-[5-(2-fluoroethoxy)-4,6-dimethoxy-pyrimidin-2-yl]-7-(1-methylpyrazol-3-yl)-1H-indole-3-sulfonamide;
6-chloro-N-[5-(2,2-difluoroethoxy)-4,6-dimethoxy-pyrimidin-2-yl]-7-(3-methylpyrazin-2-yl)-1H-indole-3-sulfonamide;
6-chloro-N-[5-(2,2-difluoroethyl)-4,6-dimethoxy-pyrimidin-2-yl]-7-(3-methylpyrazin-2-yl)-1H-indole-3-sulfonamide;
6-chloro-N-[5-(2,2-difluoroethyl)-4,6-dimethoxy-pyrimidin-2-yl]-7-(4-pyrimidyl)-1H-indole-3-sulfonamide;
6-chloro-N-[5-(2,2-difluoroethoxy)-4,6-dimethoxy-pyrimidin-2-yl]-7-(4-pyrimidyl)-1H-indole-3-sulfonamide;
6-chloro-N-[5-(difluoromethoxy)-4,6-dimethoxy-pyrimidin-2-yl]-7-(3-methylpyrazin-2-yl)-1H-indole-3-sulfonamide;
6-chloro-N-[5-(2,2-difluoroethyl)-4,6-dimethoxy-pyrimidin-2-yl]-7-(5-methylpyrazin-2-yl)-1H-indole-3-sulfonamide;
6-chloro-N-(4,6-dimethoxy-5-methyl-pyrimidin-2-yl)-7-(2-pyrimidyl)-1H-indole-3-sulfonamide;
6-chloro-N-[5-(2,2-difluoroethyl)-4,6-dimethoxy-pyrimidin-2-yl]-7-(6-methylpyrazin-2-yl)-1H-indole-3-sulfonamide;
6-chloro-N-[5-(difluoromethoxy)-4,6-dimethoxy-pyrimidin-2-yl]-7-(6-methylpyrazin-2-yl)-1H-indole-3-sulfonamide;
6-chloro-N-[5-(2,2-difluoroethoxy)-4,6-dimethoxy-pyrimidin-2-yl]-7-(6-methylpyrazin-2-yl)-1H-indole-3-sulfonamide;
6-chloro-N-[5-(2,2-difluoroethoxy)-4,6-dimethoxy-pyrimidin-2-yl]-7-(5-pyrimidyl)-1H-indole-3-sulfonamide;
6-chloro-N-[5-(2,2-difluoroethoxy)-4,6-dimethoxy-pyrimidin-2-yl]-7-(6-methyl-2-pyridyl)-1H-indole-3-sulfonamide;
6-chloro-N-[5-(2,2-difluoroethyl)-4,6-dimethoxy-pyrimidin-2-yl]-7-(6-methyl-2-pyridyl)-1H-indole-3-sulfonamide;
6-chloro-N-[5-(difluoromethoxy)-4,6-dimethoxy-pyrimidin-2-yl]-7-(6-methyl-2-pyridyl)-1H-indole-3-sulfonamide;
6-chloro-N-[5-(2,2-difluoroethoxy)-4,6-dimethoxy-pyrimidin-2-yl]-7-pyridazin-4-yl-1H-indole-3-sulfonamide;
6-chloro-N-[5-(2,2-difluoroethoxy)-4,6-dimethoxy-pyrimidin-2-yl]-7-(6-methylpyrimidin-4-yl)-1H-indole-3-sulfonamide;
6-chloro-N-[5-(2,2-difluoroethyl)-4,6-dimethoxy-pyrimidin-2-yl]-7-(3-fluoropyrazin-2-yl)-1H-indole-3-sulfonamide;
6-chloro-N-[5-(2,2-difluoroethoxy)-4,6-dimethoxy-pyrimidin-2-yl]-7-(3-fluoropyrazin-2-yl)-1H-indole-3-sulfonamide;
6-chloro-N-[5-(difluoromethoxy)-4,6-dimethoxy-pyrimidin-2-yl]-7-(3-fluoropyrazin-2-yl)-1H-indole-3-sulfonamide;
6-chloro-N-[5-(2,2-difluoroethoxy)-4,6-dimethoxy-pyrimidin-2-yl]-7-(3-methyl-2-pyridyl)-1H-indole-3-sulfonamide;
6-chloro-N-[5-(2,2-difluoroethoxy)-4,6-dimethoxy-pyrimidin-2-yl]-7-(1-methyltriazol-4-yl)-1H-indole-3-sulfonamide;
6-chloro-N-[5-(2,2-difluoroethyl)-4,6-dimethoxy-pyrimidin-2-yl]-7-(3-methyl-2-pyridyl)-1H-indole-3-sulfonamide;
6-chloro-N-[5-(2,2-difluoroethoxy)-4,6-dimethoxy-pyrimidin-2-yl]-7-(2-methyltriazol-4-yl)-1H-indole-3-sulfonamide;
6-chloro-N-[5-(2,2-difluoroethyl)-4,6-dimethoxy-pyrimidin-2-yl]-7-(2-methyltriazol-4-yl)-1H-indole-3-sulfonamide;
6-chloro-N-[5-(2-fluoroethoxy)-4,6-dimethoxy-pyrimidin-2-yl]-7-(2-methyltriazol-4-yl)-1H-indole-3-sulfonamide;
6-chloro-N-[5-(2,2-difluoroethoxy)-4,6-dimethoxy-pyrimidin-2-yl]-7-(1,3,4-oxadiazol-2-yl)-1H-indole-3-sulfonamide;
6-chloro-N-[5-(2,2-difluoroethoxy)-4,6-dimethoxy-pyrimidin-2-yl]-7-thiazol-4-yl-1H-indole-3-sulfonamide;
N-[5-(2,2-difluoroethyl)-4,6-dimethoxy-pyrimidin-2-yl]-7-(6,7-dihydro-5H-pyrrol[1,2-c]imidazol-3-yl)-1H-indole-3-sulfonamide;
and pharmaceutically acceptable salts thereof.

Processes for the manufacture of compounds of formula I as described herein are an object of the invention.

The present compounds of formula I and their pharmaceutically acceptable salts can be prepared by methods known in the art, for example, by processes described below, which process comprises reacting a compound of formula III with a compound of formula II in the presence of a base selected from N-ethyldiisopropylamine, pyridine, potassium phosphate or sodium hydride, to provide a compound of formula I, 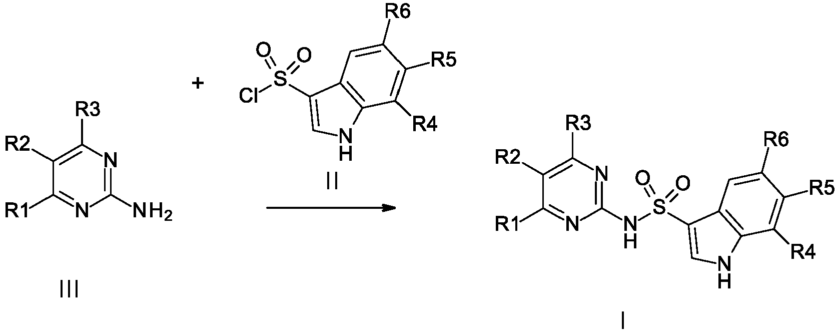 wherein R¹, R², R³, R³, R⁴, R⁵ and R⁶ are as described above.

### General Synthetic Schemes

The compounds of formula I may be prepared in accordance with the process variant described above and with the following scheme 1. The starting materials are commercially available or may be prepared in accordance with known methods.

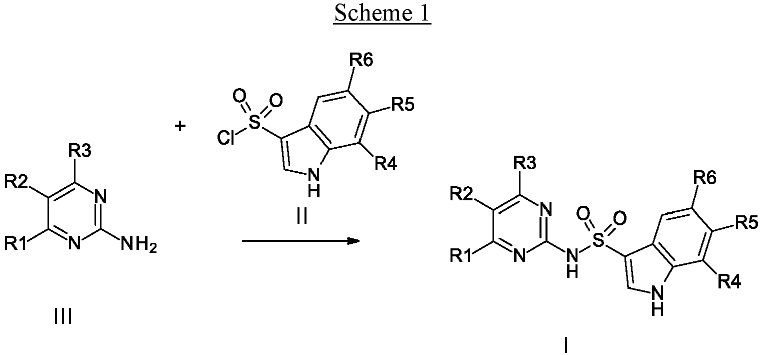

Compounds of general formula I can be prepared by reacting sulfonylchloride II with amines III in the presence of a base like N-ethyldiisopropylamine, pyridine, potassium phosphate or sodium hydride. The starting materials are commercially available or may be prepared in accordance with known methods or methods described in the following schemes.

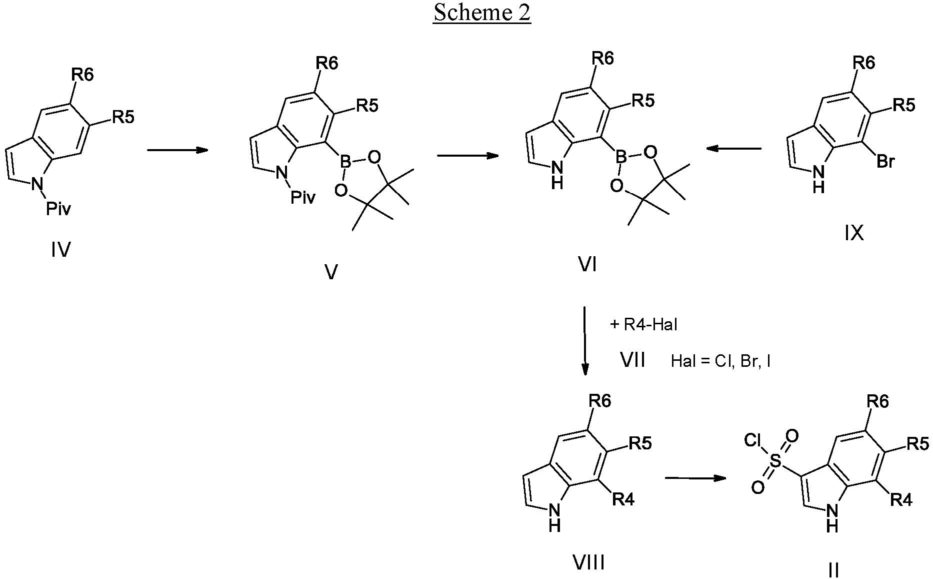

Intermediate of formula II can be prepared by a reaction sequence as described in Scheme 2. Pivaloyl-potected indole IV can be reacted with boron tribromide followed by treatment with pinacol to form compound V, which is then deprotected by treatment with a base like trimethylamine to form intermediate VI. Alternatively, this intermediate can be obtained by reaction of bromoindole derivative IX with bis(pinacolato)diboron using palladium catalysis. Intermediate VI can then be reacted with heterocyclic compound VII to obtain compound VIII using a suitable palladium catalyst and a base. Reaction of the resulting compound VIII with a) a chlorosulfonylating agent like chlorosulfonic acid, or b) a sulfonylating agent like sulfuric acid or sulfur trioxide N,N-dimethylformamide complex, followed by chlorination of the intermediate sulfonic acid with a chlorinating agent like thionylchloride provides compound II.

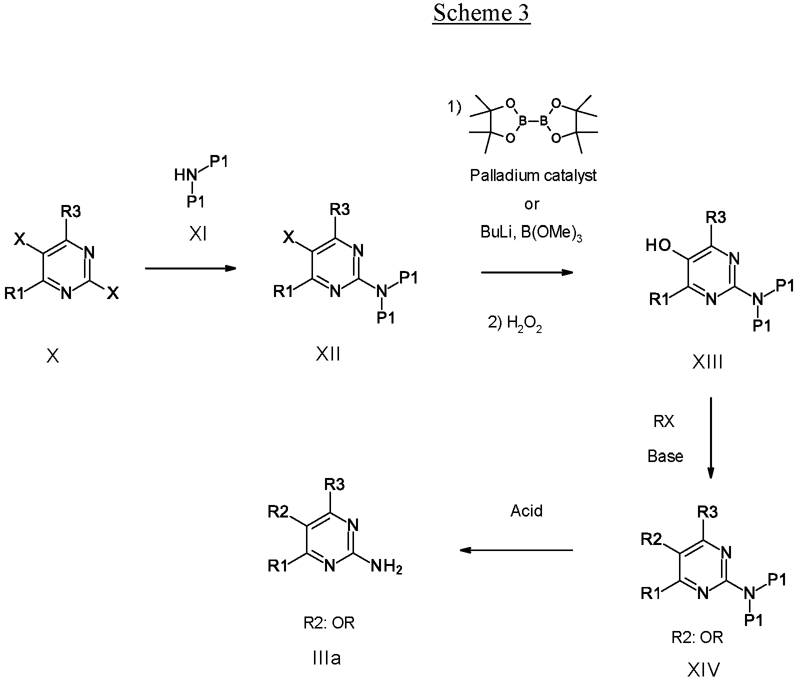

2-Amino-pyrimidine of formula IIIa wherein R² is an alkoxy group can be prepared by deprotection of intermediate XIV in the presence of an acid like trifluoro acetic acid wherein P1 is a protective group like p-methoxy-benzyl, 3-4-dimethoxybenzyl or a Boc group. XIV can be obtained by alkylation of alcohol XIII in the presence of a base like cesium- or potassium-carbonate or sodium- or potassium-hydroxide and an alkylating agent RX. Alcohol XIII can be prepared from dihalogenated starting material X, by reacting XI with a protected amine XI to provide intermediate XII which is first transformed into a boronic ester that is then oxidized in the presence of an oxidant like hydrogen peroxide.

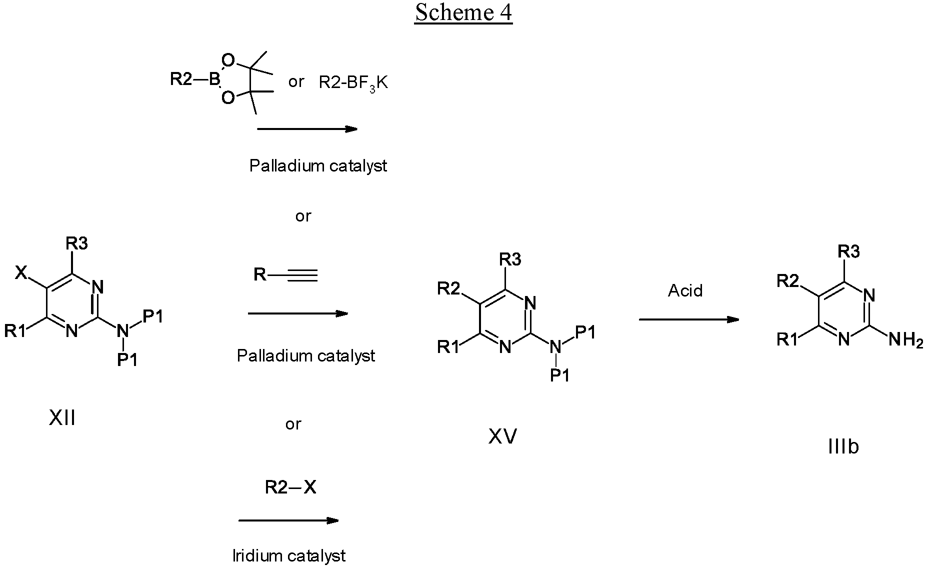

2-Amino-pyrimidine of formula IIIb wherein R² is an alkyl, alkenylalkyl, alkynyl, cyanoalkyl, cycloalkyl, heterocycloalkyl can be prepared by deprotection of intermediate XV in the presence of an acid like trifluoro acetic acid wherein P1 is a protective group like p-methoxy-benzyl or a Boc group. Compound XV can be obtained from intermediate XII under well known metal-catalyzed cross coupling reactions conditions.

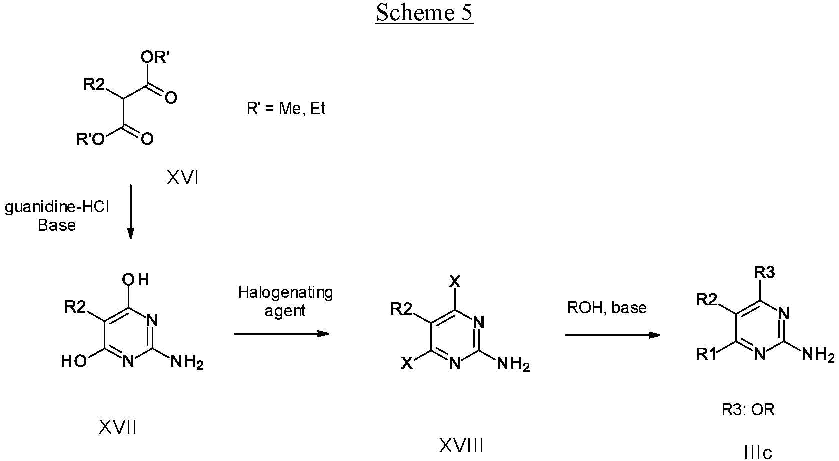

2-Amino-pyrimidine of formula IIIc wherein R1 and R3 is an alkoxy group can be prepared by reaction of halogenated starting material XVIII in the presence of an alcohol and a base like sodium hydride. Compound XVII can be prepared from malonester XVI by its reaction with guanidine-hydrochloride salt in the presence of a base like sodium methoxide to provide intermediate XVII which is then reacted with a halogenating agent like phosphorus oxychloride to give halogenated starting material XVIII.

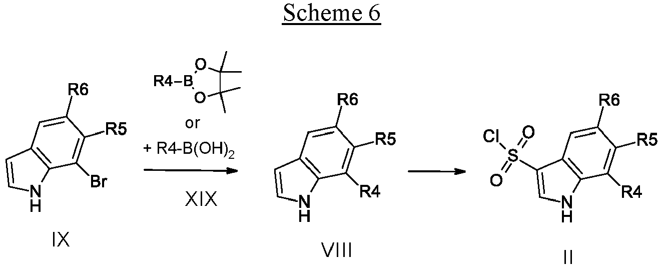

Furthermore, intermediates of formula II can be prepared by a reaction sequence as described in Scheme 6. Bromoindole IX can be reacted with boronic acid derivative XIX to obtain compound VIII using a suitable palladium catalyst and a base. Reaction of the resulting compound VIII with a) a chlorosulfonylating agent like chlorosulfonic acid, or b) a sulfonylating agent like sulfuric acid or sulfur trioxide N,N-dimethylformamide complex, followed by chlorination of the intermediate sulfonic acid with a chlorinating agent like thionylchloride provides compound II.

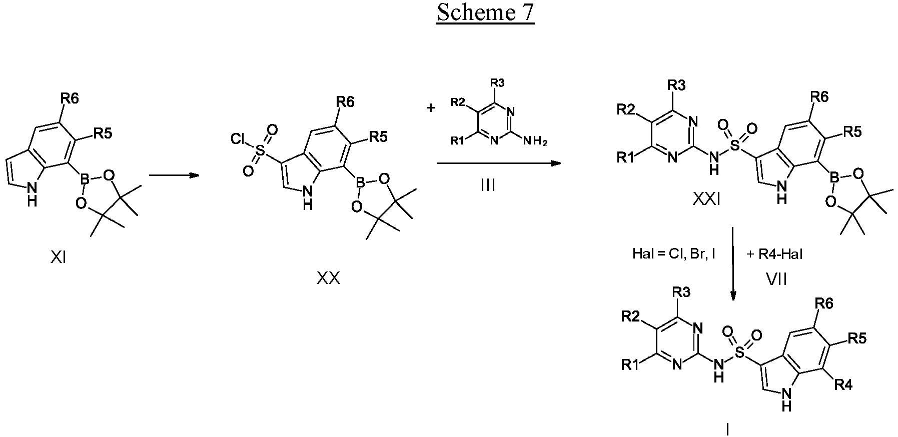

Furthermore, compounds of formula I can be prepared by a reaction sequence as described in Scheme 7. Intermediate XI can first be reacted with a chlorosulfonylating agent like chlorosulfonic acid to provide compound XX which is then coupled with amine III in presence of a suitable base to give compound XXI. Reaction of this intermediate with heteroaryl halide VII using a palladium catalyst and a base provides compounds of formula I.

Another embodiment of the invention provides a pharmaceutical composition or medicament containing a compound of the invention and a therapeutically inert carrier, diluent or excipient, as well as a method of using the compounds of the invention to prepare such composition and medicament. In one example, the compound of formula I may be formulated by mixing at ambient temperature at the appropriate pH, and at the desired degree of purity, with physiologically acceptable carriers, i.e., carriers that are non-toxic to recipients at the dosages and concentrations employed into a galenical administration form. The pH of the formulation depends mainly on the particular use and the concentration of compound, but preferably ranges anywhere from about 3 to about 8. In one example, a compound of formula I is formulated in an acetate buffer, at pH 5. In another embodiment, the compound of formula I is sterile. The compound may be stored, for example, as a solid or amorphous composition, as a lyophilized formulation or as an aqueous solution.

Compositions are formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners.

The compounds of the invention may be administered by any suitable means, including oral, topical (including buccal and sublingual), rectal, vaginal, transdermal, parenteral, subcutaneous, intraperitoneal, intrapulmonary, intradermal, intrathecal and epidural and intranasal, and, if desired for local treatment, intralesional administration. Parenteral infusions include intramuscular, intravenous, intraarterial, intraperitoneal, or subcutaneous administration.

The compounds of the present invention may be administered in any convenient administrative form, e.g., tablets, powders, capsules, solutions, dispersions, suspensions, syrups, sprays, suppositories, gels, emulsions, patches, etc. Such compositions may contain components conventional in pharmaceutical preparations, e.g., diluents, carriers, pH modifiers, sweeteners, bulking agents, and further active agents.

A typical formulation is prepared by mixing a compound of the present invention and a carrier or excipient. Suitable carriers and excipients are well known to those skilled in the art and are described in detail in, e.g., Ansel, Howard C., et al., Ansel's Pharmaceutical Dosage Forms and Drug Delivery Systems. Philadelphia: Lippincott, Williams & Wilkins, 2004; Gennaro, Alfonso R., et al. Remington: The Science and Practice of Pharmacy. Philadelphia: Lippincott, Williams & Wilkins, 2000; and Rowe, Raymond C. Handbook of Pharmaceutical Excipients. Chicago, Pharmaceutical Press, 2005. The formulations may also include one or more buffers, stabilizing agents, surfactants, wetting agents, lubricating agents, emulsifiers, suspending agents, preservatives, antioxidants, opaquing agents, glidants, processing aids, colorants, sweeteners, perfuming agents, flavoring agents, diluents and other known additives to provide an elegant presentation of the drug (i.e., a compound of the present invention or pharmaceutical composition thereof) or aid in the manufacturing of the pharmaceutical product (i.e., medicament).

The compounds of formula I and their pharmaceutically acceptable salts can be processed with pharmaceutically inert, inorganic or organic adjuvants for the production of tablets, coated tablets, dragées,hard gelatin capsules, injection solutions or topical formulations Lactose, corn starch or derivatives thereof, talc, stearic acid or its salts etc. can be used, for example, as such adjuvants for tablets, dragées and hard gelatin capsules.

Suitable adjuvants for soft gelatin capsules, are, for example, vegetable oils, waxes, fats, semi-solid substances and liquid polyols, etc.

Suitable adjuvants for the production of solutions and syrups are, for example, water, polyols, saccharose, invert sugar, glucose, etc.

Suitable adjuvants for injection solutions are, for example, water, alcohols, polyols, glycerol, vegetable oils, etc.

Suitable adjuvants for suppositories are, for example, natural or hardened oils, waxes, fats, semi-solid or liquid polyols, etc.

Suitable adjuvants for topical ocular formulations are, for example, cyclodextrins, mannitol or many other carriers and excipients known in the art.

Moreover, the pharmaceutical preparations can contain preservatives, solubilizers, viscosity-increasing substances, stabilizers, wetting agents, emulsifiers, sweeteners, colorants, flavorants, salts for varying the osmotic pressure, buffers, masking agents or antioxidants. They can also contain still other therapeutically valuable substances.

The dosage can vary in wide limits and will, of course, be fitted to the individual requirements in each particular case. In general, in the case of oral administration a daily dosage of about 0.1 mg to 20 mg per kg body weight, preferably about 0.5 mg to 4 mg per kg body weight (e.g. about 300 mg per person), divided into preferably 1-3 individual doses, which can consist, for example, of the same amounts, should it be appropriate. In the case of topical administration, the formulation can contain 0.001% to 15% by weight of medicament and the required dose, which can be between 0.1 and 25 mg in can be administered either by single dose per day or per week, or by multiple doses (2 to 4) per day, or by multiple doses per week It will, however, be clear that the upper or lower limit given herein can be exceeded when this is shown to be indicated.

The invention also relates in particular to:
A compound of formula I for use as therapeutically active substance;
A compound of formula I for use in the treatment of a disease modulated by GPR17;
Likewise an object of the present invention is a pharmaceutical composition comprising a compound according to formula I as described herein and a therapeutically inert carrier.

The use of a compound of formula I for the treatment or prophylaxis of conditions resulting from direct damage to myelin sheaths (including but not limited central pontine and extra-pontine myelinolysis, carbon monoxide poisoning, nutritional deficiency, and virus-induced demyelination), demyelinating disorders (including but not limited to multiple sclerosis, acute and multiphasic disseminated encephalomyelitis, neuromyelitis optica spectrum disorders, and leukodystrophies), CNS disorders associated with myelin loss (including but not limited to Alzheimer's disease, schizophrenia, Parkinson's disease, Huntington's disease, Amyotrophic lateral sclerosis, and Ischemia due to stroke), and Inflammation in the CNS for instance following encephalitis, primary angiitis, meningitis and obesity.

An embodiment of the present invention is the use of a compound of formula I for the treatment or prophylaxis of multiple sclerosis, Alzheimer's disease, Parkinson's disease, or Huntington's disease.

A particular embodiment of the invention is the use of a compound of formula I for the treatment or prophylaxis of multiple sclerosis.

The use of a compound of formula I for the preparation of a medicament for the treatment or prophylaxis of conditions resulting from direct damage to myelin sheaths (including but not limited central pontine and extra-pontine myelinolysis, carbon monoxide poisoning, nutritional deficiency, and virus-induced demyelination), demyelinating disorders (including but not limited to multiple sclerosis, acute and multiphasic disseminated encephalomyelitis, neuromyelitis optica spectrum disorders, and leukodystrophies), CNS disorders associated with myelin loss (including but not limited to Alzheimer's disease, schizophrenia, Parkinson's disease, Huntington's disease, Amyotrophic lateral sclerosis, and Ischemia due to stroke), and Inflammation in the CNS for instance following encephalitis, primary angiitis, meningitis and obesity.

An embodiment of the present invention is the use of a compound of formula I for the preparation of a medicament for the treatment or prophylaxis of multiple sclerosis, Alzheimer's disease, Parkinson's disease, or Huntington's disease.

A particular embodiment of the invention is the use of a compound of formula I for the preparation of a medicament for the treatment or prophylaxis of multiple sclerosis.

A compound according to formula I for use in the treatment or prophylaxis of conditions resulting from direct damage to myelin sheaths (including but not limited central pontine and extra-pontine myelinolysis, carbon monoxide poisoning, nutritional deficiency, and virus-induced demyelination), demyelinating disorders (including but not limited to multiple sclerosis, acute and multiphasic disseminated encephalomyelitis, neuromyelitis optica spectrum disorders, and leukodystrophies), CNS disorders associated with myelin loss (including but not limited to Alzheimer's disease, schizophrenia, Parkinson's disease, Huntington's disease, Amyotrophic lateral sclerosis, and Ischemia due to stroke), and Inflammation in the CNS for instance following encephalitis, primary angiitis, meningitis and obesity.

An embodiment of the present invention is a compound of formula I for use in the treatment or prophylaxis of multiple sclerosis, Alzheimer's disease, Parkinson's disease, or Huntington's disease.

A particular embodiment of the invention is a compound according to formula I for use in the treatment or prophylaxis of multiple sclerosis.

A method for the treatment or propylaxis of conditions resulting from direct damage to myelin sheaths (including but not limited central pontine and extra-pontine myelinolysis, carbon monoxide poisoning, nutritional deficiency, and virus-induced demyelination), demyelinating disorders (including but not limited to multiple sclerosis, acute and multiphasic disseminated encephalomyelitis, neuromyelitis optica spectrum disorders, and leukodystrophies), CNS disorders associated with myelin loss (including but not limited to Alzheimer's disease, schizophrenia, Parkinson's disease, Huntington's disease, Amyotrophic lateral sclerosis, and Ischemia due to stroke), and Inflammation in the CNS for instance following encephalitis, primary angiitis, meningitis and obesity, which method comprises administering an effective amount of a compound of formula I to a patient in need thereof.

An embodiment of the present invention is a method for the treatment or prophylaxis of multiple sclerosis, Alzheimer's disease, Parkinson's disease, or Huntington's disease, which method comprises administering an effective amount of a compound of formula I to a patient in need thereof.

A particular embodiment of the invention is a method for the treatment or prophylaxis of multiple sclerosis, which method comprises administering an effective amount of a compound of formula I to a patient in need thereof.

Also an embodiment of the present invention provides compounds of formula I as described herein, when manufactured according to any one of the described processes.

### Assay Procedures

### GPR17 cAMP Assay Protocol:

CHO-K1 cells stably expressing vector containing untagged human GPR17 short isoform (Roche) were cultured at 37°C / 5% CO2 in DMEM (Dulbecco's Modified Eagle Medium):F-12 (1:1) supplemented with 10% foetal bovine serum and 400 µg/ml Geneticin.

Changes in intracellular cyclic adenosine monophosphate (cAMP) levels were quantified using the Nano-TRF Detection Assay kit (Roche Diagnostics, Cat. No. 05214386001). This assay allows for direct cAMP quantification in a homogeneous solution. cAMP is detected based on time-resolved fluorescence energy transfer (TR-FRET) and competitive binding of ruthenylated cAMP and endogenous cAMP to an anti-cAMP monoclonal antibody labeled with AlexaFluor-700. The Ruthenium complex serves as the FRET donor and transfers energy to AlexaFluor-700. The FRET signal is inversely proportional to the cAMP concentration.

CHO-GPR17S cells were detached with Accutase and resuspended in assay buffer consisting of Hank's Balanced Salt Solution (HBSS), 10mM HEPES (4-(2-hydroxyethyl) piperazine-1-ethanesulfonic acid solution) and 0.1% bovine serum albumin (pH 7.4). The cells were seeded in black 384-well plates (Corning) at a density of 10'000 cells / 20µl assay buffer until the addition of compounds.

Test antagonist compounds were serially diluted in dimethyl sulfoxide (DMSO) and spotted in 384-well plates. The compounds were then diluted in HBSS buffer supplemented with an EC80 concentration of MDL29,951 (3-(2-Carboxy-4,6-dichloroindol-3-yl)propionic acid) (GPR17 agonist) plus 3-Isobutyl-1-methylxanthine (IBMX) (0.5mM final concentration) and added to the cells at room temperature. Forskolin (15µM final concentration) was added 5 minutes after the test compounds and the cells were incubated at room temperature for 30 minutes. The assay was stopped by adding cAMP detection mix (containing detergents for cell lysis) for 90 minutes at room temperature.

Cellular cAMP was measured using a Paradigm reader (Molecular Devices). The raw data was used to calculate the FRET signal based on the assay's P-factor as per cAMP kit instructions. The data was normalized to the maximal activity of a reference antagonist and dose response curves were fitted to the percent activity of the test compounds using a sigmoidal dose response model (Genedata Screener).

Results in the hGPR17 cAMP assay are provided for compounds of formula I in Table 1

**Table 1:**

| **Example number** | **hGPR17 cAMP IC50 [µM]** |
|---|---|
| 1 | 0.048 |
| 2 | 0.098 |
| 3 | 0.046 |
| 4 | 0.044 |
| 5 | 0.026 |
| 6 | 0.042 |
| 7 | 0.034 |
| 8 | 0.039 |
| 9 | 0.069 |
| 10 | 0.030 |
| 11 | 0.050 |
| 12 | 0.085 |
| 13 | 0.057 |
| 14 | 0.032 |
| 15 | 0.027 |
| 16 | 0.025 |
| 17 | 0.806 |
| 18 | 0.019 |
| 19 | 0.018 |
| 20 | 0.032 |
| 21 | 0.040 |
| 22 | 0.080 |
| 23 | 0.050 |
| 24 | 0.061 |
| 25 | 0.056 |
| 26 | 0.218 |
| 27 | 0.120 |
| 28 | 0.307 |
| 29 | 0.604 |
| 30 | 0.130 |
| 31 | 0.084 |
| 32 | 0.129 |
| 33 | 0.132 |
| 34 | 0.407 |
| 35 | 0.034 |
| 36 | 0.042 |
| 37 | 0.067 |
| 38 | 0.054 |
| 39 | 0.017 |
| 40 | 0.009 |
| 41 | 0.015 |
| 42 | 0.038 |
| 43 | 0.032 |
| 44 | 0.182 |
| 45 | 0.050 |
| 46 | 0.034 |
| 47 | 0.017 |
| 48 | 0.086 |
| 49 | 0.301 |
| 50 | 0.288 |
| 51 | 0.074 |
| 52 | 0.022 |
| 53 | 0.043 |
| 54 | 0.064 |
| 55 | 0.157 |
| 56 | 0.129 |
| 57 | 0.109 |
| 58 | 0.051 |
| 59 | 0.068 |
| 60 | 0.121 |
| 61 | 0.023 |
| 62 | 0.037 |
| 63 | 0.045 |
| 64 | 0.035 |
| 65 | 0.039 |
| 66 | 0.055 |
| 67 | 0.024 |
| 68 | 0.018 |
| 69 | 0.056 |
| 70 | 0.102 |
| 71 | 0.099 |
| 72 | 1.416 |
| 73 | 0.037 |
| 74 | 0.019 |
| 75 | 0.135 |
| 76 | 0.021 |
| 77 | 0.024 |
| 78 | 0.122 |
| 79 | 0.059 |
| 80 | 0.260 |
| 81 | 0.031 |
| 82 | 0.047 |
| 83 | 0.097 |
| 84 | 0.052 |
| 85 | 0.044 |
| 86 | 0.029 |
| 87 | 0.031 |
| 88 | 0.027 |
| 89 | 0.042 |
| 90 | 0.261 |
| 91 | 0.225 |
| 92 | 0.028 |
| 93 | 0.024 |
| 94 | 0.020 |
| 95 | 0.046 |
| 96 | 0.032 |
| 97 | 0.034 |
| 98 | 0.035 |
| 99 | 0.038 |
| 100 | 0.040 |
| 101 | 0.043 |
| 102 | 0.042 |
| 103 | 0.054 |
| 104 | 0.113 |
| 105 | 0.221 |
| 106 | 0.034 |
| 107 | 0.076 |
| 108 | 0.032 |
| 109 | 0.036 |
| 110 | 0.046 |
| 111 | 0.053 |
| 112 | 0.052 |
| 113 | 0.082 |
| 114 | 0.055 |
| 115 | 0.040 |
| 116 | 0.059 |
| 117 | 0.106 |
| 118 | 0.063 |
| 119 | 0.129 |
| 120 | 0.149 |
| 121 | 0.046 |

The invention will now be illustrated by the following examples which have no limiting character.

In case the preparative examples are obtained as a mixture of enantiomers, the pure enantiomers can be obtained by methods described herein or by methods known to those skilled in the art, such as e.g. chiral chromatography or crystallization.

### Examples

All examples and intermediates were prepared under nitrogen atmosphere if not specified otherwise.

### Intermediates A

### Intermediate A1: 6-chloro-7-pyrimidin-2-yl-1H-indole-3-sulfonyl chloride

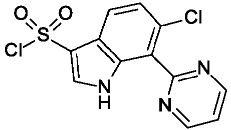

### Step 1: 1-(6-chloroindol-1-yl)-2,2-dimethyl-propan-1-one

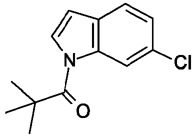

6-Chloro-1H-indole (3.0 g, 19.8 mmol), triethylamine (3.0 g, 4.15 ml, 30 mmol) and 4-dimethylaminopyridine (242 mg, 1.98 mmol) was dissolved in dichloromethane (60 ml). Under argon, pivaloyl chloride (2.86 g, 2.9 ml, 23.8 mmol) was added at 0°C within 7 min. The solution was stirred for about 10 min and was then allowed to warm to room temperature. After 2.5 h the suspension was poured into water and extracted twice with dichloromethane. The combined organic layers were dried over sodium sulfate, filtered and concentrated in vacuo. The residue was purified by flash chromatography (silica gel, 5% ethyl acetate in heptane) to give 1-(6-chloroindol-1-yl)-2,2-dimethyl-propan-1-one (4.26 g, 91% yield) as a white solid. MS (ESI) m/z: 236.2 [M+H]⁺

### Step 2: 6-chloro-7-(4,4,5,5-tetramethyl-1,3,2-dioxaborolan-2-yl)-1H-indole

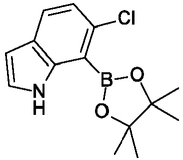

To a solution of 1-(6-chloroindol-1-yl)-2,2-dimethyl-propan-1-one (5.0 g, 20.2 mmol) in dichloromethane (55 ml), under argon, was added boron tribromide solution in dichloromethane (1 M, 58.7 g, 22.2 ml, 22.2 mmol) dropwise within 20 min at room temperature. The resulting suspension was stirred at room temperature for 1 h. After cooling to 0°C a solution of pinacol (4.76 g, 40.3 mmol) and pyridine (16 g, 16.3 ml, 202 mmol) in dichloromethane (25 ml) was added within 15 min. The yellow solution was allowed to warm to room temperature, then heated to 40°C for 2 h and stirred again at room temperature overnight. The mixture was concentrated in vacuo, then methanol (70 ml) and triethylamine (10.2 g, 14 ml, 100.8 mmol) were added. The orange solution was stirred at 60°C for 7 h to remove the pivaloyl group. The mixture was concentrated in vacuo, tetrahydrofuran (70 ml), pinacol (2.38 g, 20.15 mmol) and magnesium sulfate (3 spatula) were added and the suspension was stirred at room temperature overnight. Then the mixture was distributed between water and ethyl acetate. The combined organic layers were dried over sodium sulfate, filtered and concentrated in vacuo. The residue was purified by flash chromatography (silica gel, 5% ethyl acetate in heptane) to give 6-chloro-7-(4,4,5,5-tetramethyl-1,3,2-dioxaborolan-2-yl)-1H-indole (1.42 g, 25% yield) as a white solid. MS (ESI) m/z: 278.2 [M+H]⁺

### Step 3: 6-chloro-7-pyrimidin-2-yl-1H-indole

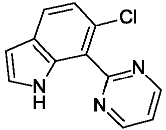

A mixture of 6-chloro-7-(4,4,5,5-tetramethyl-1,3,2-dioxaborolan-2-yl)-1H-indole (500 mg, 1.64 mmol), cesium carbonate (1.08 g, 3.28 mmol), tetrakis(triphenylphosphine)palladium(0) (189 mg, 164 umol) and 2-bromopyrimidine (313 mg, 1.97 mmol) in 1,4-dioxane (10 ml) and water (2 ml) was heated in a microwave to 100°C for 30 min. The reaction mixture was poured into water and extracted twice with ethyl acetate. The combined organic layers were dried over sodium sulfate, filtered and concentrated in vacuo. The residue was purified by flash chromatography (silica gel, 0% to 60% ethyl acetate in heptane) to provide 6-chloro-7-(2-pyrimidyl)-1H-indole (375 mg, 99% yield) as light yellow solid. MS (ESI) m/z: 230.1 [M+H]⁺

### Step 4: 6-chloro-7-pyrimidin-2-yl-1H-indole-3-sulfonyl chloride

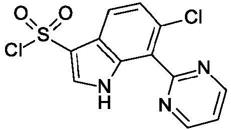

A solution of 6-chloro-7-(2-pyrimidyl)-1H-indole (374 mg, 1.63 mmol) in acetonitrile (10 ml) was cooled to 0 °C. Chlorosulfonic acid (493 mg, 284 ul, 4.23 mmol) was added and the reaction mixture was stirred at 0 °C for 30 min. Then phosphorus oxychloride (1.25 g, 759 ul, 8.14 mmol) was added and the reaction mixture was stirred at 70°C for 16 h. The mixture was poured into ice/ethyl acetate and extracted twice with ethyl acetate. The combined organic layers were washed with brine, dried over sodium sulfate, filtered and concentrated in vacuo. The residue was triturated with ethyl acetate, filtered through sintered glass and dried in vacuo to provide the title compound (321 mg, 60% yield) as white solid. MS (ESI) m/z: 328.0 [M+H]⁺

### Intermediate A2: 6-chloro-7-oxazol-2-yl-1H-indole-3-sulfonyl chloride

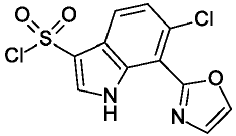

### Step 1: 2-(6-chloro-1H-indol-7-yl)oxazole

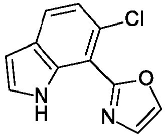

A mixture of 6-chloro-7-(4,4,5,5-tetramethyl-1,3,2-dioxaborolan-2-yl)-1H-indole (see Intermediate A1, 100 mg, 353 umol), potassium carbonate (99 mg, 706 umol), 1,1'-bis(diphenylphosphino)ferrocene dichloropalladium(II) (27 mg, 35 umol, 0.100 eq) and 2-bromooxazole (82.5 mg, 530 umol) in 1,4-dioxane (1.6 ml) and water (0.4 ml) was heated in a microwave at 110 °C for 30 min. The reaction mixture was poured into water and extracted with ethyl acetate twice. The organic layers were dried over sodium sulfate, filtered and concentrated in vacuo. The residue was purified by flash chromatography (silica gel (0% to 10% ethyl acetate) to provide 2-(6-chloro-1H-indol-7-yl)oxazole (47 mg, 61% yield) as white solid. MS (ESI) m/z: 219.1 [M+H]⁺

### Step 2: 6-chloro-7-pyrimidin-2-yl-1H-indole-3-sulfonyl chloride

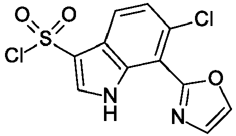

A solution of 2-(6-chloro-1H-indol-7-yl)oxazole (52 mg, 209 umol) in acetonitrile (1.5 ml) was cooled to 0 °C. Chlorosulfonic acid (63 mg, 36 ul, 544 umol) was added and the reaction mixture was stirred at 0 °C for 1 h at room temperature. Then phosphoryl chloride (160 mg, 98 ul, 1.05 mmol) was added. The reaction mixture was heated to 70 °C for 16 h. The mixture was poured into ice and ethyl acetate and extracted with ethyl acetate twice. The combined organic layers were dried over sodium sulfate, filtered and concentrated in vacuo to provide the title compound (61 mg, 92% yield) as off-white solid. MS (ESI) m/z: 316.9 [M+H]⁺

### Intermediate A3: 6-chloro-7-pyridazin-3-yl-1H-indole-3-sulfonyl chloride

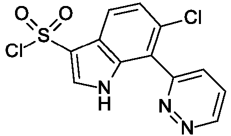

### Step 1: 6-chloro-7-pyridazin-3-yl-1H-indole

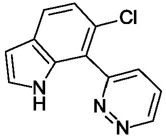

A mixture of 6-chloro-7-(4,4,5,5-tetramethyl-1,3,2-dioxaborolan-2-yl)-1H-indole (see, Intermediate A1, 110 mg, 388 umol), 1,1'-bis(diphenylphosphino)ferrocene-palladium(II) dichloride dichloromethane complex (32 mg, 39 umol, 0.1 eq), potassium carbonate (217 mg, 1.55 mmol) and 3-chloropyridazine (56 mg, 466 umol) in 1,4-dioxane (2.2 ml) and water (1.1 ml) was heated in microwave at 90 °C for 30 min. The reaction mixture was poured into water and extracted with ethyl acetate twice. The combined organic layers were dried over sodium sulfate, filtered and concentrated in vacuo. The residue was purified by flash chromatography (silica gel, 0% to 60% ethyl acetate in heptane) to provide 6-chloro-7-pyridazin-3-yl-1H-indole (69 mg, 73% yield) as light yellow solid. MS (ESI) m/z: 230.0 [M+H]⁺

### Step 2: 6-chloro-7-pyrimidin-2-yl-1H-indole-3-sulfonyl chloride

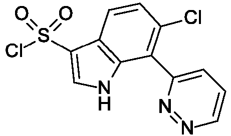

A suspension of 6-chloro-7-pyridazin-3-yl-1H-indole (68 mg, 278 umol) in acetonitrile (2 ml) was cooled to 0 °C. Chlorosulfonic acid (84 mg, 48 ul, 723 umol) was added and the reaction mixture was stirred at 0 °C for 1 h, then phosphorus oxychloride (213 mg, 130 ul, 1.39 mmol) was added. The reaction mixture was heated to 70 °C and stirred for 17 h. The mixture was poured into ice and ethyl acetate and extracted with ethyl acetate twice. The combined organic layers were dried over sodium sulfate, filtered and concentrated in vacuo to provide the title compound (90 mg, 99% yield) as light brown solid. MS (ESI) m/z: 327.9 [M+H]⁺

### Intermediate A4: 7-(1-methylimidazol-2-yl)-1H-indole-3-sulfonyl chloride

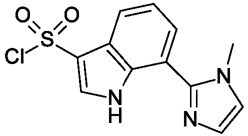

The title compound was prepared in analogy to Intermediate A1 from 2-iodo-1-methyl-imidazole instead of 2-bromopyrimidine and 7-(4,4,5,5-tetramethyl-1,3,2-dioxaborolan-2-yl)-1H-indole (CAS 642494-37-9) instead of 6-chloro-7-(4,4,5,5-tetramethyl-1,3,2-dioxaborolan-2-yl)-1H-indole in Step 3) as an orange semisolid. MS (ESI) m/z: 296.0 [M+H]⁺

### Intermediate A5: 6-chloro-7-(2-pyridyl)-1H-indole-3-sulfonyl chloride

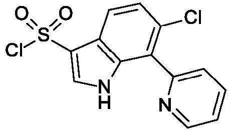

The title compound was prepared in analogy to Intermediate A1 from 2-bromopyridine instead of 2-bromopyrimidine in Step 3) as a off-white solid. MS (ESI) m/z: 325.0 [M-H]⁻

### Intermediate A6: 6-chloro-7-(1-methylimidazol-2-yl)-1H-indole-3-sulfonyl chloride

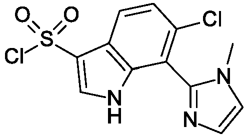

The title compound was prepared in analogy to Intermediate A1 from 2-iodo-1-methyl-imidazole instead of 2-bromopyrimidine in Step 3) as off-white solid. MS (ESI) m/z: 330.0 [M+H]⁺

### Intermediate A7: 7-pyridazin-3-yl-1H-indole-3-sulfonyl chloride

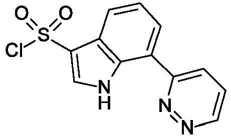

The title compound was prepared in analogy to Intermediate A3 from 7-(4,4,5,5-tetramethyl-1,3,2-dioxaborolan-2-yl)-1H-indole (CAS 642494-37-9) instead of 6-chloro-7-(4,4,5,5-tetramethyl-1,3,2-dioxaborolan-2-yl)-1H-indole as an off-white solid. MS (ESI) m/z: 294.1 [M+H]⁺

### Intermediate A8: 7-(4-methylpyridazin-3-yl)-1H-indole-3-sulfonyl chloride

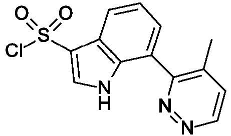

The title compound was prepared in analogy to Intermediate A3 from 3-chloro-4-methylpyridazine instead of 3-chloro-pyridazine and 7-(4,4,5,5-tetramethyl-1,3,2-dioxaborolan-2-yl)-1H-indole (CAS 642494-37-9) instead of 6-chloro-7-(4,4,5,5-tetramethyl-1,3,2-dioxaborolan-2-yl)-1H-indole as a light brown solid. MS (ESI) m/z: 308.1 [M+H]⁺

### Intermediate A9: 6-chloro-7-oxazol-4-yl-1H-indole-3-sulfonyl chloride

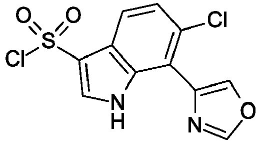

### Step 1: 4-(6-chloro-1H-indol-7-yl)oxazole

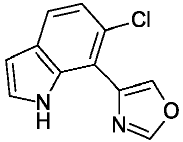

A mixture of 6-chloro-7-(4,4,5,5-tetramethyl-1,3,2-dioxaborolan-2-yl)-1H-indole (see Intermediate A1, 400 mg, 1.31 mmol), cesium carbonate (1.29 g, 3.93 mmol), XPhos Pd G2 (CAS 1310584-14-5, 103 mg, 131 umol, 0.10 eq) and 4-bromooxazole (245 mg, 1.57 mmol) in 1,4-dioxane (3 ml) and water (1.5 ml) was heated in a microwave at 90 °C for 30 min. The reaction mixture was poured into brine and extracted with ethyl acetate twice. The organic layers were dried over sodium sulfate, filtered and concentrated in vacuo. The residue was purified by flash chromatography (silica gel, 0% to 30% ethyl acetate in heptan) to provide 4-(6-chloro-1H-indol-7-yl)oxazole (40 mg, 14%) as light yellow solid. MS (ESI) m/z: 219.0 [M+H]⁺

### Step 2: 6-chloro-7-oxazol-4-yl-1H-indole-3-sulfonyl chloride

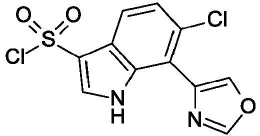

A solution of 4-(6-chloro-1H-indol-7-yl)oxazole (39 mg, 178 umol) in acetonitrile (1.5 ml) was cooled to 0 °C. Chlorosulfonic acid (54 mg, 31 uL, 464 umol) was added. The reaction mixture was stirred at 0 °C for 30 min and at room temperature for 3 days. Then phosphorus oxychloride (137 mg, 83 ul, 892 umol) was added. The reaction mixture was heated to 70 °C and stirred for 10 h, then it was poured into ice and ethyl acetate and extracted wit ethyl acetate twice. The combined organic layers were washed with brine, dried over sodium sulfate, filtered and concentrated in vacuo to provide the title compound (55 mg, 97% yield) as light brown solid. MS (ESI) m/z: 315.0 [M-H]⁻

### Intermediate A10: 6-chloro-7-pyrazin-2-yl-1H-indole-3-sulfonyl chloride

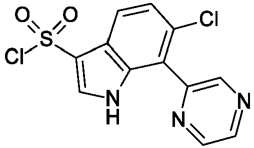

The title compound was prepared in analogy to Intermediate A1 from 2-bromopyrazine instead of 2-bromopyrimidine in Step 3) s light yellow solid. MS (ESI) m/z: 328.0 [M+H]⁺

### Intermediate A11: 7-pyrimidin-2-yl-1H-indole-3-sulfonyl chloride

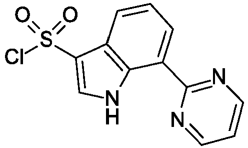

The title compound was prepared in analogy to Intermediate A1 from 7-(4,4,5,5-tetramethyl-1,3,2-dioxaborolan-2-yl)-1H-indole (CAS 642494-37-9) instead of 6-chloro-7-(4,4,5,5-tetramethyl-1,3,2-dioxaborolan-2-yl)-1H-indole in Step 3) as light yellow solid. MS (ESI) m/z: 294.0 [M+H]⁺

### Intermediate A12: 7-pyrazin-2-yl-1H-indole-3-sulfonyl chloride

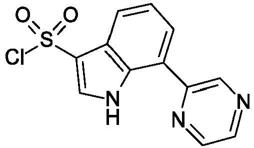

The title compound was prepared in analogy to Intermediate A1 from 2-bromopyrazine instead of 2-bromopyrimidine and 7-(4,4,5,5-tetramethyl-1,3,2-dioxaborolan-2-yl)-1H-indole (CAS 642494-37-9) instead of 6-chloro-7-(4,4,5,5-tetramethyl-1,3,2-dioxaborolan-2-yl)-1H-indole in Step 3) as yellow solid. MS (ESI) m/z: 294.1 [M+H]⁺

### Intermediate A13: 6-fluoro-7-pyrimidin-2-yl-1H-indole-3-sulfonyl chloride

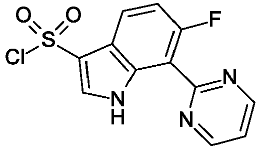

The title compound was prepared in analogy to Intermediate A1 from 6-fluoro-1H-indole instead of 6-chloro-1H-indole as light yellow solid. MS (ESI) m/z: 312.1 [M+H]⁺

### Intermediate A14: 6-fluoro-7-(1-methylimidazol-2-yl)-1H-indole-3-sulfonyl chloride

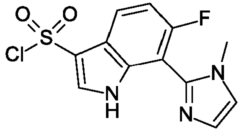

The title compound was prepared in analogy to Intermediate A1 from 6-fluoro-1H-indole instead of 6-chloro-1H-indole in Step 1) and from 2-iodo-1-methyl-imidazole instead of 2-bromopyrimidine in Step 3) as light brown oil which was directly used in the next step.

### Intermediate A15: 6-fluoro-7-pyrazin-2-yl-1H-indole-3-sulfonyl chloride

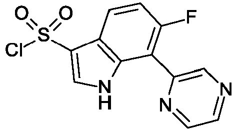

The title compound was prepared in analogy to Intermediate A1 from 6-fluoro-1H-indole instead of 6-chloro-1H-indole in Step 1) and from 2-bromopyrazine instead of 2-bromopyrimidine in Step 3) as yellow solid, MS (ESI) m/z: 312.1 [M+H]⁺

### Intermediate A16: 6-bromo-7-pyrimidin-2-yl-1H-indole-3-sulfonyl chloride

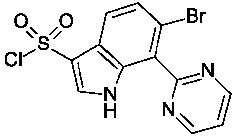

The title compound was prepared in analogy to Intermediate A1 from 6-bromo-1H-indole instead of 6-chloro-1H-indole in Step 1) as yellow solid, MS (ESI) m/z: 374.0 [M+H]⁺

### Intermediate A17: 6-bromo-7-pyrazin-2-yl-1H-indole-3-sulfonyl chloride

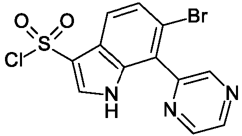

The title compound was prepared in analogy to Intermediate A1 from 6-bromo-1H-indole instead of 6-chloro-1H-indole in Step 1) and from 2-bromopyrazine instead of 2-bromopyrimidine in Step 3) as orange solid, MS (ESI) m/z: 374.0 [M+H]⁺

### Intermediate A18: 6-chloro-7-(5-fluoropyrazin-2-yl)-1H-indole-3-sulfonyl chloride

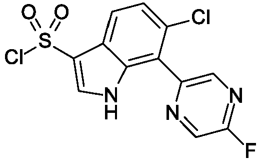

The title compound was prepared in analogy to Intermediate A1 from 2-bromo-5-fluoro-pyrazine instead of 2-bromopyrimidine in Step 3) as light brown solid, MS (ESI) m/z: 344.0 [M-H]⁻

### Intermediate A19: 6-bromo-7-pyridazin-3-yl-1H-indole-3-sulfonyl chloride

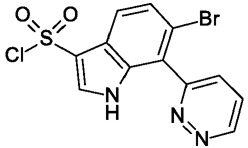

The title compound was prepared in analogy to Intermediate A1 from 6-bromo-1H-indole instead of 6-chloro-1H-indole in Step 1) and from 3-chloropyridazine instead of 2-bromopyrimidine in Step 3) as brown gum, MS (ESI) m/z: 374.0 [M+H]⁺

### Intermediate A20: 6-(difluoromethyl)-7-pyrimidin-2-yl-1H-indole-3-sulfonyl chloride

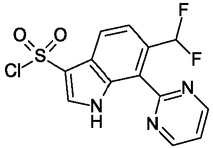

### Step 1: 7-bromo-6-(difluoromethyl)-1H-indole

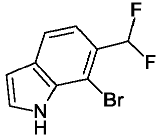

To the stirred solution of 2-bromo-1-(difluoromethyl)-3-nitro-benzene (CAS 1261819-77-5, 24.6 g, 97.6 mmol) in dry tetrahydrofuran (1000 ml) at -40°C was added vinylmagnesium bromide (1M solution in THF, 488 mL, 488 mmol) and mixture was stirred at -40°C for further 30 min. The mixture was quenched with ammonium chloride solution (500 ml) and extracted with ethyl acetate twice. The combined organic layers were washed with brine (300 ml), dried over sodium sulfate and evaporated under reduced pressure. The residue was purified by chromatography (silica gel, 0-30 % ethyl acetate in heptane) to afford 7-bromo-6-(difluoromethyl)-1H-indole (10.2 g, 42% yield) as light brown solid. MS (ESI) m/z: 245.8 [M-H]⁻

### Step 2: 6-(difluoromethyl)-7-(4,4,5,5-tetramethyl-1,3,2-dioxaborolan-2-yl)-1H-indole

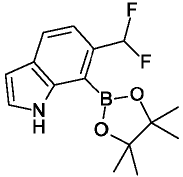

A mixture of 7-bromo-6-(difluoromethyl)-1H-indole (2 g, 8.12 mmol), bis(pinacolato)diboron (3.10 g, 12.2 mmol), potassium acetate (2.39 g, 24.4 mmol) and XPhos Pd(crotyl)Cl (CAS 1798782-02-1, 273 mg, 0.41 mmol, 5 mol%) in methanol (70 ml) was stirred and heated to 40°C for 4 h. After cooling the mixture was filtered, the solid washed with methanol and the solution was concentrated in vacuo. The crude material was purified by flash chromatography (silica gel, 0 % to 30 % ethyl acetate in heptane) to afford 6-(difluoromethyl)-7-(4,4,5,5-tetramethyl-1,3,2-dioxaborolan-2-yl)-1H-indole (1.2 g, 50% yield) as dark brown oil. MS (ESI) m/z: 294.2 [M+H]⁺

### Step 3: 6-(difluoromethyl)-7-pyrimidin-2-yl-1H-indole

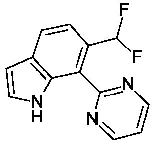

A mixture of 6-(difluoromethyl)-7-(4,4,5,5-tetramethyl-1,3,2-dioxaborolan-2-yl)-1H-indole (729 mg, 2.49 mmol), 2-bromopyrimidine (475 mg, 2.98 mmol), tetrakis(triphenylphosphine)palladium(0) (288 mg, 249 umol, 0.1 eq) and cesium carbonate (1.62 g, 4.97 mmol) in 1,4-dioxane (10 ml) and water (2.5 ml) was heated in microwave at 100 °C for 30 min. The mixture was poured into water and extracted with ethyl acetate twice. The combined organic layers were dried over sodium sulfate, filtered and concentrated in vacuo. The residue was purified by flash chromatography (SiliaSep Amine silicagel, 0% to 80% ethyl acetate in heptane) to provide 6-(difluoromethyl)-7-pyrimidin-2-yl-1H-indole (193 mg, 32% yield) as white semisolid. MS (ESI) m/z: 246.1 [M+H]⁺

### Step 4: 6-(difluoromethyl)-7-pyrimidin-2-yl-1H-indole-3-sulfonyl chloride

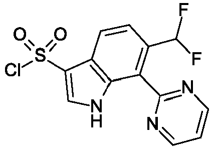

To a solution of 6-(difluoromethyl)-7-(2-pyrimidyl)-1H-indole (195 mg, 795 umol) in acetonitrile (5 ml) was added chlorosulfonic acid (139 mg, 80 ul, 1.19 mmol) at 0°C. The ice bath was removed and the reaction mixture was stirred 2 h at room temperature. Phosphorus oxychloride (488 mg, 297 ul, 3.18 mmol) was added and the reaction mixture was stirred at 70°C overnight. The mixture was poured into ice/water and extracted with ethyl acetate twice. The combined organic layers were washed with ice and brine, dried over Na2SO4, filtered and concentrated in vacuo to the title compound (207 mg, 76% yield) as brown solid. MS (ESI) m/z: 344.1 [M+H]⁺

### Intermediate A21: 6-fluoro-7-(1H-imidazol-2-yl)-1H-indole-3-sulfonyl chloride

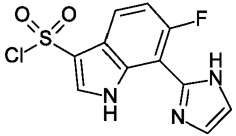

The title compound was prepared in analogy to Intermediate A1 from 6-fluoro-1H-indole instead of 6-chloro-1H-indole in Step 1) and from 2-iodoimidazole instead of 2-bromopyrimidine in Step 3) as light brown solid which was used directly for the next step.

### Intermediate A22: 6-methyl-7-pyrimidin-2-yl-1H-indole-3-sulfonyl chloride

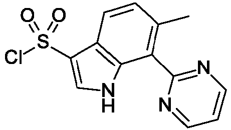

The title compound was prepared in analogy to Intermediate A1 from 6-methyl-1H-indole instead of 6-chloro-1H-indole in Step 1) as light brown foam. MS (ESI) m/z: 308.1 [M+H]⁺.

### Intermediate A23: 6-chloro-7-phenyl-1H-indole-3-sulfonyl chloride

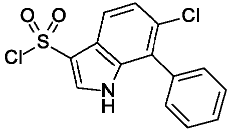

### Step 1: 6-chloro-7-phenyl-1H-indole

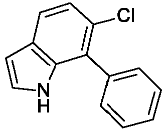

A mixture of 7-bromo-6-chloro-1H-indole (CAS 1427439-04-0, 220 mg, 0.955 mmol), phenylboronic acid (151 mg, 1.24 mmol), triphenylphosphine (50 mg, 191 umol, 0.20 eq) and palladium(II) acetate (21 mg, 95 umol, 0.100 eq) in 1,2-dimethoxyethane (6.6 ml) and 2 M Na2CO3 solution (1.57 ml, 3.15 mmol) was heated in the microwave at 90 °C for 2 h. The reaction mixture was poured into water and extracted with ethyl acetate. The organic layers were washed with brine, dried over MgSO4, filtered through Celite and concentrated in vacuo. The residue was purified by flash column chromatography (silica gel, 0% to 35% ethyl acetate in heptane) to provide 6-chloro-7-phenyl-1H-indole (204 mg, 89% yield) as yellow oil, MS (ESI) m/z: 228.0 [M+H]⁺.

### Step 2: 6-chloro-7-phenyl-1H-indole-3-sulfonyl chloride

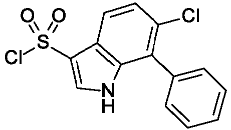

A stirred solution of 6-chloro-7-phenyl-1H-indole (198 mg, 826 umol) was cooled to 0°C, then chlorosulfonic acid (250 mg, 143 ul, 2.15 mmol) was added dropwise. The reaction mixture was stirred for 1 h at 0°C. The reaction mixture was warmed to room temperature and stirred for another 2 h. Phosphoryl chloride (507 mg, 308 ul, 3.3 mmol) was added in one portion. The reaction mixture was warmed to 60°C and stirred for 16 h. The reaction mixture was poured into ice / ethyl acetate and extracted twice with ethyl acetate. The combined organic layers were washed with brine, dried over Na2SO4, filtered and concentrated in vacuo to provide 6-chloro-7-phenyl-1H-indole-3-sulfonyl chloride (282 mg, 89% yield) as brown viscous oil, MS (ESI) m/z: 324.0 [M+H]⁺.

### Intermediate A24: 6-chloro-7-(3-methylpyrazin-2-yl)-1H-indole-3-sulfonyl chloride

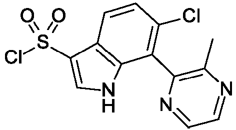

The title compound was prepared in analogy to Intermediate A1 from 2-bromo-3-methylpyrazine instead of 2-bromopyrimidine in Step 3) as off-white foam, MS (ESI) m/z: 342.0 [M+H]⁺.

### Intermediate A25: 6-chloro-7-oxazol-5-yl-1H-indole-3-sulfonyl chloride

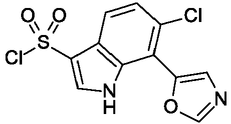

### Step 1: 5-(6-chloro-1H-indol-7-yl)oxazole

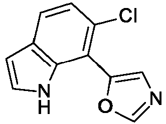

To a solution of 7-bromo-6-chloro-1H-indole (CAS 1427439-04-0, 1.18 g, 5.13 mmol) in tetrahydrofuran (20 ml) was added 5-(4,4,5,5-tetramethyl-1,3,2-dioxaborolan-2-yl)oxazole (CAS 942070-84-0, 1.0 g, 5.13 mmol), K3PO4 (3.26 g, 15.38 mmol) and cataCXium A Pd G3 (CAS 1651823-59-4, 373 mg, 0.51 mmol, 0.10 eq) at room temperature under nitrogen in a glove box, then the mixture was stirred at 60°C for 12 h under nitrogen. The reaction mixture was diluted with water (20 ml) and extracted with ethyl acetate (30 ml x 3). The combined organic layers were washed with brine (10 ml), dried over Na2SO4, filtered and concentrated under reduced pressure. The residue was purified by flash chromatography (silica gel, 0~17% ethyl acetate / petroleum ether) followed by preparative HPLC (column: Phenomenex luna C18, 150 * 40 mm * 15 um, water with formic acid / acetonitrile) to give 5-(6-chloro-1H-indol-7-yl)oxazole (806 mg, 68% yield) as a white solid, MS (ESI) m/z: 218.9 [M+H]⁺.

### Step 2: 6-chloro-7-oxazol-5-yl-1H-indole-3-sulfonyl chloride

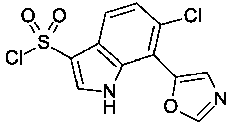

A stirring solution of 5-(6-chloro-1H-indol-7-yl)oxazole (250 mg, 1.07 mmol) was cooled to 0 °C and chlorosulfonic acid (325 mg, 186 ul, 2.79 mmol) was added dropwise. A white precipitate formed. The reaction was stirred for 30 min at 0°C. Phosphoryl chloride (824 mg, 500 ul, 5.37 mmol) was added in one portion and the reaction was heated to 70°C for 18 h. The reaction mixture was poured into ice / ethyl acetate and extracted three times with ethyl acetate. The combined organic layers were washed with brine, dried with Na2SO4, filtered and concentrated in vacuo to give 6-chloro-7-oxazol-5-yl-1H-indole-3-sulfonyl chloride (301 mg, 88% yield) as light yellow solid, MS (ESI) m/z: 316.9 [M+H]+.

### Intermediate A26: 6-chloro-7-pyrimidin-4-yl-1H-indole-3-sulfonyl chloride

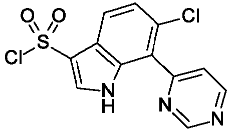

The title compound was prepared in analogy to Intermediate A2 from 4-chloropyrimidine hydrochloride instead of 2-bromooxazole in Step 1) as light yellow solid, MS (ESI) m/z: 328.0 [M+H]⁺.

### Intermediate A27: 7-(3-methylpyrazin-2-yl)-1H-indole-3-sulfonyl chloride

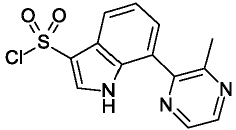

The title compound was prepared in analogy to Intermediate A1 from 7-(4,4,5,5-tetramethyl-1,3,2-dioxaborolan-2-yl)-1H-indole (CAS 642494-37-9) instead of 6-chloro-7-(4,4,5,5-tetramethyl-1,3,2-dioxaborolan-2-yl)-1H-indole and 2-bromo-3-methyl-pyrazine instead of 2-bromopyrimidine in Step 3) as yellow foam. MS (ESI) m/z: 306.1 [M-H]⁻.

### Intermediate A28: 6-chloro-7-isoxazol-5-yl-1H-indole-3-sulfonyl chloride

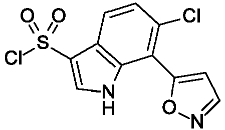

The title compound was prepared in analogy to Intermediate A25 from 5-(4,4,5,5-tetramethyl-1,3,2-dioxaborolan-2-yl)isoxazole (CAS 928664-98-6) instead of 5-(4,4,5,5-tetramethyl-1,3,2-dioxaborolan-2-yl)oxazole in Step 1) as brown solid, MS (ESI) m/z: 314.9 [M-H]⁻.

### Intermediate A29: 6-methyl-7-pyridazin-3-yl-1H-indole-3-sulfonyl chloride

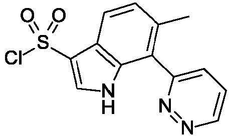

The title compound was prepared in analogy to Intermediate A3 from 2,2-dimethyl-1-[6-methyl-7-(4,4,5,5-tetramethyl-1,3,2-dioxaborolan-2-yl)indol-1-yl]propan-1-one (CAS 2376879-97-7) instead of 6-chloro-7-(4,4,5,5-tetramethyl-1,3,2-dioxaborolan-2-yl)-1H-indole as a light brown solid. MS (ESI) m/z: 308.1 [M+H]⁺.

### Intermediate A30: 6-chloro-7-(1,5-dimethylimidazol-4-yl)-1H-indole-3-sulfonyl chloride

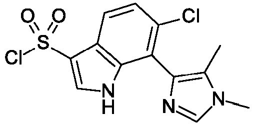

The title compound was prepared in analogy to Intermediate A2 from 4-bromo-1,5-dimethylimidazole (CAS 158585-81-0) instead of 2-bromooxazole in Step 1) as off-white foam, MS (ESI) m/z: 344.1 [M+H]⁺.

### Intermediate A31: 6-methyl-7-pyrazin-2-yl-1H-indole-3-sulfonyl chloride

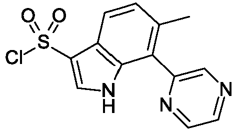

The title compound was prepared in analogy to Intermediate A3 from 2,2-dimethyl-1-[6-methyl-7-(4,4,5,5-tetramethyl-1,3,2-dioxaborolan-2-yl)indol-1-yl]propan-1-one (CAS 2376879-97-7) instead of 6-chloro-7-(4,4,5,5-tetramethyl-1,3,2-dioxaborolan-2-yl)-1H-indole and 2-bromopyrazine instead of 3-chloropyridazine in step 1) as a light brown solid.

### Intermediate A32: 6-chloro-7-(5-methylpyrazin-2-yl)-1H-indole-3-sulfonyl chloride

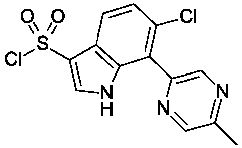

The title compound was prepared in analogy to Intermediate A1 from 2-bromo-5-methylpyrazine instead of 2-bromopyrimidine in Step 3) as yellow foam. MS (ESI) m/z: 342.0 [M+H]⁺.

### Intermediate A33: 6-chloro-7-(6-methylpyrazin-2-yl)-1H-indole-3-sulfonyl chloride

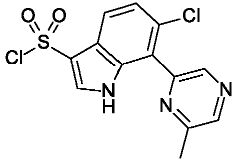

The title compound was prepared in analogy to Intermediate A1 from 2-bromo-6-methylpyrazine instead of 2-bromopyrimidine in Step 3) as off-white solid. MS (ESI) m/z: 342.0 [M+H]⁺.

### Intermediate A34: 6-chloro-5-fluoro-7-pyrimidin-2-yl-1H-indole-3-sulfonyl chloride

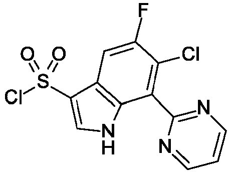

The title compound was prepared in analogy to Intermediate A1 from 6-chloro-5-fluoro-1H-indole instead of 6-chloro-1H-indole in Step 1) as light yellow solid. MS (ESI) m/z: 346.0 [M+H]⁺.

### Intermediate A35: 6-chloro-7-pyrimidin-5-yl-1H-indole-3-sulfonyl chloride

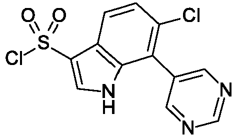

The title compound was prepared in analogy to Intermediate A1 from 5-bromopyrimidine instead of 2-bromopyrimidine in Step 3) as white solid. MS (ESI) m/z: 328.0 [M+H]⁺.

### Intermediate A36: 6-chloro-7-(1-methylpyrazol-3-yl)-1H-indole-3-sulfonyl chloride

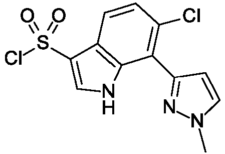

The title compound was prepared in analogy to Intermediate A1 from 3-iodo-1-methyl-pyrazole (CAS 92525-10-5) instead of 2-bromopyrimidine in Step 3) as off-white solid. MS (ESI) m/z: 330.1 [M+H]⁺.

### Intermediate A37: 6-chloro-7-(1-methylimidazol-4-yl)-1H-indole-3-sulfonyl chloride

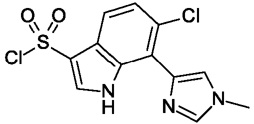

The title compound was prepared in analogy to Intermediate A1 from 4-iodo-1-methyl-imidazole CAS (71759-87-0) instead of 2-bromopyrimidine in Step 3) as light brown foam. MS (ESI) m/z: 330.1 [M+H]⁺.

### Intermediate A38: 6-chloro-7-(5-chloropyrazin-2-yl)-1H-indole-3-sulfonyl chloride

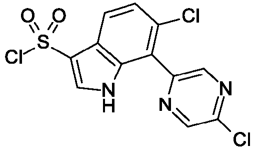

The title compound was prepared in analogy to Intermediate A2 from 2-bromo-5-chloropyrazine instead of 2-bromooxazole in Step 1) as off-white solid, MS (ESI) m/z: 362.0 [M-H]⁻

### Intermediate A39: 6-chloro-7-(6-methyl-2-pyridyl)-1H-indole-3-sulfonyl chloride

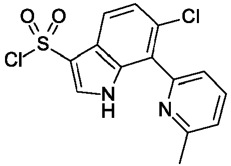

The title compound was prepared in analogy to Intermediate A1 from 2-bromo-6-methylpyridine instead of 2-bromopyrimidine in Step 3) as off-white solid. MS (ESI) m/z: 341.1 [M+H]⁺.

### Intermediate A40: 6-chloro-7-pyridazin-4-yl-1H-indole-3-sulfonyl chloride

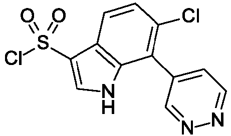

The title compound was prepared in analogy to Intermediate A1 from 4-bromopyridazine hydrobromide instead of 2-bromopyrimidine in Step 3) as light yellow solid. MS (ESI) m/z: 328.0 [M+H]⁺.

### Intermediate A41: 6-chloro-7-(2-methylpyrimidin-4-yl)-1H-indole-3-sulfonyl chloride

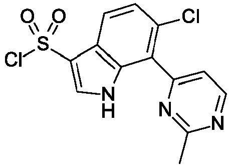

The title compound was prepared in analogy to Intermediate A1 from 4-chloro-2-methylpyrimidine instead of 2-bromopyrimidine in Step 3) as light yellow solid. MS (ESI) m/z: 342.0 [M+H]⁺.

### Intermediate A42: 6-chloro-7-(6-methylpyrimidin-4-yl)-1H-indole-3-sulfonyl chloride

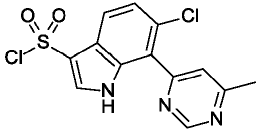

The title compound was prepared in analogy to Intermediate A1 from 4-bromo-6-methylpyrimidine instead of 2-bromopyrimidine in Step 3) as light yellow solid. MS (ESI) m/z: 342.0 [M+H]⁺.

### Intermediate A43: 6-chloro-7-(3-fluoropyrazin-2-yl)-1H-indole-3-sulfonyl chloride

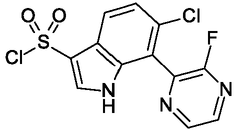

The title compound was prepared in analogy to Intermediate A1 from 2-bromo-3-fluoro-pyrazine instead of 2-bromopyrimidine in Step 3) as light brown solid. MS (ESI) m/z: 346.0 [M+H]⁺.

### Intermediate A44: 6-chloro-7-(6-methylpyridazin-3-yl)-1H-indole-3-sulfonyl chloride

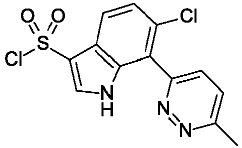

The title compound was prepared in analogy to Intermediate A2 from 3-chloro-6-methylpyridazine instead of 2-bromooxazole in Step 1) as off-white solid, MS (ESI) m/z: 342.0 [M+H]⁺.

### Intermediate A45: 6-chloro-7-(3-chloropyrazin-2-yl)-1H-indole-3-sulfonyl chloride

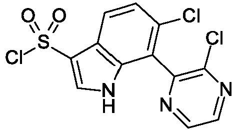

The title compound was prepared in analogy to Intermediate A2 from 2,3-dichloropyrazine instead of 2-bromooxazole in Step 1) as light brown solid, MS (ESI) m/z: 362.0 [M+H]⁺.

### Intermediate A46: 6-chloro-7-(3-methyl-2-pyridyl)-1H-indole-3-sulfonyl chloride

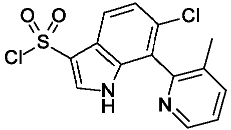

The title compound was prepared in analogy to Intermediate A1 from 2-bromo-3-methylpyridine instead of 2-bromopyrimidine in Step 3) as off-white solid. MS (ESI) m/z: 341.1 [M+H]⁺.

### Intermediate A47: 6-chloro-7-(1-methyltriazol-4-yl)-1H-indole-3-sulfonyl chloride

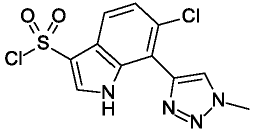

The title compound was prepared in analogy to Intermediate A2 from 4-bromo-1-methyl-triazole instead of 2-bromooxazole in Step 1) as off-white solid, MS (ESI) m/z: 331.0 [M+H]⁺.

### Intermediate A48: 6-bromo-7-(1-methylpyrazol-4-yl)-1H-indole-3-sulfonyl chloride

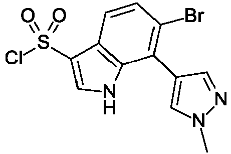

The title compound was prepared in analogy to Intermediate A1 from 6-bromo-1H-indole instead of 6-chloro-1H-indole in Step 1) and from 4-iodo-1-methyl-pyrazole instead of 2-bromopyrimidine in Step 3) as off-white solid, MS (ESI) m/z: 375.9 [M+H]⁺

### Intermediate A49: 6-chloro-7-(1-methyl-1,2,4-triazol-3-yl)-1H-indole-3-sulfonyl chloride

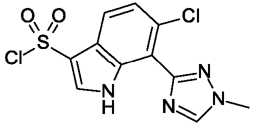

The title compound was prepared in analogy to Intermediate A1 from 3-bromo-1-methyl-1,2,4-triazole instead of 2-bromopyrimidine in Step 3) as white solid. MS (ESI) m/z: 233.1 [M+H]⁺.

### Intermediate A50: 6-chloro-7-(5-fluoropyrimidin-2-yl)-1H-indole-3-sulfonyl chloride

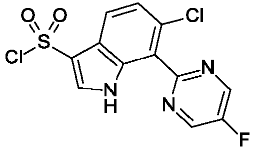

The title compound was prepared in analogy to Intermediate A1 from 2-bromo-5-fluoro-pyrimidine instead of 2-bromopyrimidine in Step 3) as light green solid. MS (ESI) m/z: 346.0 [M+H]⁺.

### Intermediate A51: 6-chloro-7-(2-methyltriazol-4-yl)-1H-indole-3-sulfonyl chloride

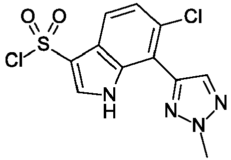

The title compound was prepared in analogy to Intermediate A2 from 4-bromo-2-methyl-triazole instead of 2-bromooxazole in Step 1) as pink solid, MS (ESI) m/z: 331.0 [M+H]⁺.

### Intermediate A52: 6-chloro-7-(1,3,4-oxadiazol-2-yl)-1H-indole-3-sulfonyl chloride

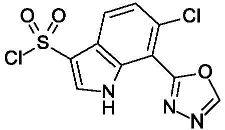

### Step 1: 6-chloro-1H-indole-7-carboxylic acid

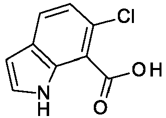

To a mixture of 6-chloro-1H-indole-7-carbonitrile (CAS 1427391-07-8, 1.5 g, 8.49 mmol) in ethanol (15 ml) was added a solution of potassium hydroxide (2.38 g, 42.5 mmol) in water (2.5 ml). The mixture was stirred at 80 °C for 48 h. The mixture was adjusted to pH 4 with 1N aqueous hydrochloric acid, then extracted with ethyl acetate (20 ml × 2). The combined organic layers were washed with brine, dried over Na2SO4 and concentrated under reduced pressure. The residue was purified by column chromatography (silica gel, petroleum ether / ethyl acetate = 1:0 to 1:1) to give 6-chloro-1H-indole-7-carboxylic acid (1.3 g, 78% yield) as a brown solid, MS (ESI) m/z: 194.1 [M-H]⁻.

### Step 2: tert-butyl N-[(6-chloro-1H-indole-7-carbonyl)amino]carbamate

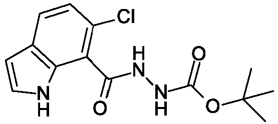

To a solution of 6-chloro-1H-indole-7-carboxylic acid (1.30 g, 6.65 mmol) and tert-butyl carbazate (1.32 g, 9.97 mmol) in dimethylformamide (20 ml) were added N-ethyl diisopropylamine (2.31 ml, 13.3 mmol) and HATU (1.88 g, 7.98 mmol) and the mixture was stirred at 20 °C for 16 h. The reaction mixture was diluted with water (30 mL) and extracted with ethyl acetate (20 ml × 2). The combined organic layers were washed with brine, dried over Na2SO4, filtered and concentrated under reduced pressure. The residue was purified by column chromatography (silica gel, petroleum ether / ethyl acetate = 1:0 to 5:1) to give tert-butyl N-[(6-chloro-1H-indole-7-carbonyl)amino]carbamate (900 mg, 44% yield) as a white solid, MS (ESI) m/z: 308.2 [M-H]⁻.

### Step 3: 6-chloro-1H-indole-7-carbohydrazide

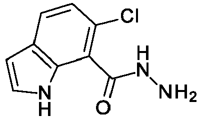

A mixture of tert-butyl N-[(6-chloro-1H-indole-7-carbonyl)amino]carbamate (900 mg, 2.91 mmol) and HCl in dioxane (4M, 18.0 ml, 72.0 mmol) was stirred at 20 °C for 1 h. Then the pH of the mixture was adjusted to 7 with saturated NaHCO3 solution and the mixture was extracted with ethyl acetate (20 ml × 2). The combined organic layers were washed with brine, dried over Na2SO4 and concentrated under reduced pressure to give 6-chloro-1H-indole-7-carbohydrazide (600 mg, 98% yield) as a yellow solid, MS (ESI) m/z: 208.1 [M-H]⁻.

### Step 4: 2-(6-chloro-1H-indol-7-yl)-1,3,4-oxadiazole

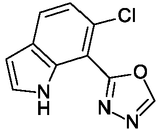

A solution of 6-chloro-1H-indole-7-carbohydrazide (600 mg, 2.86 mmol) and ammonium chloride (152 mg, 2.86 mmol) in triethyl orthoformate (15 ml, 98.28 mmol) was stirred at 145 °C for 1 h. The mixture was concentrated under reduced pressure and the residue was purified by column chromatography (silica gel, petroleum ether / ethyl acetate = 1:0 to 5:1) to give 2-(6-chloro-1H-indol-7-yl)-1,3,4-oxadiazole (425 mg, 62% yield) as a white solid. MS (ESI) m/z: 220.2 [M+H]⁺.

### Step 5: 6-chloro-7-(1,3,4-oxadiazol-2-yl)-1H-indole-3-sulfonyl chloride

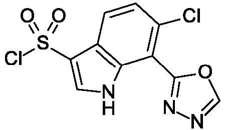

A stirred suspension of 2-(6-chloro-1H-indol-7-yl)-1,3,4-oxadiazole (100 mg, 0.414 mmol) in acetonitrile (5 ml) was cooled to 0 °C and chlorosulfonic acid (125 mg, 72 ul, 1.08 mmol) was added dropwise. The reaction mixture was stirred at 0°C for 30 min. Phosphoryl chloride (318 mg, 193 ul, 2.07 mmol) was added in one portion. The reaction mixture was heated to 60 °C for 22 h. The reaction mixture was poured into ice / ethyl acetate and extracted with ethyl acetate. The combined organic layers were washed with brine, dried over Na2SO4, filtered and concentrated to give the title compound (81 mg, 54% yield) as pink solid. MS (ESI) m/z: 318.0 [M+H]⁺.

### Intermediate A53: 6-chloro-7-(1-methylpyrazol-4-yl)-1H-indole-3-sulfonyl chloride

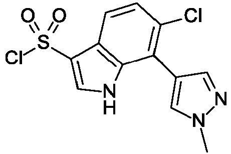

The title compound was prepared in analogy to Intermediate A1 from 4-iodo-1-methyl-pyrazole instead of 2-bromopyrimidine in Step 3) as light yellow solid, MS (ESI) m/z: 330.1 [M+H]⁺.

### Intermediate A54: 6-chloro-7-thiazol-4-yl-1H-indole-3-sulfonyl chloride

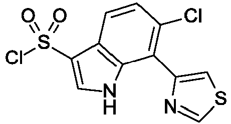

The title compound was prepared in analogy to Intermediate A2 from 4-bromothiazole instead of 2-bromooxazole in Step 1) as white solid, MS (ESI) m/z: 332.8 [M+H]⁺.

### Intermediates B

### Intermediate B1: 5-(2,2-difluoroethoxy)-4,6-dimethoxy-pyrimidin-2-amine

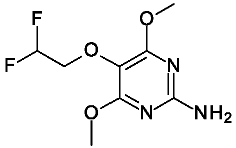

### Step 1: 5-bromo-4,6-dimethoxy-pyrimidin-2-amine

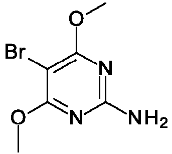

To a stirred solution of (4,6-dimethoxypyrimidin-2-yl)amine (7 g, 44.22 mmol, CAS: 36315-01-2) in acetonitrile (100 mL) was added a solution of N-bromosuccinimide (10.33 g, 57.48 mmol) in acetonitrile (100 mL) dropwise at room temperature. The reaction mixture was stirred at room temperature for 30 min. The resulting white suspension was diluted with ethyl acetate and washed with water. The organic layer was dried over sodium sulfate, filtered, diluted with heptane and concentrated in vacuo. The precipitate was filtered off and washed with heptane to provide the title compound as a white solid (9.26 g, 87 % yield). MS (ESI) m/z= 234.1 [M+H]⁺

### Step 2: 5-bromo-4,6-dimethoxy-N,N-bis[(4-methoxyphenyl)methyl]pyrimidin-2-amine

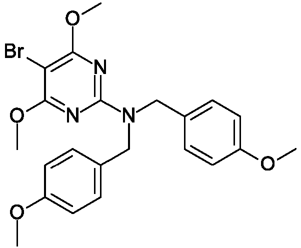

A solution of 5-bromo-4,6-dimethoxy-pyrimidin-2-amine (517 mg, 2.21 mmol) in N,N-dimethylacetamide (9 mL) was cooled to 0 °C. Sodium hydride (265.05 mg, 6.63 mmol) was added portionwise (3 x 88 mg). The stirring was continued at 0 °C for 30 min. 4-methoxybenzyl chloride (706. mg, 608.62 uL, 4.42 mmol) was added dropwise. The reaction mixture was allowed to warm to room temperature, stirred for 1 hour, carefully quenched with a saturated ammonium chloride solution, poured into water and extracted twice with ethyl acetate. The organic layers were dried over sodium sulfate, filtered and concentrated in vacuo. The residue was purified by flash chromatography over silica gel using a gradient ethylacetate/heptane 0-20% to provide the title compound as a white solid (1.11 g, 100 % yield). MS (ESI) m/z= 476.2 [M+H]⁺

### Step 3: 2-[bis[(4-methoxyphenyl)methyl]amino]-4,6-dimethoxy-pyrimidin-5-ol

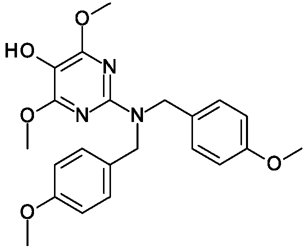

To a colorless solution of 5-bromo-4,6-dimethoxy-N,N-bis[(4-methoxyphenyl)methyl]-pyrimidin-2-amine (500 mg, 1 mmol) in tetrahydrofuran (3.5 mL) was added a 1.6 M n-butyllithium solution in hexanes (699.71 mg, 813.61 uL, 1.3 mmol) dropwise at -78 °C. The resulting yellow solution was stirred at -78 °C for 30 min. Trimethyl borate (156.08 mg, 167.47 uL, 1.5 mmol) was added dropwise and the stirring was continued at -78 °C for 1.5 hours. The reaction mixture was allowed to warm to 0 °C and acetic acid (120.27 mg, 114.62 uL, 2 mmol) was added dropwise, followed by hydrogen peroxide 35% (145.98 mg, 131.51 uL, 1.5 mmol). The stirring was continued at 0 °C for 1.5 hours. The resulting pink suspension was poured into a 0.1 N sodium thiosulfate solution and extracted twice with ethyl acetate. The organic layers were washed with brine, dried over sodium sulfate, filtered and concentrated in vacuo. The residue was purified by flash chromatography over silica gel using a gradient ethylacetate/heptane 0-30% to provide the title compound as a light yellow viscous oil (194 mg, 47 % yield). MS (ESI) m/z= 412.3 [M+H]⁺

### Step 4: 5-(2,2-difluoroethoxy)-4,6-dimethoxy-N,N-bis[(4-methoxyphenyl)methyl]pyrimidin-2-amine

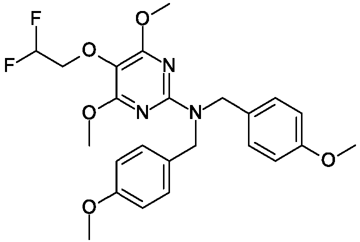

To a solution of 2-[bis[(4-methoxyphenyl)methyl]amino]-4,6-dimethoxy-pyrimidin-5-ol (100 mg, 0.214 mmol) in N,N-dimethylformamide (1.75 mL) was added potassium carbonate (88.68 g, 0.642 mmol) and 1,1-difluoro-2-iodoethane (123.16 mg, 56.5 uL, 0.642 mmol). The reaction mixture was stirred at 80 °C for 1.5 hours, cooled to room temperature, poured into water and extracted twice with ethyl acetate. The organic layers were dried over sodium sulfate, filtered and concentrated in vacuo. The residue was purified by flash chromatography over silica gel using a gradient ethylacetate/heptane 0-20% to provide the title compound as an off-white solid (92 mg, 85 % yield). MS (ESI) m/z= 476.2 [M+H]⁺

### Step 5: 5-(2,2-difluoroethoxy)-4,6-dimethoxy-pyrimidin-2-amine

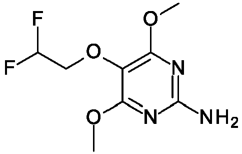

To a stirred solution of 5-(2,2-difluoroethoxy)-4,6-dimethoxy-N,N-bis[(4-methoxyphenyl)methyl]pyrimidin-2-amine (92 mg, 0.193 mmol) in dichloromethane (0.340 mL) was added trifluoroacetic acid (1.34 g, 897.97 uL, 11.61 mmol) at 0 °C. The reaction mixture was stirred at room temperature for 18 hours and at 50 °C for 4 hours. The resulting red solution was concentrated in vacuo, poured into sat. NaHCO3 and extracted twice with ethyl acetate. The organic layers were dried over sodium sulfate, filtered and concentrated in vacuo. The residue was purified by flash chromatography over silica gel using a gradient ethylacetate/heptane 0-30% to provide the title compound as a light yellow solid (41 mg, 87 % yield). MS (ESI) m/z= 236.2 [M+H]⁺

### Intermediate B2: 5-(2,2-difluoroethyl)-4,6-dimethoxy-pyrimidin-2-amine

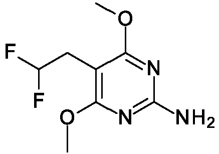

### Step 1: diethyl 2-(2,2-difluoroethyl)propanedioate

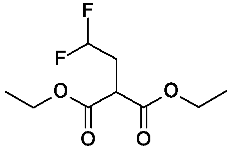

Diethyl malonate (75.8 ml, 500 mmol) was combined with tetrahydrofuran (450 ml). Sodium ethoxide (prepared from ethanol (150 mL) and sodium (11.48 g, 500 mmol)) was added at room temperature and the reaction mixture was stirred 15 min at room temperature. A solution of 2,2-difluoroethyl trifluoromethanesulfonate (76 ml, 500 mmol) in tetrahydrofuran (10 ml) was added slowly. The reaction mixture was stirred for 18 hours at 20 °C, then cooled to 0 °C, quenched with a saturated ammonium chloride solution and extracted twice with ethyl acetate. The organic layers were washed with brine, dried over sodium sulfate, filtered and concentrated in vacuo to provide the title compound (100.5 g, 90 % yield). MS (ESI) m/z= 225.0 [M+H]⁺

### Step 2: 2-amino-5-(2,2-difluoroethyl)pyrimidine-4,6-diol

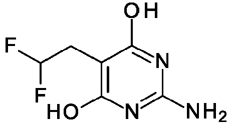

To a stirred solution of diethyl 2-(2,2-difluoroethyl)propanedioate (46.8 g, 209 mmol) in ethanol (5 mL) was added guanidine hydrochloride (19.9 g, 208 mmol), followed by sodium ethoxide (prepared from ethanol and sodium (14.38 g, 625 mmol)). The resulting orange suspension was heated to 80 °C and stirred for 4 hours. The reaction mixture was concentrated by half, 50 ml of water was added, followed by acetic acid (42.57 g, 709 mmol). The mixture was heated to 80 °C and stirred for 10 min, then cooled to room temperature. The solid product was filtered off, washed successively with water, ethanol and methyl tert-butyl ether to provide the title compound (22.3 g, 50 % yield). MS (ESI) m/z= 192.0 [M+H]+

### Step 3: 4,6-dichloro-5-(2,2-difluoroethyl)pyrimidin-2-amine

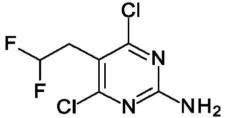

2-amino-5-(2,2-difluoroethyl)pyrimidine-4,6-diol (13.2 g, 69.1 mmol) was suspended in phosphorus oxychloride (80.5 ml, 863 mmol). The reaction mixture was heated to 100 °C and stirred for 18 hours and concentrated in vacuo. The residue was diluted with ethyl acetate and carefully poured into ice / saturated sodium bicarbonate solution. The resulting biphasic mixture was stirred at room temperature for 5 min and extracted twice with ethyl acetate. The organic layers were dried over sodium sulfate, filtered and concentrated in vacuo. The residue was purified by flash chromatography over silica gel to provide the title compound (7.35 g, 47 % yield). MS (ESI) m/z= 227.8 [M+H]⁺

### Step 4: 5-(2,2-difluoroethyl)-4,6-dimethoxy-pyrimidin-2-amine

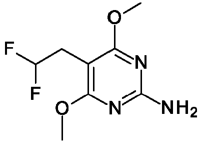

In a sealed tube, a mixture of 4,6-dichloro-5-(2,2-difluoroethyl)pyrimidin-2-amine (7.6 g, 33.33 mmol) and sodium methylate (prepared from sodium (7.66 g, 333.29 mmol) in methanol (50 ml)) was heated to 75 °C and stirred for 18 hours. The reaction mixture was quenched with water and extracted twice with ethyl acetate. The organic layers were dried over sodium sulfate, filtered and concentrated in vacuo to provide the title compound as a light yellow solid (6.6 g, 86 % yield). MS (ESI) m/z= 220.0 [M+H]⁺

### Intermediate B3: 5-(difluoromethoxy)-4,6-dimethoxy-pyrimidin-2-amine

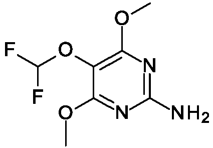

### Step 1: 5-(difluoromethoxy)-4,6-dimethoxy-N,N-bis[(4-methoxyphenyl)methyl]pyrimidin-2-amine

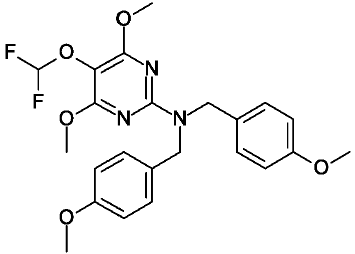

To a solution of 2-[bis[(4-methoxyphenyl)methyl]amino]-4,6-dimethoxy-pyrimidin-5-ol (130 mg, 0.316 mmol, intermediate B1, step 3) in acetonitrile (5 mL) was added a 5 M potassium hydroxide solution (1.26 mL, 6.32 mmol) dropwise at 0 °C, followed by addition of bromodifluoromethyl diethylphosphonate (168.72 mg, 112.26 uL, 0.632 mmol) in acetonitrile (1 mL) dropwise at 0 °C. The reaction mixture was stirred at 0 °C for 10 min. The resulting light yellow biphasic mixture was poured into water and extracted twice with ethyl acetate. The organic layers were dried over sodium sulfate, filtered and concentrated in vacuo. The residue was purified by flash chromatography over silica gel using a gradient ethylacetate/heptane 0-30% to provide the title compound as a white solid (57 mg, 39 % yield). MS (ESI) m/z= 462.3 [M+H]+

### Step 2: 5-(difluoromethoxy)-4,6-dimethoxy-pyrimidin-2-amine

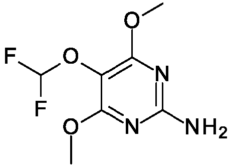

To a stirred solution of 5-(difluoromethoxy)-4,6-dimethoxy-N,N-bis[(4-methoxyphenyl)methyl]pyrimidin-2-amine (56 mg, 0.121 mmol) in dichloromethane (100 uL) was added trifluoroacetic acid (838.62 mg, 563.21 uL, 7.28 mmol). The reaction mixture was stirred at room temperature for 40 hours, at 50 °C for 6 hours and concentrated in vacuo. The residue was poured into sat. NaHCO3 and extracted twice with ethyl acetate. The organic layers were dried over sodium sulfate, filtered and concentrated in vacuo. The residue was purified by flash chromatography over silica gel using a gradient ethylacetate/heptane 0-100% to provide the title compound as a white solid (24 mg, 89 % yield). MS (ESI) m/z= 222.1 [M+H]⁺

### Intermediate B4: 5-(2-fluoroethoxy)-4,6-dimethoxy-pyrimidin-2-amine

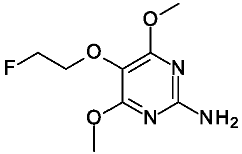

### Step 1: 5-(2-fluoroethoxy)-4,6-dimethoxy-N,N-bis[(4-methoxyphenyl)methyl]pyrimidin-2-amine

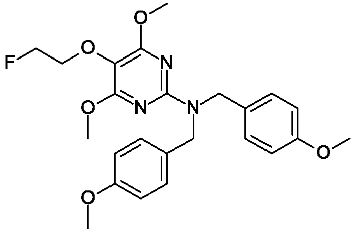

A suspension of 2-[bis[(4-methoxyphenyl)methyl]amino]-4,6-dimethoxy-pyrimidin-5-ol (2 g, 4.52 mmol, intermediate B1, step 3), potassium carbonate (1.89 g, 13.56 mmol) and 1-bromo-2-fluoroethane (1.76 g, 1.03 mL, 13.56 mmol) in N,N-dimethylformamide (45 mL) was heated to 80 °C and stirred for 2.5 hours. The reaction mixture was poured into brine and extracted twice with ethyl acetate. The organic layers were dried over sodium sulfate, filtered and concentrated in vacuo. The residue was purified by flash chromatography over silica gel using a gradient ethylacetate/heptane 0-30% to provide the title compound as a white solid (1.79 g, 85 % yield). MS (ESI) m/z= 458.3 [M+H]⁺

### Step 2: 5-(2-fluoroethoxy)-4,6-dimethoxy-pyrimidin-2-amine

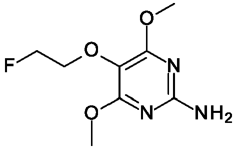

To a stirred solution of 5-(2-fluoroethoxy)-4,6-dimethoxy-N,N-bis[(4-methoxyphenyl)methyl]pyrimidin-2-amine (1.79 g, 3.83 mmol) in dichloromethane (4 mL) was added trifluoroacetic acid (26.23 g, 17.62 mL, 230.06 mmol). The reaction mixture was stirred at 50 °C for 3 hours, at room temperature for 15 hours and concentrated in vacuo. The residue was poured into sat. NaHCO3 and extracted twice with ethyl acetate. The organic layers were dried over sodium sulfate, filtered and concentrated in vacuo. The residue was purified by flash chromatography over silica gel using a gradient ethylacetate/heptane 0-60% to provide the title compound as an off-white solid (885 mg, 100 % yield). MS (ESI) m/z= 218.1 [M+H]⁺

### Intermediate B5: 2-(2-amino-4,6-dimethoxy-pyrimidin-5-yl)cyclopropanecarbonitrile

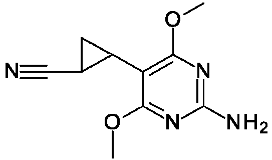

### Step 1: N,N-bis[(2,4-dimethoxyphenyl)methyl]-4,6-dimethoxy-pyrimidin-2-amine

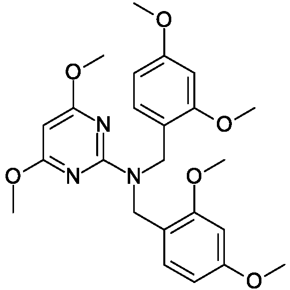

To a solution of 2-chloro-4,6-dimethoxypyrimidine (5.0 g, 28.6 mmol) and 1-(2,4-dimethoxyphenyl)-N-[(2,4-dimethoxyphenyl)methyl]methanamine (10.9 g, 34.37 mmol) in N-methylpyrrolidone (100 ml) was added cesium carbonate (18.7 g, 57.3 mmol) and the reaction mixture was stirred at 120 °C for 16 h. The mixture was diluted with water (500 ml) and extracted with ethyl acetate (100 ml × 2). The combined organic layers were washed with brine (100 ml × 2), dried over Na2SO4, filtered and concentrated under reduced pressure. The residue was purified by column chromatography (silica gel, petroleum ether/ethyl acetate = 1:0 to 5:1) to give N-bis[(2,4-dimethoxyphenyl)methyl]-4,6-dimethoxy-pyrimidin-2-amine (12.0 g, 89% yield) as a white solid. MS (ESI) m/z= 456.3 [M+H]⁺

### Step 2: N,N-bis[(2,4-dimethoxyphenyl)methyl]-5-iodo-4,6-dimethoxy-pyrimidin-2-amine

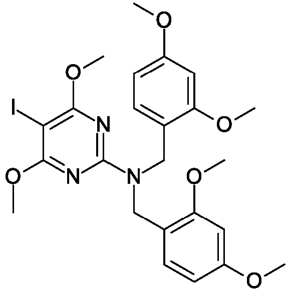

To a solution of N,N-bis[(2,4-dimethoxyphenyl)methyl]-4,6-dimethoxy-pyrimidin-2-amine (12.0 g, 26.3 mmol) in acetonitrile (150 ml) was added N-iodosuccinimide (7.11 g, 31.6 mmol) in portions at 20 °C and the mixture was stirred at 20 °C for 2 h. The reaction mixture was quenched by pouring into saturated sodium hydrogencarbonate solution (300 ml) and extracted with ethyl acetate (100 ml × 2). The combined organic layers were washed with brine (100 ml × 2), dried over sodium sulfate, filtered and concentrated under reduced pressure. The residue was purified by column chromatography (silica gel, petroleum ether / ethyl acetate = 1:0 to 3:1) to give N,N-bis[(2,4-dimethoxyphenyl)methyl]-5-iodo-4,6-dimethoxy-pyrimidin-2-amine (12.0 g, 78% yield) as a yellow solid. MS (ESI) m/z= 582.2 [M+H]⁺

### Step 3: (E)-3-[2-[bis[(2,4-dimethoxyphenyl)methyl]amino]-4,6-dimethoxy-pyrimidin-5-yl]prop-2-enenitrile

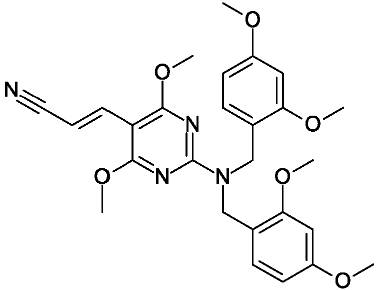

To a mixture of N,N-bis[(2,4-dimethoxyphenyl)methyl]-5-iodo-4,6-dimethoxy-pyrimidin-2-amine (9.0 g, 15.5 mmol) and acrylonitrile (6.0 ml, 90.5 mmol) in 1,4-dioxane (90 ml) was added cesium carbonate (10.8 g, 33.1 mmol) and bis(triphenylphosphine)palladium chloride (681 mg, 1.55 mmol) under nitrogen atmosphere and the reaction mixture was stirred in a sealed tube at 100 °C for 16 h. The mixture was concentrated under reduced pressure.

The residue was purified by column chromatography (silica gel, petroleum ether/ethyl acetate = 1:0 to 5:1) to give (E)-3-[2-[bis[(2,4-dimethoxyphenyl)methyl]amino]-4,6-dimethoxy-pyrimidin-5-yl]prop-2-enenitrile (7.5 g, 96% yield) as a brown solid. MS (ESI) m/z= 507.3 [M+H]⁺

### Step 4: 2-[2-[bis[(2,4-dimethoxyphenyl)methyl]amino]-4,6-dimethoxy-pyrimidin-5-yl]cyclopropanecarbonitrile

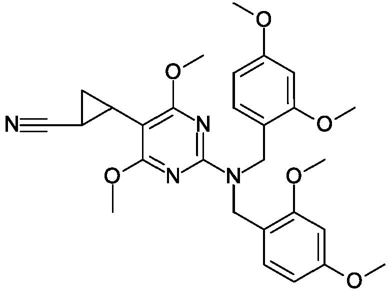

To a mixture of trimethylsulfoxonium iodide (6.52 g, 29.6 mmol) in dimethylsulfoxide (50 ml) was added sodium hydride (60% in mineral oil, 1.18 g, 29.6 mmol) in portions at 30 °C and the mixture was stirred at 30 °C for 0.5 h. The above mixture was added to a mixture of (E)-3-[2-[bis[(2,4-dimethoxyphenyl)methyl]amino]-4,6-dimethoxy-pyrimidin-5-yl]prop-2-enenitrile (5.0 g, 9.87 mmol) in dimethylsulfoxide (50 ml) at 30 °C and the mixture was stirred at 30 °C for 16 hr. The reaction mixture was diluted with water (300 ml) and extracted with ethyl acetate (100 ml × 2). The combined organic layers were washed with brine (100 ml × 2), dried over sodium sulfate, filtered and concentrated under reduced pressure. The residue was purified by column chromatography (silica gel, petroleum ether/ethyl acetate = 1:0 to 3:1) to give 2-[2-[bis[(2,4-dimethoxyphenyl)methyl]amino]-4,6-dimethoxy-pyrimidin-5-yl]cyclopropanecarbonitrile (3.0 g, 54% yield) as yellow oil. MS (ESI) m/z= 521.3 [M+H]⁺

### Step 5: 2-(2-amino-4,6-dimethoxy-pyrimidin-5-yl)cyclopropanecarbonitrile

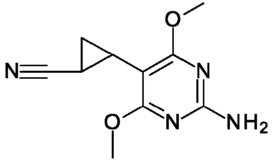

To a solution of 2-[2-[bis[(2,4-dimethoxyphenyl)methyl]amino]-4,6-dimethoxy-pyrimidin-5-yl]cyclopropanecarbonitrile (3.0 g, 5.76 mmol) in dichloromethane (10 ml) was added trifluoroacetic acid (15 ml, 197 mmol) dropwise at 10 °C and the mixture was stirred at 10 °C for 4 h. The reaction mixture was diluted with water (100 ml) and adjusted pH to 7 with saturated sodium bicarbonate solution at 0-10 °C. The mixture was extracted with ethyl acetate (50 ml × 2). The combined organic layers were washed with brine (50 ml × 2), dried over sodium sulfate, filtered and concentrated under reduced pressur. The residue was triturated with ethyl acetate (4 ml) and filtered. The filter cake was dried under reduced pressure to give the title compound (907 mg, 70 % yield) as a white solid. MS (ESI) m/z= 221.1 [M+H]⁺

### Intermediate B6: 5-bromo-4,6-dimethoxy-pyrimidin-2-amine

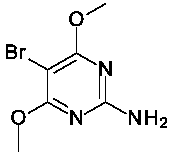

To a stirred solution of (4,6-dimethoxypyrimidin-2-yl)amine (7 g, 44.2 mmol, CAS: 36315-01-2) in acetonitrile (100 ml) was added a solution of N-bromosuccinimide (10.3 g, 57.5 mmol) in acetonitrile (100 ml) dropwise at room temperature. The reaction mixture was stirred at room temperature for 30 min. The resulting white suspension was diluted with ethyl acetate and washed with water. The organic layer was dried over sodium sulfate, filtered, diluted with heptane and concentrated in vacuo. The precipitate was filtered off and washed with heptane to provide the title compound as a white solid (9.26 g, 87% yield). MS (ESI) m/z= 234.1 [M+H]⁺.

### Intermediate B7: 2-(2-amino-4,6-dimethoxy-pyrimidin-5-yl)oxyacetonitrile

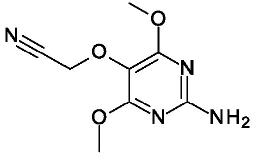

### Step 1: 2-[2-[bis[(4-methoxyphenyl)methyl]amino]-4,6-dimethoxy-pyrimidin-5-yl]oxyacetonitrile

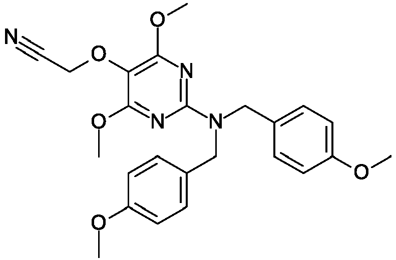

A mixture of 2-[bis[(4-methoxyphenyl)methyl]amino]-4,6-dimethoxy-pyrimidin-5-ol (1 g, 2.43 mmol, see Intermediate B1, step 3), iodoacetonitrile (1.22 g, 529 ul, 7.29 mmol) and potassium carbonate (1.01 g, 7.29 mmol) in N,N-dimethylformamide (16 ml) was stirred at 80°C overnight.

The reaction mixture was poured into water and extracted twice with ethyl acetate. The organic layers were washed with brine, dried over sodium sulfate, filtered and concentrated in vacuo. The residue was purified by flash chromatography (silica gel, ethyl acetate / heptane 0-20%) to provide the title compound as a white solid (855 mg, 77 % yield). MS (ESI) m/z= 451.3 [M+H]⁺.

### Step 2: 2-(2-amino-4,6-dimethoxy-pyrimidin-5-yl)oxyacetonitrile

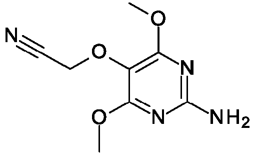

A mixture of 2-[2-[bis[(4-methoxyphenyl)methyl]amino]-4,6-dimethoxy-pyrimidin-5-yl]oxyacetonitrile (855 mg, 1.9 mmol) and trifluoroacetic acid (6.49 g, 4.39 ml, 56.94 mmol) in dichloromethane (6 ml) was stirred at 50 °C overnight. The reaction mixture was concentrated in vacuo, poured into saturated NaHCO3 solution and extracted twice with ethyl acetate. The combined organic layers were washed with brine, dried over sodium sulfate, filtered and concentrated in vacuo. The residue was purified by flash chromatography (silica gel, ethyl acetate / heptane 0-39%) to provide the title compound as an off-white solid (406 mg, 92 % yield). MS (ESI) m/z= 211.1 [M+H]⁺.

### Intermediate B8: 3-(2-amino-4,6-dimethoxy-pyrimidin-5-yl)propanenitrile

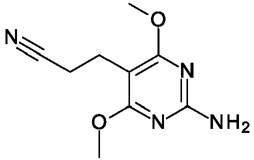

### Step 1: 3-[2-[bis[(4-methoxyphenyl)methyl]amino]-4,6-dimethoxy-pyrimidin-5-yl]propanenitrile

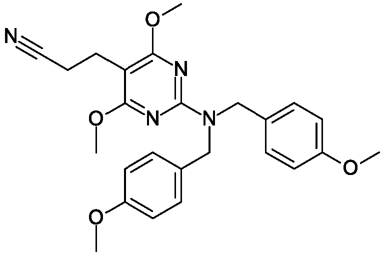

To a solution of 5-bromo-4,6-dimethoxy-N,N-bis[(4-methoxyphenyl)methyl]pyrimidin-2-amine (5.0 g, 10.54 mmol, intermediate B1, step 2) in methoxycyclopentane (75.0 ml) was added 3-(4,4,5,5-tetramethyl-1,3,2-dioxaborolan-2-yl)propanenitrile (2.29 g, 12.6 mmol), cesium carbonate (10.3 g, 31.62 mmol) and cataCXium^{®} A Pd G3 (768 mg, 1.05 mmol, CAS: 1651823-59-4) under nitrogen. The mixture was purged three times with nitrogen, stirred at 90 °C for 12 h under nitrogen, poured into NaHCO3 solution and extracted three times with ethyl acetate. The combined organic layers were washed with dried over sodium sulfate, filtered and concentrated in vacuo. The residue was purified by flash chromatography (silica gel, ethyl acetate / petroleum ether 0-30%) to provide the title compound as a yellow solid (2.9 g, 61 % yield). MS (ESI) m/z= 449.0 [M+H]⁺.

### Step 2: 3-(2-amino-4,6-dimethoxy-pyrimidin-5-yl)propanenitrile

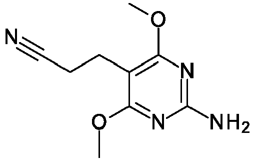

A mixture of 3-[2-[bis[(4-methoxyphenyl)methyl]amino]-4,6-dimethoxy-pyrimidin-5-yl]propanenitrile (2300 mg, 5.13 mmol) in trifluoroacetic acid (23 ml) was stirred at 25 °C for 48 h, quenched with ice/saturated NaHCO3 solution and extracted three times with ethyl acetate. The combined organic layers were dried over sodium sulfate, filtered and concentrated in vacuo. The residue was purified by flash chromatography (silica gel, ethyl acetate / petroleum ether 0-100%) to provide the title compound as a yellow solid (1000 mg, 89 % yield). MS (ESI) m/z= 209.0 [M+H]⁺.

### Intermediate B9: 5-(2-fluoroethoxy)-4-methoxy-pyrimidin-2-amine

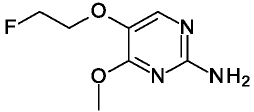

### Step 1: [4-methoxy-5-(4,4,5,5-tetramethyl-1,3,2-dioxaborolan-2-yl)pyrimidin-2-yl]-bis(p-anisyl)amine

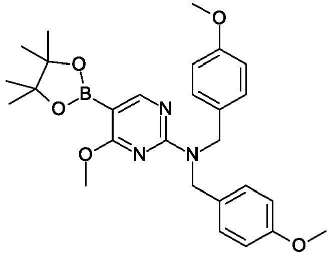

A suspension of (5-bromo-4-methoxy-pyrimidin-2-yl)-bis(p-anisyl)amine (500 mg, 1.13 mmol, see Intermediate B3 step 1), bis(pinacolato)diboron (354 mg, 1.35 mmol) and potassium acetate (335 mg, 3.38 mmol) in 1,4-dioxane (10 ml) was purged with argon for 5 min. dichloro[1,1'-bis(diphenylphosphino)ferrocene]palladium(II) dichloromethane adduct (92 mg, 0.113 mmol) was added. The reaction mixture was heated to 90°C and stirred for 16 h. The resulting dark suspension was poured into ethyl acetate and washed once with brine. The organic layer was dried over sodium sulfate, filtered and concentrated in vacuo. The residue was purified by column chromatography on silica gel using a gradient ethyl acetate / heptane 0-30% to provide the title compound as a colorless viscous oil (157 mg, 29% yield). MS (ESI): m/z= 492.4 [M+H]⁺

### Step 2: 2-[bis(p-anisyl)amino]-4-methoxy-pyrimidin-5-ol

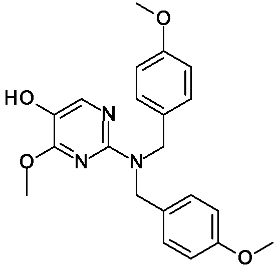

A solution of [4-methoxy-5-(4,4,5,5-tetramethyl-1,3,2-dioxaborolan-2-yl)pyrimidin-2-yl]-bis(p-anisyl)amine (130 mg, 0.265 mmol) in tetrahydrofuran (2.5 ml) was cooled to 0°C. Hydrogen peroxide 35% (500 ul, 5.71 mmol) was added. The reaction mixture was stirred at 0°C for 15 min, allowed to warm to room temperature and stirred for 3 h. The reaction mixture was poured into cold 0.1 N sodium sulfite solution and extracted twice with ethyl acetate. The organic layers were washed twice with brine, dried over sodium sulfate, filtered and concentrated in vacuo to provide the title compound as a light yellow viscous oil (103 mg, 100 % yield). MS (ESI): m/z= 382.3 [M+H]⁺

### Step 3: [5-(2-fluoroethoxy)-4-methoxy-pyrimidin-2-yl]-bis(p-anisyl)amine

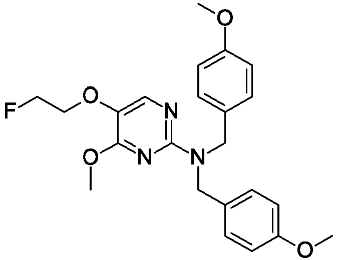

A suspension of 2-[bis(p-anisyl)amino]-4-methoxy-pyrimidin-5-ol (100 mg, 0.236 mmol), potassium carbonate (98.8 mg, 0.708 mmol) and 1-bromo-2-fluoroethane (61 mg, 36 ul, 0.472 mmol) in acetonitrile (2.5 ml) was stirred at room temperature for 15 min and at 80°C for 6 h. The reaction mixture was poured into water and extracted twice with ethyl acetate. The organic layers were dried over sodium sulfate, filtered and concentrated in vacuo. The residue was purified by flash chromatography (silica gel, ethyl acetate / heptane 0-30%) to provide the title compound as a colorless viscous oil (22 mg, 22 % yield). MS (ESI): m/z= 428.3 [M+H]⁺

### Step 4: [5-(2-fluoroethoxy)-4-methoxy-pyrimidin-2-yl]amine

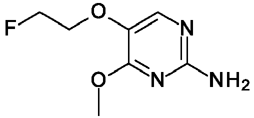

A solution of [5-(2-fluoroethoxy)-4-methoxy-pyrimidin-2-yl]-bis(p-anisyl)amine (87 mg, 0.204 mmol) in dichloromethane (500 ul) was cooled to 0 °C. Trifluoroacetic acid (1.41 g, 945 ul, 12.2 mmol) was added. The reaction mixture was allowed to warm to room temperature and stirred for 16 h and at 55°C for two additional hours. The resulting purple solution was poured into a saturated aqueous sodium bicarbonate solution and extracted twice with ethyl acetate. The organic layers were dried over sodium sulfate, filtered and concentrated in vacuo. The residue was purified by flash chromatography (silica gel, ethyl acetate / heptane 0-100%) to provide the title compound as an off-white solid (27 mg, 71 % yield). MS (ESI): m/z= 188.1 [M+H]⁺

### Intermediate B10: 4,6-dimethoxy-5-methyl-pyrimidin-2-amine

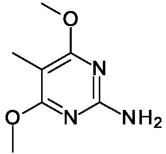

Intermediate B10 is known (CAS 341009-90-3).

### Examples

### Example 1: 6-chloro-N-[5-(2,2-difluoroethoxy)-4,6-dimethoxy-pyrimidin-2-yl]-7-(2-pyrimidyl)-1H-indole-3-sulfonamide

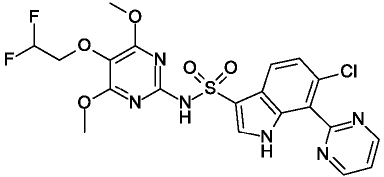

A suspension of 6-chloro-7-(2-pyrimidyl)-1H-indole-3-sulfonyl chloride (Intermediate A1, 50 mg, 117 umol), [5-(2,2-difluoroethoxy)-4,6-dimethoxy-pyrimidin-2-yl]amine (Intermediate B1, 25 mg, 106 umol) and potassium phosphate tribasic (33 mg, 148 umol) in acetonitrile (1.5 ml) was stirred at room temperature for 30 min. n-Ethyl diisopropylamine (21 mg, 28 ul, 159 umol) was added and stirring was continued at room temperature for 10 min. The reaction mixture was poured into water and extracted with ethyl acetate twice. The combined organic layers were dried over sodium sulfate, filtered and concentrated in vacuo. The residue was purified by flash chromatography (silica gel, 0% to 70% ethyl acetate in heptane) to provide the title compound (14 mg, 25% yield) as off-white solid. MS (ESI) m/z: 527.0 [M+H]⁺

The following **Examples 2-7** were prepared in analogy to Example 1 by coupling the indicated sulfonylchloride intermediates A and amine intermediates B.

| Ex. | Structure | Name | Int. A | Int. B | MS (ESI): m/z |
|---|---|---|---|---|---|
| 2 | 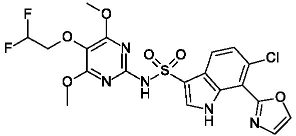 | 6-chloro-N-[5-(2,2-difluoroethoxy)-4,6-dimethoxy-pyrimidin-2-yl]-7-oxazol-2-yl-1H-indole-3-sulfonamide | A2 | B1 | 516.1 [M+H]⁺ |
| 3 | 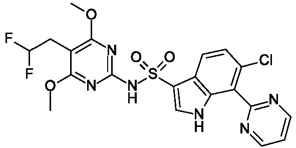 | 6-chloro-N-[5-(2,2-difluoroethyl)-4,6-dimethoxy-pyrimidin-2-yl]-7-(2-pyrimidyl)-1H-indole-3-sulfonamide | A1 | B2 | 511.0 [M+H]⁺ |
| 4 | 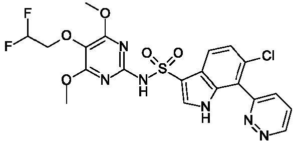 | 6-chloro-N-[5-(2,2-difluoroethoxy)-4,6-dimethoxy-pyrimidin-2-yl]-7-pyridazin-3-yl-1H-indole-3-sulfonamide | A3 | B1 | 527.1 [M+H]⁺ |
| 5 | 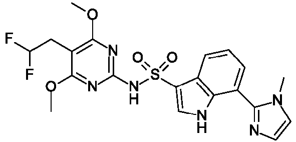 | N-[5-(2,2-difluoroethyl)-4,6-dimethoxy-pyrimidin-2-yl]-7-(1-methylimidazol-2-yl)-1H-indole-3-sulfonamide | A4 | B2 | 478.1 [M+H]⁺ |
| 6 | 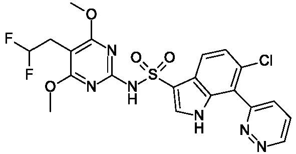 | 6-chloro-N-[5-(2,2-difluoroethyl)-4,6-dimethoxy-pyrimidin-2-yl]-7-pyridazin-3-yl-1H-indole-3-sulfonamide | A3 | B2 | 511.1 [M+H]⁺ |
| 7 | 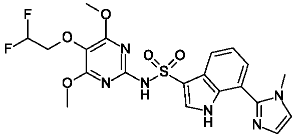 | N-[5-(2,2-difluoroethoxy)-4,6-dimethoxy-pyrimidin-2-yl]-7-(1-methylimidazol-2-yl)-1H-indole-3-sulfonamide | A4 | B1 | 495.2 [M+H]⁺ |

### Example 8: 6-chloro-N-[5-(2,2-difluoroethoxy)-4,6-dimethoxy-pyrimidin-2-yl]-7-(1-methylimidazol-2-yl)-1H-indole-3-sulfonamide

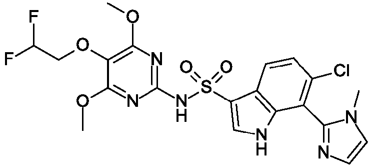

A solution of [5-(2,2-difluoroethoxy)-4,6-dimethoxy-pyrimidin-2-yl]amine (Intermediate B1, 30 mg, 128 umol) in pyridine (1.2 ml) was heated to 80 °C. 6-Chloro-7-(1-methylimidazol-2-yl)-1H-indole-3-sulfonyl chloride (Intermediate A6, 43 mg, 128 umol) was added in 5 portions within 15 min. The reaction mixture was stirred for 5 min at 80 °C. The reaction mixture was concentrated in vacuo and the residue was poured into water and extracted with ethyl acetate twice. The combined organic layers were dried over sodium sulfate, filtered and concentrated in vacuo. The residue was purified by flash chromatography (silica gel, 0% to 100% ethyl acetate in heptane) to provide the title compound (17 mg, 25%) as white solid. MS (ESI) m/z: 529.2 [M+H]⁺

The following **Examples 9-10** were prepared in analogy to Example 8 by coupling the indicated sulfonylchloride intermediates A and amine intermediates B.

| Ex. | Structure | Name | Int. A | Int. B | MS (ESI): m/z |
|---|---|---|---|---|---|
| 9 | 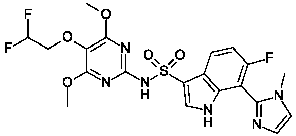 | N-[5-(2,2-difluoroethoxy)-4,6-dimethoxy-pyrimidin-2-yl]-6-fluoro-7-(1-methylimidazol-2-yl)-1H-indole-3-sulfonamide | A14 | B1 | 511.4 [M-H] |
| 10 | 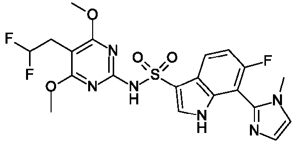 | N-[5-(2,2-difluoroethyl)-4,6-dimethoxy-pyrimidin-2-yl]-6-fluoro-7-(1-methylimidazol-2-yl)-1H-indole-3-sulfonamide | A14 | B2 | 497.3 [M+H]⁺ |

### Example 11: N-[5-(2,2-difluoroethyl)-4,6-dimethoxy-pyrimidin-2-yl]-7-pyridazin-3-yl-1H-indole-3-sulfonamide

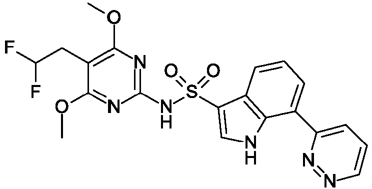

To a stirred solution of [5-(2,2-difluoroethyl)-4,6-dimethoxy-pyrimidin-2-yl]amine (Intermediate B2, 40 mg, 182 umol) and N-ethyl diisopropylamine (48 mg, 64 ul, 365 umol) in dichloromethane (1.2 ml) was added 7-pyridazin-3-yl-1H-indole-3-sulfonyl chloride (Intermediate A7, 62 mg, 201 umol) portionwise at room temperature. The reaction mixture was stirred at room temperature for 3 days. The reaction mixture was poured into water and extracted with ethyl acetate twice. The combined organic layers were dried over sodium sulfate, filtered and concentrated in vacuo. The residue was purified by flash chromatography (silica gel, 0% to 100% ethyl acetate in heptane) to provide the title compound (11 mg, 13% yield) as white solid. MS (ESI) m/z: 477.2 [M+H]⁺

The following **Examples 12-13** were prepared in analogy to Example 11 by coupling the indicated sulfonylchloride intermediates A and amine intermediates B.

| Ex. | Structure | Name | Int. A | Int. B | MS (ESI): m/z |
|---|---|---|---|---|---|
| 12 | 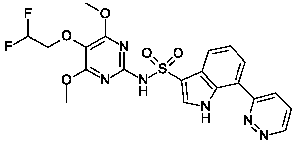 | N-[5-(2,2-difluoroethoxy)-4,6-dimethoxy-pyrimidin-2-yl]-7-pyridazin-3-yl-1H-indole-3-sulfonamide | A7 | B1 | 493.2 [M+H]⁺ |
| 13 | 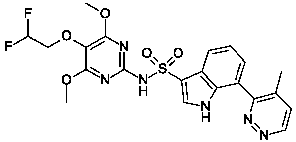 | N-[5-(2,2-difluoroethoxy)-4,6-dimethoxy-pyrimidin-2-yl]-7-(4-methylpyridazin-3-yl)-1H-indole-3-sulfonamide | A8 | B1 | 507.2 [M+H]⁺ |

### Example 14: 6-chloro-N-[5-(2,2-difluoroethoxy)-4,6-dimethoxy-pyrimidin-2-yl]-7-oxazol-4-yl-1H-indole-3-sulfonamide

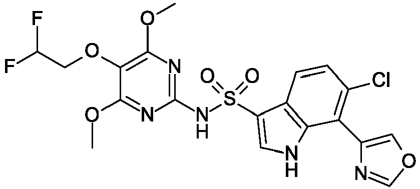

To a stirred solution of [5-(2,2-difluoroethoxy)-4,6-dimethoxy-pyrimidin-2-yl]amine (Intermediate B1, 36 mg, 153 umol) and N-ethyl diisopropylamine (40 mg, 53 ul, 306 umol) in dichloromethane (1 ml) was added a solution of 6-chloro-7-oxazol-4-yl-1H-indole-3-sulfonyl chloride (Intermediate A9, 56 mg, 168 umol) in ethyl acetate (1 ml) dropwise at room temperature. The reaction mixture was stirred at room temperature for 15 min. The mixture was poured into water and extracted with ethyl acetate twice. The combined organic layers were dried over sodium sulfate, filtered and concentrated in vacuo. The residue was purified by flash chromatography (silica gel (12 g, 0% to 60% ethyl acetate in heptane) to provide the title compound (38 mg, 49% yield) as off-white solid. MS (ESI) m/z: 516.2 [M+H]⁺

The following **Examples 15-33** were prepared in analogy to Example 14 by coupling the indicated sulfonylchloride intermediates A and amine intermediates B.

| Ex. | Structure | Name | Int. A | Int. B | MS (ESI): m/z |
|---|---|---|---|---|---|
| 15 | 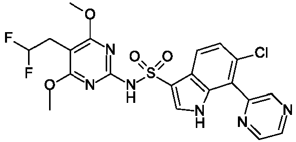 | 6-chloro-N-[5-(2,2-difluoroethyl)-4,6-dimethoxy-pyrimidin-2-yl]-7-pyrazin-2-yl-1H-indole-3-sulfonamide | A10 | B2 | 493.2 [M+H]⁺ |
| 16 | 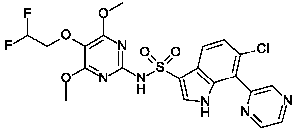 | 6-chloro-N-[5-(2,2-difluoroethoxy)-4,6-dimethoxy-pyrimidin-2-yl]-7-pyrazin-2-yl-1H-indole-3-sulfonamide | A10 | B1 | 527.1 [M+H]⁺ |
| 17 | 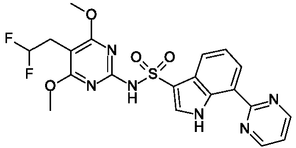 | N-[5-(2,2-difluoroethyl)-4,6-dimethoxy-pyrimidin-2-yl]-7-(2-pyrimidyl)-1H-indole-3-sulfonamide | A11 | B2 | 477.2 [M+H]⁺ |
| 18 | 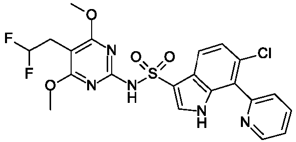 | 6-chloro-N-[5-(2,2-difluoroethyl)-4,6-dimethoxy-pyrimidin-2-yl]-7-(2-pyridyl)-1H-indole-3-sulfonamide | A5 | B2 | 510.2 [M+H]⁺ |
| 19 | 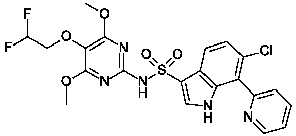 | 6-chloro-N-[5-(2,2-difluoroethoxy)-4,6-dimethoxy-pyrimidin-2-yl]-7-(2-pyridyl)-1H-indole-3-sulfonamide | A5 | B1 | 526.2 [M+H]⁺ |
| 20 | 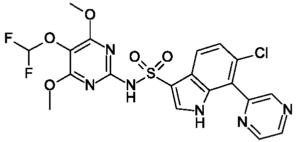 | 6-chloro-N-[5-(difluoromethoxy)-4,6-dimethoxy-pyrimidin-2-yl]-7-pyrazin-2-yl-1H-indole-3-sulfonamide | A10 | B3 | 513.2 [M+H]⁺ |
| 21 | 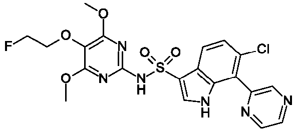 | 6-chloro-N-[5-(2-fluoroethoxy)-4,6-dimethoxy-pyrimidin-2-yl]-7-pyrazin-2-yl-1H-indole-3-sulfonamide | A10 | B4 | 509.2 [M+H]⁺ |
| 22 | 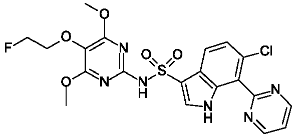 | 6-chloro-N-[5-(2-fluoroethoxy)-4,6-dimethoxy-pyrimidin-2-yl]-7-(2-pyrimidyl)-1H-indole-3-sulfonamide | A1 | B4 | 509.2 [M+H]⁺ |
| 23 | 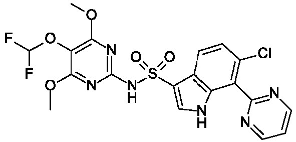 | 6-chloro-N-[5-(difluoromethoxy)-4,6-dimethoxy-pyrimidin-2-yl]-7-(2-pyrimidyl)-1H-indole-3-sulfonamide | A1 | B3 | 513.2 [M+H]⁺ |
| 24 | 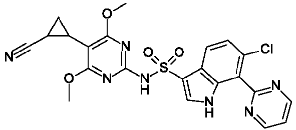 | 6-chloro-N-[5-(2-cyanocyclopropyl)-4,6-dimethoxy-pyrimidin-2-yl]-7-(2-pyrimidyl)-1H-indole-3-sulfonamide | A1 | B5 | 512.3 [M+H]⁺ |
| 25 | 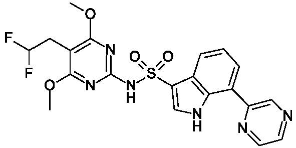 | N-[5-(2,2-difluoroethyl)-4,6-dimethoxy-pyrimidin-2-yl]-7-pyrazin-2-yl-1H-indole-3-sulfonamide | A12 | B2 | 477.2 [M+H]⁺ |
| 26 | 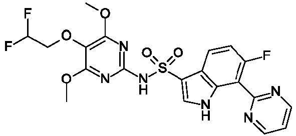 | N-[5-(2,2-difluoroethoxy)-4,6-dimethoxy-pyrimidin-2-yl]-6-fluoro-7-(2-pyrimidyl)-1H-indole-3-sulfonamide | A13 | B1 | 511.3 [M+H]⁺ |
| 27 | 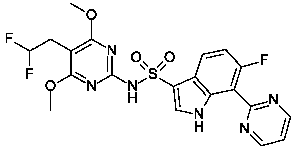 | N-[5-(2,2-difluoroethyl)-4,6-dimethoxy-pyrimidin-2-yl]-6-fluoro-7-(2-pyrimidyl)-1H-indole-3-sulfonamide | A13 | B2 | 495.3 [M+H]⁺ |
| 28 | 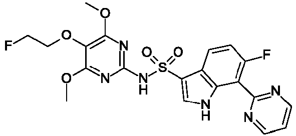 | 6-fluoro-N-[5-(2-fluoroethoxy)-4,6-dimethoxy-pyrimidin-2-yl]-7-(2-pyrimidyl)-1H-indole-3-sulfonamide | A13 | B4 | 493.3 [M+H]⁺ |
| 29 | 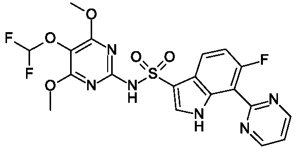 | N-[5-(difluoromethoxy)-4,6-dimethoxy-pyrimidin-2-yl]-6-fluoro-7-(2-pyrimidyl)-1H-indole-3-sulfonamide | A13 | B3 | 497.5 [M+H]⁺ |
| 30 | 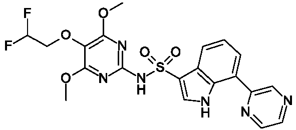 | N-[5-(2,2-difluoroethoxy)-4,6-dimethoxy-pyrimidin-2-yl]-7-pyrazin-2-yl-1H-indole-3-sulfonamide | A12 | B1 | 493.3 [M+H]⁺ |
| 31 | 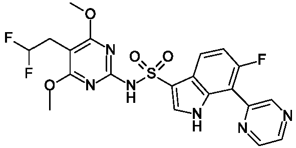 | N-[5-(2,2-difluoroethyl)-4,6-dimethoxy-pyrimidin-2-yl]-6-fluoro-7-pyrazin-2-yl-1H-indole-3-sulfonamide | A15 | B2 | 495.3 [M+H]⁺ |
| 32 | 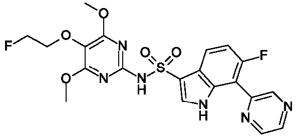 | 6-fluoro-N-[5-(2-fluoroethoxy)-4,6-dimethoxy-pyrimidin-2-yl]-7-pyrazin-2-yl-1H-indole-3-sulfonamide | A15 | B4 | 493.3 [M+H]⁺ |
| 33 | 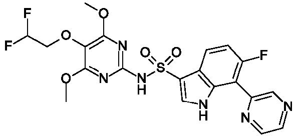 | N-[5-(2,2-difluoroethoxy)-4,6-dimethoxy-pyrimidin-2-yl]-6-fluoro-7-pyrazin-2-yl-1H-indole-3-sulfonamide | A15 | B1 | 511.3 [M+H]⁺ |
| 34 | 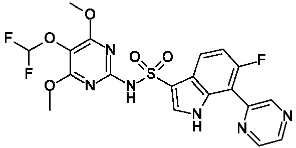 | N-[5-(difluoromethoxy)-4,6-dimethoxy-pyrimidin-2-yl]-6-fluoro-7-pyrazin-2-yl-1H-indole-3-sulfonamide | A15 | B3 | 497.3 [M+H]⁺ |
| 35 | 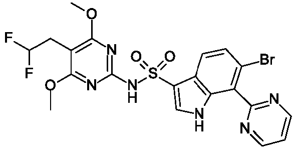 | 6-bromo-N-[5-(2,2-difluoroethyl)-4,6-dimethoxy-pyrimidin-2-yl]-7-(2-pyrimidyl)-1H-indole-3-sulfonamide | A16 | B2 | 557.1 [M+H]⁺ |
| 36 | 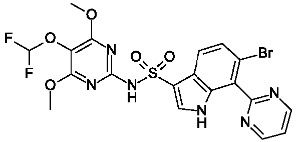 | 6-bromo-N-[5-(difluoromethoxy)-4,6-dimethoxy-pyrimidin-2-yl]-7-(2-pyrimidyl)-1H-indole-3-sulfonamide | A16 | B3 | 559.0 [M+H]⁺ |
| 37 | 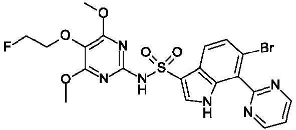 | 6-bromo-N-[5-(2-fluoroethoxy)-4,6-dimethoxy-pyrimidin-2-yl]-7-(2-pyrimidyl)-1H-indole-3-sulfonamide | A16 | B4 | 555.1 [M+H]⁺ |
| 38 | 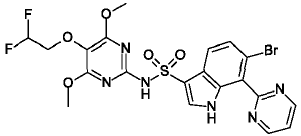 | 6-bromo-N-[5-(2,2-difluoroethoxy)-4,6-dimethoxy-pyrimidin-2-yl]-7-(2-pyrimidyl)-1H-indole-3-sulfonamide | A16 | B1 | 573.0 [M+H]⁺ |
| 39 | 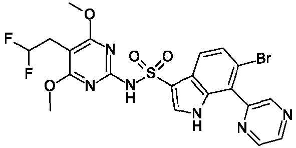 | 6-bromo-N-[5-(2,2-difluoroethyl)-4,6-dimethoxy-pyrimidin-2-yl]-7-pyrazin-2-yl-1H-indole-3-sulfonamide | A17 | B2 | 557.1 [M+H]⁺ |
| 40 | 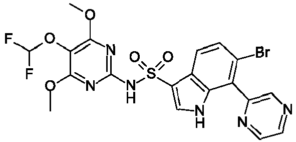 | 6-bromo-N-[5-(difluoromethoxy)-4,6-dimethoxy-pyrimidin-2-yl]-7-pyrazin-2-yl-1H-indole-3-sulfonamide | A17 | B3 | 559.1 [M+H]⁺ |
| 41 | 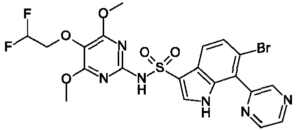 | 6-bromo-N-[5-(2,2-difluoroethoxy)-4,6-dimethoxy-pyrimidin-2-yl]-7-pyrazin-2-yl-1H-indole-3-sulfonamide | A17 | B1 | 573.1 [M+H]⁺ |
| 42 | 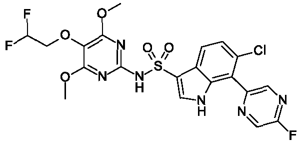 | 6-chloro-N-[5-(2,2-difluoroethoxy)-4,6-dimethoxy-pyrimidin-2-yl]-7-(5-fluoropyrazin-2-yl)-1H-indole-3-sulfonamide | A18 | B1 | 545.1 [M+H]⁺ |
| 43 | 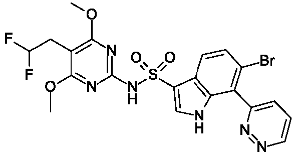 | 6-bromo-N-[5-(2,2-difluoroethyl)-4,6-dimethoxy-pyrimidin-2-yl]-7-pyridazin-3-yl-1H-indole-3-sulfonamide | A19 | B2 | 557.1 [M+H]⁺ |
| 44 | 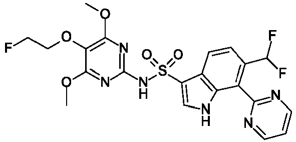 | 6-(difluoromethyl)-N-[5-(2-fluoroethoxy)-4,6-dimethoxy-pyrimidin-2-yl]-7-(2-pyrimidyl)-1H-indole-3-sulfonamide | A20 | B4 | 525.1 [M+H]⁺ |
| 45 | 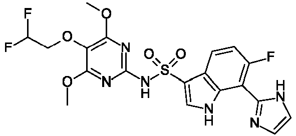 | N-[5-(2,2-difluoroethoxy)-4,6-dimethoxy-pyrimidin-2-yl]-6-fluoro-7-(1H-imidazol-2-yl)-1H-indole-3-sulfonamide | A21 | B1 | 499.3 [M+H]⁺ |

The following **Examples 46-47** were prepared in analogy to Example 8 by coupling the indicated sulfonylchloride intermediates A and amine intermediates B.

| Ex. | Structure | Name | Int. A | Int. B | MS (ESI): m/z |
|---|---|---|---|---|---|
| 46 | 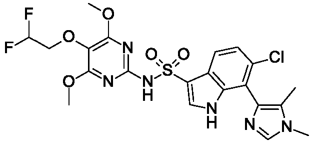 | 6-chloro-N-[5-(2,2-difluoroethoxy)-4,6-dimethoxy-pyrimidin-2-yl]-7-(1,5-dimethylimidazol-4-yl)-1H-indole-3-sulfonamide | A30 | B1 | 543.2 [M+H]⁺ |
| 47 | 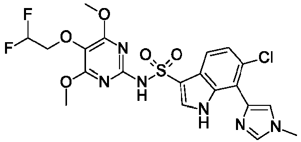 | 6-chloro-N-[5-(2,2-difluoroethoxy)-4,6-dimethoxy-pyrimidin-2-yl]-7-(1-methylimidazol-4-yl)-1H-indole-3-sulfonamide | A37 | B1 | 529.2 [M+H]⁺ |

The following **Examples 48-71** were prepared in analogy to Example 14 by coupling the indicated sulfonylchloride intermediates A and amine intermediates B.

| Ex. | Structure | Name | Int. A | Int. B | MS (ESI): m/z |
|---|---|---|---|---|---|
| 48 | 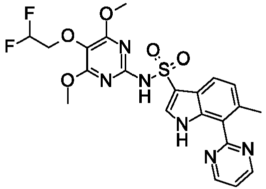 | N-[5-(2,2-difluoroethoxy)-4,6-dimethoxy-pyrimidin-2-yl]-6-methyl-7-(2-pyrimidyl)-1H-indole-3-sulfonamide | A22 | B1 | 507.3 [M+H]⁺ |
| 49 | 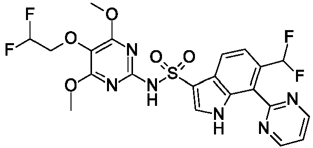 | N-[5-(2,2-difluoroethoxy)-4,6-dimethoxy-pyrimidin-2-yl]-6-(difluoromethyl)-7-(2-pyrimidyl)-1H-indole-3-sulfonamide | A20 | B1 | 543.2 [M+H]⁺ |
| 50 | 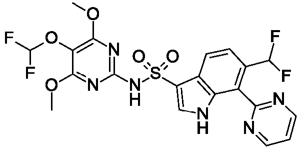 | N-[5-(difluoromethoxy)-4,6-dimethoxy-pyrimidin-2-yl]-6-(difluoromethyl)-7-(2-pyrimidyl)-1H-indole-3-sulfonamide | A20 | B3 | 529.1 [M+H]⁺ |
| 51 | 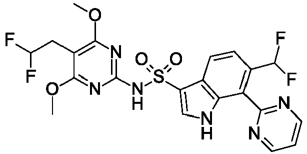 | N-[5-(2,2-difluoroethyl)-4,6-dimethoxy-pyrimidin-2-yl]-6-(difluoromethyl)-7-(2-pyrimidyl)-1H-indole-3-sulfonamide | A20 | B2 | 527.1 [M+H]⁺ |
| 52 | 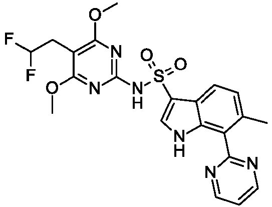 | N-[5-(2,2-difluoroethyl)-4,6-dimethoxy-pyrimidin-2-yl]-6-methyl-7-(2-pyrimidyl)-1H-indole-3-sulfonamide | A22 | B2 | 491.3 [M+H]⁺ |
| 53 | 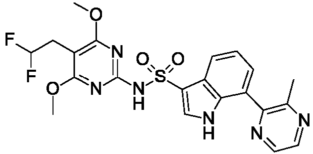 | N-[5-(2,2-difluoroethyl)-4,6-dimethoxy-pyrimidin-2-yl]-7-(3-methylpyrazin-2-yl)-1H-indole-3-sulfonamide | A27 | B2 | 491.3 [M+H]⁺ |
| 54 | 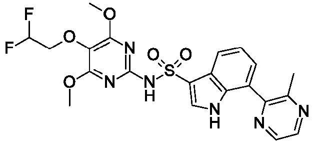 | N-[5-(2,2-difluoroethoxy)-4,6-dimethoxy-pyrimidin-2-yl]-7-(3-methylpyrazin-2-yl)-1H-indole-3-sulfonamide | A27 | B1 | 507.3 [M+H]⁺ |
| 55 | 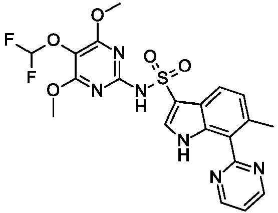 | N-[5-(difluoromethoxy)-4,6-dimethoxy-pyrimidin-2-yl]-6-methyl-7-(2-pyrimidyl)-1H-indole-3-sulfonamide | A22 | B3 | 493.3 [M+H]⁺ |
| 56 | 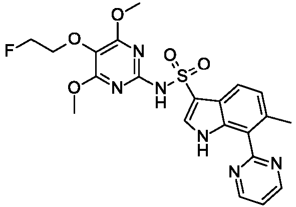 | N-[5-(2-fluoroethoxy)-4,6-dimethoxy-pyrimidin-2-yl]-6-methyl-7-(2-pyrimidyl)-1H-indole-3-sulfonamide | A22 | B4 | 489.3 [M+H]⁺ |
| 57 | 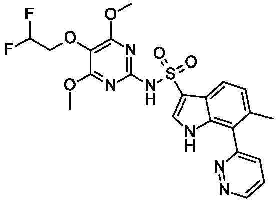 | N-[5-(2,2-difluoroethoxy)-4,6-dimethoxy-pyrimidin-2-yl]-6-methyl-7-pyridazin-3-yl-1H-indole-3-sulfonamide | A29 | B1 | 507.3 [M+H]⁺ |
| 58 | 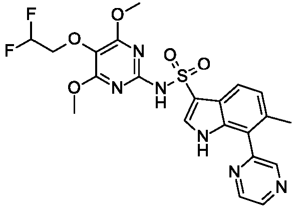 | N-[5-(2,2-difluoroethoxy)-4,6-dimethoxy-pyrimidin-2-yl]-6-methyl-7-pyrazin-2-yl-1H-indole-3-sulfonamide | A31 | B1 | 507.3 [M+H]⁺ |
| 59 | 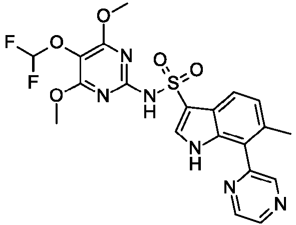 | N-[5-(difluoromethoxy)-4,6-dimethoxy-pyrimidin-2-yl]-6-methyl-7-pyrazin-2-yl-1H-indole-3-sulfonamide | A31 | B3 | 493.3 [M+H]⁺ |
| 60 | 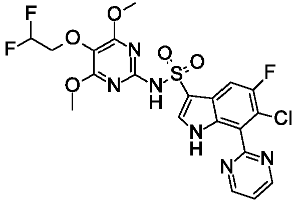 | 6-chloro-N-[5-(2,2-difluoroethoxy)-4,6-dimethoxy-pyrimidin-2-yl]-5-fluoro-7-(2-pyrimidyl)-1H-indole-3-sulfonamide | A34 | B1 | 545.2 [M+H]⁺ |
| 61 | 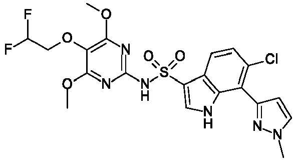 | 6-chloro-N-[5-(2,2-difluoroethoxy)-4,6-dimethoxy-pyrimidin-2-yl]-7-(1-methylpyrazol-3-yl)-1H-indole-3-sulfonamide | A36 | B1 | 529.3 [M+H]⁺ |
| 62 | 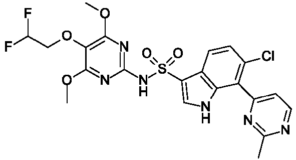 | 6-chloro-N-[5-(2,2-difluoroethoxy)-4,6-dimethoxy-pyrimidin-2-yl]-7-(2-methylpyrimidin-4-yl)-1H-indole-3-sulfonamide | A41 | B1 | 541.1 [M+H]⁺ |
| 63 | 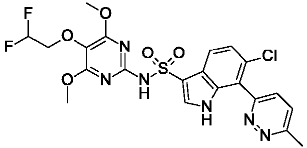 | 6-chloro-N-[5-(2,2-difluoroethoxy)-4,6-dimethoxy-pyrimidin-2-yl]-7-(6-methylpyridazin-3-yl)-1H-indole-3-sulfonamidemide | A44 | B1 | 539.2 [M-H] |
| 64 | 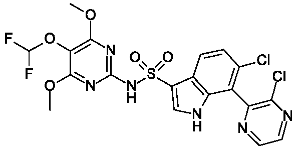 | 6-chloro-7-(3-chloropyrazin-2-yl)-N-[5-(difluoromethoxy)-4,6-dimethoxy-pyrimidin-2-yl]-1H-indole-3-sulfonamide | A45 | B3 | 547.2 [M+H]⁺ |
| 65 | 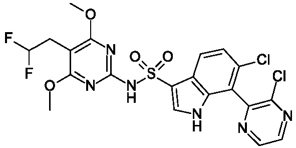 | 6-chloro-7-(3-chloropyrazin-2-yl)-N-[5-(2,2-difluoroethyl)-4,6-dimethoxy-pyrimidin-2-yl]-1H-indole-3-sulfonamide | A45 | B2 | 545.2 [M+H]⁺ |
| 66 | 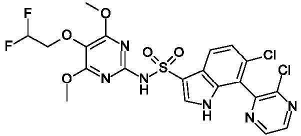 | 6-chloro-7-(3-chloropyrazin-2-yl)-N-[5-(2,2-difluoroethoxy)-4,6-dimethoxy-pyrimidin-2-yl]-1H-indole-3-sulfonamide | A45 | B1 | 561.2 [M+H]⁺ |
| 67 | 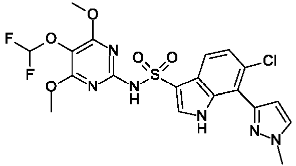 | 6-chloro-N-[5-(difluoromethoxy)-4,6-dimethoxy-pyrimidin-2-yl]-7-(1-methylpyrazol-3-yl)-1H-indole-3-sulfonamide | A36 | B3 | 515.1 [M+H]⁺ |
| 68 | 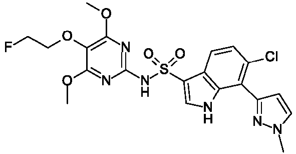 | 6-chloro-N-[5-(2-fluoroethoxy)-4,6-dimethoxy-pyrimidin-2-yl]-7-(1-methylpyrazol-3-yl)-1H-indole-3-sulfonamide | A36 | B4 | 511.2 [M+H]⁺ |
| 69 | 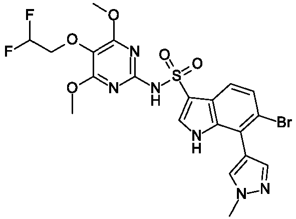 | 6-bromo-N-[5-(2,2-difluoroethoxy)-4,6-dimethoxy-pyrimidin-2-yl]-7-(1-methylpyrazol-4-yl)-1H-indole-3-sulfonamide | A48 | B1 | 575.1 [M+H]⁺ |
| 70 | 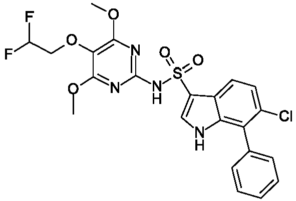 | 6-chloro-N-[5-(2,2-difluoroethoxy)-4,6-dimethoxy-pyrimidin-2-yl]-7-phenyl-1H-indole-3-sulfonamide | A23 | B1 | 525.1 [M+H]⁺ |
| 71 | 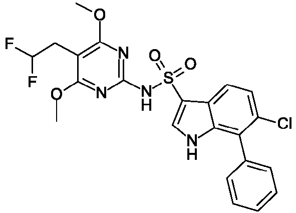 | 6-chloro-N-[5-(2,2-difluoroethyl)-4,6-dimethoxy-pyrimidin-2-yl]-7-phenyl-1H-indole-3-sulfonamide | A23 | B2 | 509.1 [M+H]⁺ |

The following **Examples 72-117** were prepared in analogy to Example 11 by coupling the indicated sulfonylchloride intermediates A and amine intermediates B.

| Ex. | Structure | Name | Int. A | Int. B | MS (ESI): m/z |
|---|---|---|---|---|---|
| 72 | 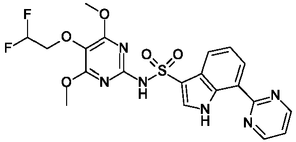 | N-[5-(2,2-difluoroethoxy)-4,6-dimethoxy-pyrimidin-2-yl]-7-(2-pyrimidyl)-1H-indole-3-sulfonamide | A11 | B1 | 493.2 [M+H]⁺ |
| 73 | 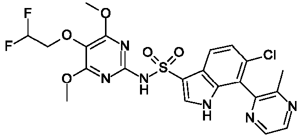 | 6-chloro-N-[5-(2,2-difluoroethoxy)-4,6-dimethoxy-pyrimidin-2-yl]-7-(3-methylpyrazin-2-yl)-1H-indole-3-sulfonamide | A24 | B1 | 541.2 [M+H]⁺ |
| 74 | 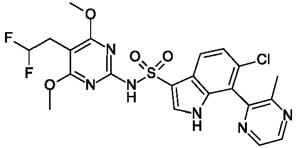 | 6-chloro-N-[5-(2,2-difluoroethyl)-4,6-dimethoxy-pyrimidin-2-yl]-7-(3-methylpyrazin-2-yl)-1H-indole-3-sulfonamide | A24 | B2 | 525.2 [M+H]⁺ |
| 75 | 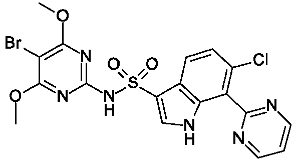 | N-(5-bromo-4,6-dimethoxy-pyrimidin-2-yl)-6-chloro-7-(2-pyrimidyl)-1H-indole-3-sulfonamide | A1 | B6 | 527.0 [M+H]⁺ |
| 76 | 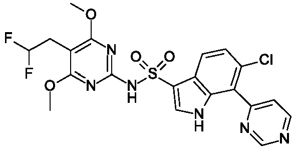 | 6-chloro-N-[5-(2,2-difluoroethyl)-4,6-dimethoxy-pyrimidin-2-yl]-7-(4-pyrimidyl)-1H-indole-3-sulfonamide | A26 | B2 | 511.1 [M+H]⁺ |
| 77 | 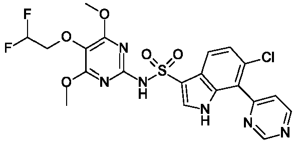 | 6-chloro-N-[5-(2,2-difluoroethoxy)-4,6-dimethoxy-pyrimidin-2-yl]-7-(4-pyrimidyl)-1H-indole-3-sulfonamide | A26 | B1 | 527.1 [M+H]⁺ |
| 78 | 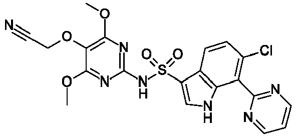 | 6-chloro-N-[5-(cyanomethoxy)-4,6-dimethoxy-pyrimidin-2-yl]-7-(2-pyrimidyl)-1H-indole-3-sulfonamide | A1 | B7 | 502.2 [M+H]⁺ |
| 79 | 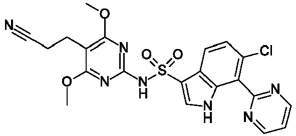 | 6-chloro-N-[5-(2-cyanoethyl)-4,6-dimethoxy-pyrimidin-2-yl]-7-(2-pyrimidyl)-1H-indole-3-sulfonamide | A1 | B8 | 500.3 [M+H]⁺ |
| 80 | 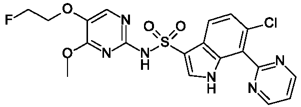 | 6-chloro-N-[5-(2-fluoroethoxy)-4-methoxy-pyrimidin-2-yl]-7-(2-pyrimidyl)-1H-indole-3-sulfonamide | A1 | B9 | 479.2 [M+H]⁺ |
| 81 | 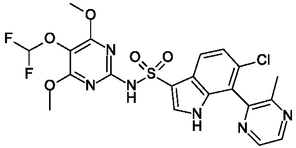 | 6-chloro-N-[5-(difluoromethoxy)-4,6-dimethoxy-pyrimidin-2-yl]-7-(3-methylpyrazin-2-yl)-1H-indole-3-sulfonamide | A24 | B3 | 527.2 [M+H]⁺ |
| 82 | 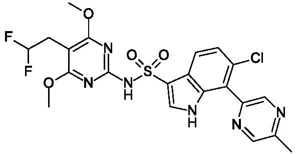 | 6-chloro-N-[5-(2,2-difluoroethyl)-4,6-dimethoxy-pyrimidin-2-yl]-7-(5-methylpyrazin-2-yl)-1H-indole-3-sulfonamide | A32 | B2 | 525.2 [M+H]⁺ |
| 83 | 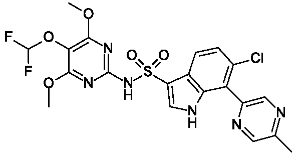 | 6-chloro-N-[5-(difluoromethoxy)-4,6-dimethoxy-pyrimidin-2-yl]-7-(5-methylpyrazin-2-yl)-1H-indole-3-sulfonamide | A32 | B3 | 527.2 [M+H]⁺ |
| 84 | 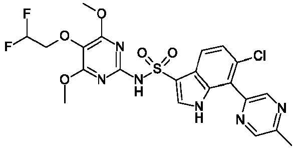 | 6-chloro-N-[5-(2,2-difluoroethoxy)-4,6-dimethoxy-pyrimidin-2-yl]-7-(5-methylpyrazin-2-yl)-1H-indole-3-sulfonamide | A32 | B1 | 541.2 [M+H]⁺ |
| 85 | 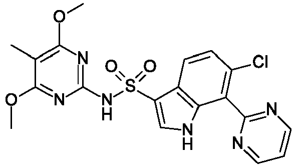 | 6-chloro-N-(4,6-dimethoxy-5-methyl-pyrimidin-2-yl)-7-(2-pyrimidyl)-1H-indole-3-sulfonamide | A1 | B10 | 461.2 [M+H]⁺ |
| 86 | 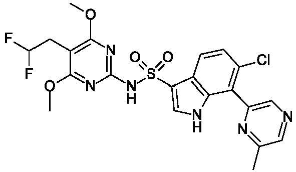 | 6-chloro-N-[5-(2,2-difluoroethyl)-4,6-dimethoxy-pyrimidin-2-yl]-7-(6-methylpyrazin-2-yl)-1H-indole-3-sulfonamide | A33 | B2 | 525.2 [M+H]⁺ |
| 87 | 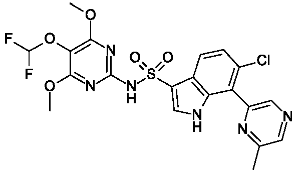 | 6-chloro-N-[5-(difluoromethoxy)-4,6-dimethoxy-pyrimidin-2-yl]-7-(6-methylpyrazin-2-yl)-1H-indole-3-sulfonamide | A33 | B3 | 527.1 [M+H]⁺ |
| 88 | 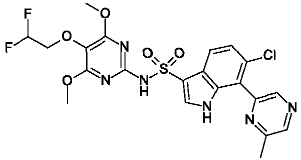 | 6-chloro-N-[5-(2,2-difluoroethoxy)-4,6-dimethoxy-pyrimidin-2-yl]-7-(6-methylpyrazin-2-yl)-1H-indole-3-sulfonamide | A33 | B1 | 541.1 [M+H]⁺ |
| 89 | 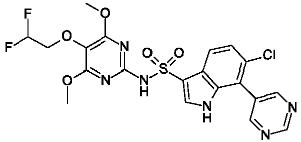 | 6-chloro-N-[5-(2,2-difluoroethoxy)-4,6-dimethoxy-pyrimidin-2-yl]-7-(5-pyrimidyl)-1H-indole-3-sulfonamide | A35 | B1 | 527.2 [M+H]⁺ |
| 90 | 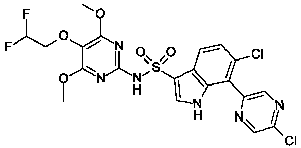 | 6-chloro-7-(5-chloropyrazin-2-yl)-N-[5-(2,2-difluoroethoxy)-4,6-dimethoxy-pyrimidin-2-yl]-1H-indole-3-sulfonamide | A38 | B1 | 561.2 [M+H]⁺ |
| 91 | 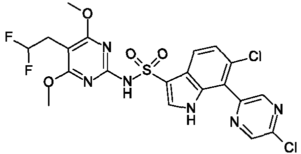 | 6-chloro-7-(5-chloropyrazin-2-yl)-N-[5-(2,2-difluoroethyl)-4,6-dimethoxy-pyrimidin-2-yl]-1H-indole-3-sulfonamide | A38 | B2 | 545.2 [M+H]⁺ |
| 92 | 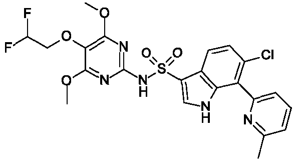 | 6-chloro-N-[5-(2,2-difluoroethoxy)-4,6-dimethoxy-pyrimidin-2-yl]-7-(6-methyl-2-pyridyl)-1H-indole-3-sulfonamide | A39 | B1 | 538.2 [M-H] |
| 93 | 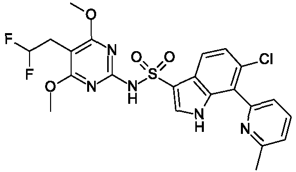 | 6-chloro-N-[5-(2,2-difluoroethyl)-4,6-dimethoxy-pyrimidin-2-yl]-7-(6-methyl-2-pyridyl)-1H-indole-3-sulfonamide | A39 | B2 | 524.2 [M+H]⁺ |
| 94 | 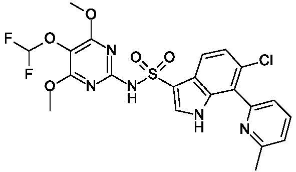 | 6-chloro-N-[5-(difluoromethoxy)-4,6-dimethoxy-pyrimidin-2-yl]-7-(6-methyl-2-pyridyl)-1H-indole-3-sulfonamide | A39 | B3 | 524.1 [M-H] |
| 95 | 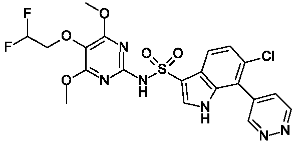 | 6-chloro-N-[5-(2,2-difluoroethoxy)-4,6-dimethoxy-pyrimidin-2-yl]-7-pyridazin-4-yl-1H-indole-3-sulfonamide | A40 | B1 | 527.2 [M+H]⁺ |
| 96 | 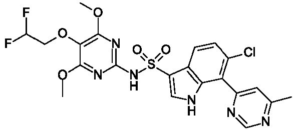 | 6-chloro-N-[5-(2,2-difluoroethoxy)-4,6-dimethoxy-pyrimidin-2-yl]-7-(6-methylpyrimidin-4-yl)-1H-indole-3-sulfonamide | A42 | B1 | 541.1 [M+H]⁺ |
| 97 | 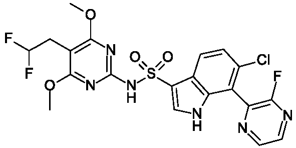 | 6-chloro-N-[5-(2,2-difluoroethyl)-4,6-dimethoxy-pyrimidin-2-yl]-7-(3 -fluoropyrazin-2-yl)-1H-indole-3-sulfonamide | A43 | B2 | 529.2 [M+H]⁺ |
| 98 | 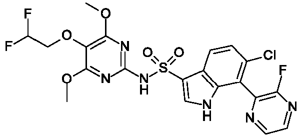 | 6-chloro-N-[5-(2,2-difluoroethoxy)-4,6-dimethoxy-pyrimidin-2-yl]-7-(3-fluoropyrazin-2-yl)-1H-indole-3-sulfonamide | A43 | B1 | 545.2 [M+H]⁺ |
| 99 | 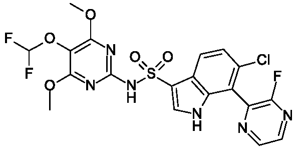 | 6-chloro-N-[5-(difluoromethoxy)-4,6-dimethoxy-pyrimidin-2-yl]-7-(3-fluoropyrazin-2-yl)-1H-indole-3-sulfonamide | A43 | B3 | 529.2 [M-H] |
| 100 | 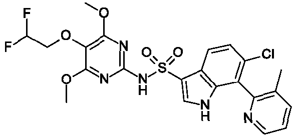 | 6-chloro-N-[5-(2,2-difluoroethoxy)-4,6-dimethoxy-pyrimidin-2-yl]-7-(3-methyl-2-pyridyl)-1H-indole-3-sulfonamide | A46 | B1 | 540.2 [M+H]⁺ |
| 101 | 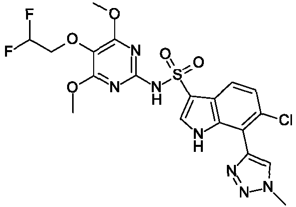 | 6-chloro-N-[5-(2,2-difluoroethoxy)-4,6-dimethoxy-pyrimidin-2-yl]-7-(1-methyltriazol-4-yl)-1H-indole-3-sulfonamide | A47 | B1 | 530.2 [M+H]⁺ |
| 102 | 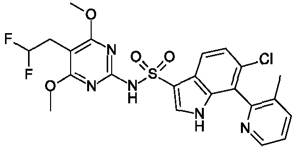 | 6-chloro-N-[5-(2,2-difluoroethyl)-4,6-dimethoxy-pyrimidin-2-yl]-7-(3-methyl-2-pyridyl)-1H-indole-3-sulfonamide | A46 | B2 | 524.2 [M+H]⁺ |
| 103 | 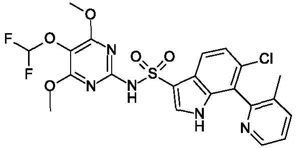 | 6-chloro-N-[5-(difluoromethoxy)-4,6-dimethoxy-pyrimidin-2-yl]-7-(3-methyl-2-pyridyl)-1H-indole-3-sulfonamide | A46 | B3 | 526.3 [M+H]⁺ |
| 104 | 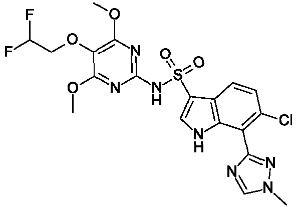 | 6-chloro-N-[5-(2,2-difluoroethoxy)-4,6-dimethoxy-pyrimidin-2-yl]-7-(1-methyl-1,2,4-triazol-3-yl)-1H-indole-3-sulfonamide | A49 | B1 | 530.2 [M+H]⁺ |
| 105 | 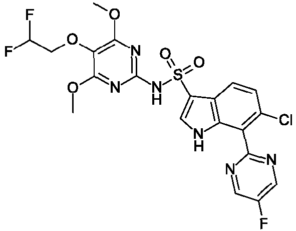 | 6-chloro-N-[5-(2,2-difluoroethoxy)-4,6-dimethoxy-pyrimidin-2-yl]-7-(5-fluoropyrimidin-2-yl)-1H-indole-3-sulfonamide | A50 | B1 | 545.1 [M+H]⁺ |
| 106 | 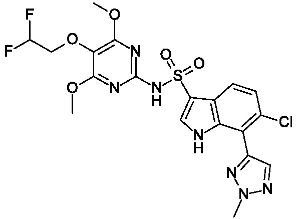 | 6-chloro-N-[5-(2,2-difluoroethoxy)-4,6-dimethoxy-pyrimidin-2-yl]-7-(2-methyltriazol-4-yl)-1H-indole-3-sulfonamide | A51 | B1 | 530.2 [M+H]⁺ |
| 107 | 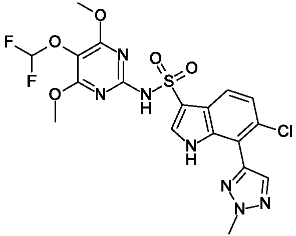 | 6-chloro-N-[5-(difluoromethoxy)-4,6-dimethoxy-pyrimidin-2-yl]-7-(2-methyltriazol-4-yl)-1H-indole-3-sulfonamide | A51 | B3 | 516.2 [M+H]⁺ |
| 108 | 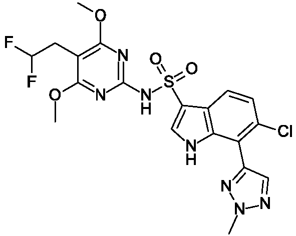 | 6-chloro-N-[5-(2,2-difluoroethyl)-4,6-dimethoxy-pyrimidin-2-yl]-7-(2-methyltriazol-4-yl)-1H-indole-3-sulfonamide | A51 | B2 | 514.2 [M+H]⁺ |
| 109 | 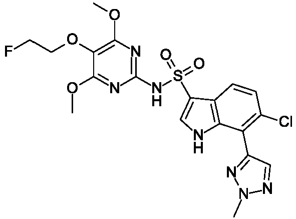 | 6-chloro-N-[5-(2-fluoroethoxy)-4,6-dimethoxy-pyrimidin-2-yl]-7-(2-methyltriazol-4-yl)-1H-indole-3-sulfonamide | A51 | B4 | 512.2 [M+H]⁺ |
| 110 | 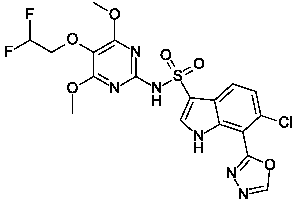 | 6-chloro-N-[5-(2,2-difluoroethoxy)-4,6-dimethoxy-pyrimidin-2-yl]-7-(1,3,4-oxadiazol-2-yl)-1H-indole-3-sulfonamide | A52 | B1 | 517.1 [M+H]⁺ |
| 111 | 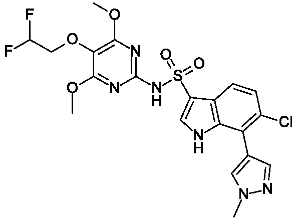 | 6-chloro-N-[5-(2,2-difluoroethoxy)-4,6-dimethoxy-pyrimidin-2-yl]-7-(1-methylpyrazol-4-yl)-1H-indole-3-sulfonamide | A53 | B1 | 529.2 [M+H]⁺ |
| 112 | 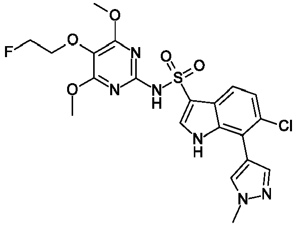 | 6-chloro-N-[5-(2-fluoroethoxy)-4,6-dimethoxy-pyrimidin-2-yl]-7-(1-methylpyrazol-4-yl)-1H-indole-3-sulfonamide | A53 | B4 | 511.1 [M+H]⁺ |
| 113 | 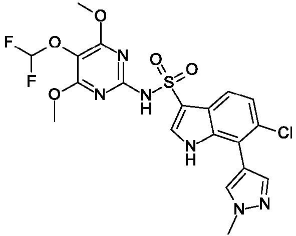 | 6-chloro-N-[5-(difluoromethoxy)-4,6-dimethoxy-pyrimidin-2-yl]-7-(1-methylpyrazol-4-yl)-1H-indole-3-sulfonamide | A53 | B3 | 515.1 [M+H]⁺ |
| 114 | 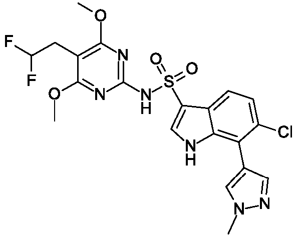 | 6-chloro-N-[5-(2,2-difluoroethyl)-4,6-dimethoxy-pyrimidin-2-yl]-7-(1-methylpyrazol-4-yl)-1H-indole-3-sulfonamide | A53 | B2 | 513.2 [M+H]⁺ |
| 115 | 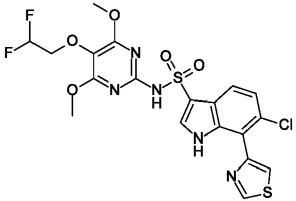 | 6-chloro-N-[5-(2,2-difluoroethoxy)-4,6-dimethoxy-pyrimidin-2-yl]-7-thiazol-4-yl-1H-indole-3-sulfonamide | A54 | B1 | 532.1 [M+H]⁺ |
| 116 | 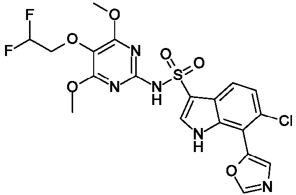 | 6-chloro-N-[5-(2,2-difluoroethoxy)-4,6-dimethoxy-pyrimidin-2-yl]-7-oxazol-5-yl-1H-indole-3-sulfonamide | A25 | B1 | 516.0 [M+H]⁺ |
| 117 | 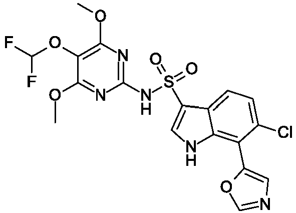 | 6-chloro-N-[5-(difluoromethoxy)-4,6-dimethoxy-pyrimidin-2-yl]-7-oxazol-5-yl-1H-indole-3-sulfonamide | A25 | B3 | 502.1 [M+H]⁺ |

### Example 118: 6-chloro-N-[5-(2,2-difluoroethoxy)-4,6-dimethoxy-pyrimidin-2-yl]-7-isoxazol-4-yl-1H-indole-3-sulfonamide

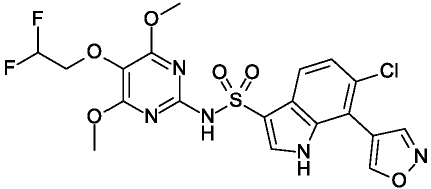

To a solution of [5-(2,2-difluoroethoxy)-4,6-dimethoxy-pyrimidin-2-yl]amine (Intermediate B1, 5 mg, 21 umol) in acetonitrile (100 ul) was added pyridine (100 ul) and 6-chloro-7-isoxazol-4-yl-1H-indole-3-sulfonyl chloride (Intermediate A28, 10 mg, 25.5 umol) and the resulting mixture was stirred for 7 h at room temperature. Water was added and the mixture was extracted two times with ethyl acetate. The combined organic layers were washed with brine, dried over sodium sulfate, filtered and then concentrated. The residue was purified by chromatography (silica gel, methanol in dichloromethane 0% to 15%) to give the title compound (49 mg, 55% yield) as white solid, MS (ESI) m/z: 516.0 [M+H]⁺.

The following **Examples 119-120** were prepared in analogy to Example 118 by coupling the indicated sulfonylchloride intermediates A and amine intermediates B.

| Ex. | Structure | Name | Int. A | Int. B | MS (ESI): m/z |
|---|---|---|---|---|---|
| 119 | 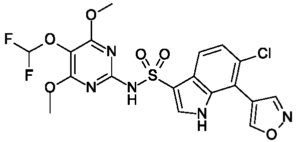 | 6-chloro-N-[5-(difluoromethoxy)-4,6-dimethoxy-pyrimidin-2-yl]-7-isoxazol-4-yl-1H-indole-3-sulfonamide | A28 | B3 | 502.0 [M+H]⁺ |
| 120 | 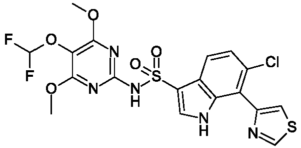 | 6-chloro-N-[5-(difluoromethoxy)-4,6-dimethoxy-pyrimidin-2-yl]-7-thiazol-4-yl-1H-indole-3-sulfonamide | A54 | B3 | 518.0 [M+H]⁺ |

### Example 121: N-[5-(2,2-difluoroethyl)-4,6-dimethoxy-pyrimidin-2-yl]-7-(6,7-dihydro-5H-pyrrol[1,2-c]imidazol-3-yl)-1H-indole-3-sulfonamide

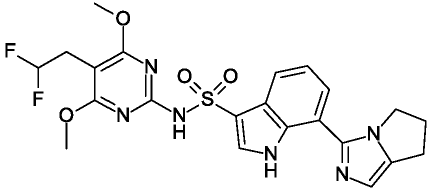

### Step 1: 7-(4,4,5,5-tetramethyl-1,3,2-dioxaborolan-2-yl)-1H-indole-3-sulfonyl chloride

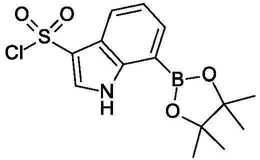

A solution of 7-(4,4,5,5-tetramethyl-1,3,2-dioxaborolan-2-yl)-1H-indole (CAS 642494-37-9, 200 mg, 0.80 mmol) in acetonitrile (3 ml) was cooled to 0 °C. Chlorosulfonic acid (242 mg, 140 ul, 2.07 mmol) was added dropwise. The reaction mixture was stirred at 0 °C for 2 h, then poured into ice/ethyl acetate and extracted with ethyl acetate twice. The combined organic layers were dried over Na2SO4, filtered and concentrated in vacuo to provide 7-(4,4,5,5-tetramethyl-1,3,2-dioxaborolan-2-yl)-1H-indole-3-sulfonyl chloride (204 mg, 56% yield) as light red gum. MS (ESI) m/z: 340.2 [M-H]⁻.

### Step 2: N-[5-(2,2-difluoroethyl)-4,6-dimethoxy-pyrimidin-2-yl]-7-(4,4,5,5-tetramethyl-1,3,2-dioxaborolan-2-yl)-1H-indole-3-sulfonamide

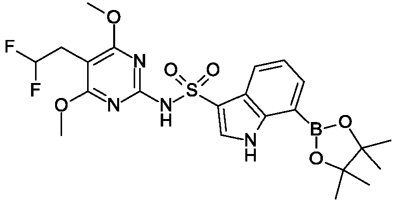

To a stirred solution of [5-(2,2-difluoroethyl)-4,6-dimethoxy-pyrimidin-2-yl]amine (Intermediate B2, 88 mg, 0.40 mmol) and N-ethyldiisopropylamine (159 mg, 210 ul, 1.2 mmol) in dichloromethane (3 ml) was added a solution of 7-(4,4,5,5-tetramethyl-1,3,2-dioxaborolan-2-yl)-1H-indole-3-sulfonyl chloride (201 mg, 0.44 mmol) dropwise at room temperature. The reaction mixture was stirred at room temperature for 15 h. The reaction mixture was poured into brine and extracted twice with ethyl acetate. The combined organic layers were dried over Na2SO4, filtered and concentrated in vacuo. The residue was purified by flash chromatography (silica gel, 0% to 45% ethyl acetate in heptane) to provide N-[5-(2,2-difluoroethyl)-4,6-dimethoxy-pyrimidin-2-yl]-7-(4,4,5,5-tetramethyl-1,3,2-dioxaborolan-2-yl)-1H-indole-3-sulfonamide (53 mg, 25% yield) as white solid. MS (ESI) m/z: 525.3 [M+H]⁺.

### Step 3: 3-iodo-6,7-dihydro-5H-pyrrolo[1,2-c]imidazole

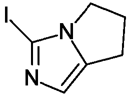

To the stirred solution of 6,7-dihydro-5H-pyrrolo[1,2-c]imidazole (CAS 52237-16-8, 500 mg, 4.62 mmol) in dry tetrahydrofuran (8 ml) n-butyllithium solution (2.5 M in hexanes, 2.2 ml, 5.55 mmol) was added at -78°C and the mixture was stirred at this temperature for 40 min. Then a solution of molecular iodine (1.52 g, 6.01 mmol) in dry tetrahydrofuran (4 ml) was slowly added and mixture was stirred at -78°C for 40 min. The mixture was quenched with ammonium chloride solution (15 ml) and extracted with ethyl acetate (25 ml). The organic layer was washed with aqueous sodium thiosulfate solution (15 ml) and brine, dried over Na2SO4 and evaporated. The residue was purified by flash chromatography (silica gel, 0% to 30% ethyl acetate in heptane) to give 3-iodo-6,7-dihydro-5H-pyrrolo[1,2-c]imidazole (600 mg, 55% yield) as white solid, MS (ESI) m/z: 235.0 [M+H]⁺.

### Step 4: N-[5-(2,2-difluoroethyl)-4,6-dimethoxy-pyrimidin-2-yl]-7-(6,7-dihydro-5H-pyrrol[1,2-c]imidazol-3-yl)-1H-indole-3-sulfonamide

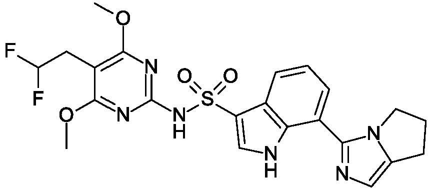

A mixture of N-[5-(2,2-difluoroethyl)-4,6-dimethoxy-pyrimidin-2-yl]-7-(4,4,5,5-tetramethyl-1,3,2-dioxaborolan-2-yl)-1H-indole-3-sulfonamide (51 mg, 97 umol), 3-iodo-6,7-dihydro-5H-pyrrol[1,2-c]imidazole (25 mg, 107 umol), cesium carbonate (64 mg, 194 umol) and tetrakis(triphenylphosphine)palladium(0) (11 mg, 10 umol, 0.10 eq) in 1,4-dioxane (500 ul) and water (250 ul) was heated to 110 °C and stirred for 15 h. The reaction mixture was poured into brine and extracted twice with ethyl acetate. The organic layers were dried over Na2SO4, filtered and concentrated in vacuo. The residue was purified by flash chromatography (silica gel, 0% to 100% ethyl acetate in heptane) followed by another flash chromatography (column C18 RediSep Rf Gold, 10% to 60% acetonitrile in water) to provide the title compound (4.5 mg, 9% yield) as white solid, MS (ESI) m/z: 503.2 [M-H]⁻.

### Example A

A compound of formula I can be used in a manner known per se as the active ingredient for the production of tablets of the following composition:

| | Per tablet |
|---|---|
| Active ingredient | 200 mg |
| Microcrystalline cellulose | 155 mg |
| Corn starch | 25 mg |
| Talc | 25 mg |
| Hydroxypropylmethylcellulose | 20 mg |
| | 425 mg |

### Example B

A compound of formula I can be used in a manner known per se as the active ingredient for the production of capsules of the following composition:

| | Per capsule |
|---|---|
| Active ingredient | 100.0 mg |
| Corn starch | 20.0 mg |
| Lactose | 95.0 mg |
| Talc | 4.5 mg |
| Magnesium stearate | 0.5 mg |
| | 220.0 mg |

## Claims

1. Compounds of formula I 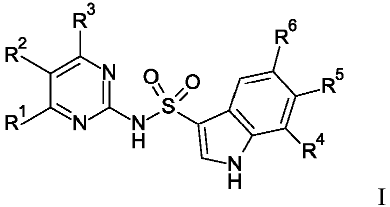 wherein
R¹ is alkoxy or haloalkoxy;
R² is halo, alkyl, alkoxy, haloalkyl, haloalkoxy, cyanoalkyl, cyanoalkoxy or cyclopropyl optional substituted with up to two substituents independently selected from cyano and halo;
R³ is H, alkoxy or haloalkoxy;
R⁵ is H, halo, alkyl, or haloalkyl;
R⁶ is H or halo;
R⁴ is a C-linked aryl or a C-linked heteroaryl, wherein the C-linked aryl or C-linked heteroaryl is optionally substituted by one or more substituents independently selected from alkyl, halo, and haloalkyl;
and pharmaceutically acceptable salts.

2. A compound according to claim 1, wherein R¹ is alkoxy.

3. A compound according to claim 1 or claim 2, wherein R² is halo, alkyl, haloalkyl, haloalkoxy, cyanoalkyl, cyanoalkoxy or cyclopropyl substituted with cyano.

4. A compound according to any of claims 1 to 3, wherein R³ is H or alkoxy.

5. A compound according to any of claims 1 to 4, wherein R⁴ is a C-linked aryl or a C-linked heteroaryl, wherein the C-linked aryl or C-linked heteroaryl is optionally substituted by one or more substituents independently selected from alkyl and halo.

6. A compound according to any of claims 1 to 5, wherein R⁴ is selected from
i. a C-linked 6-member aryl;
ii. a C-linked 6-member heteroaryl comprising 1 to 2 nitrogen heteroatoms optionally substituted with alkyl or halo;
iii. a C-linked 5-member heteroaryl comprising 1-3 heteroatoms independently selected from N, S and O, optionally substituted with 1 to 2 substituents independently selected from alkyl and halo; and
iv. a C-linked 8-membered bicyclic heteroaryl system comprising 2 N heteroatoms.

7. A compound according to claim 1, wherein
R¹ is alkoxy;
R² is halo, alkyl, haloalkyl, haloalkoxy, cyanoalkyl, cyanoalkoxy or cyclopropyl substituted with cyano;
R³ is H or alkoxy;
R⁵ is H, halo, alkyl, or haloalkyl;
R⁶ is H or halo;
R⁴ is a C-linked aryl or a C-linked heteroaryl, wherein the C-linked aryl or C-linked heteroaryl is optionally substituted by one or more substituents independently selected from alkyl and halo;
and pharmaceutically acceptable salts.

8. A compound according to claim 1, wherein
R¹ is alkoxy;
R² is halo, alkyl, haloalkyl, haloalkoxy, cyanoalkyl, cyanoalkoxy or cyclopropyl substituted with cyano;
R³ is H or alkoxy;
R⁵ is H, halo, alkyl, or haloalkyl;
R⁶ is H or halo;
R⁴ is selected from
i. a C-linked 6-member aryl;
ii. a C-linked 6-member heteroaryl comprising 1 to 2 nitrogen heteroatoms optionally substituted with alkyl or halo;
iii. a C-linked 5-member heteroaryl comprising 1-3 heteroatoms independently selected from N, S and O, optionally substituted with 1 to 2 substituents independently selected from alkyl and halo; and
iv. a C-linked 8-membered bicyclic heteroaryl system comprising 2 N heteroatoms;
and pharmaceutically acceptable salts

9. A compound according to any of claims 1 to 8, selected from
6-chloro-N-[5-(2,2-difluoroethoxy)-4,6-dimethoxy-pyrimidin-2-yl]-7-(2-pyrimidyl)-1H-indole-3-sulfonamide;
6-chloro-N-[5-(2,2-difluoroethoxy)-4,6-dimethoxy-pyrimidin-2-yl]-7-oxazol-2-yl-1H-indole-3-sulfonamide;
6-chloro-N-[5-(2,2-difluoroethyl)-4,6-dimethoxy-pyrimidin-2-yl]-7-(2-pyrimidyl)-1H-indole-3-sulfonamide;
6-chloro-N-[5-(2,2-difluoroethoxy)-4,6-dimethoxy-pyrimidin-2-yl]-7-pyridazin-3-yl-1H-indole-3-sulfonamide;
N-[5-(2,2-difluoroethyl)-4,6-dimethoxy-pyrimidin-2-yl]-7-(1-methylimidazol-2-yl)-1H-indole-3-sulfonamide;
6-chloro-N-[5-(2,2-difluoroethyl)-4,6-dimethoxy-pyrimidin-2-yl]-7-pyridazin-3-yl-1H-indole-3-sulfonamide;
N-[5-(2,2-difluoroethoxy)-4,6-dimethoxy-pyrimidin-2-yl]-7-(1-methylimidazol-2-yl)-1H-indole-3-sulfonamide;
6-chloro-N-[5-(2,2-difluoroethoxy)-4,6-dimethoxy-pyrimidin-2-yl]-7-(1-methylimidazol-2-yl)-1H-indole-3-sulfonamide;
N-[5-(2,2-difluoroethoxy)-4,6-dimethoxy-pyrimidin-2-yl]-6-fluoro-7-(1-methylimidazol-2-yl)-1H-indole-3-sulfonamide;
N-[5-(2,2-difluoroethyl)-4,6-dimethoxy-pyrimidin-2-yl]-6-fluoro-7-(1-methylimidazol-2-yl)-1H-indole-3-sulfonamide;
N-[5-(2,2-difluoroethyl)-4,6-dimethoxy-pyrimidin-2-yl]-7-pyridazin-3-yl-1H-indole-3-sulfonamide;
N-[5-(2,2-difluoroethoxy)-4,6-dimethoxy-pyrimidin-2-yl]-7-pyridazin-3-yl-1H-indole-3-sulfonamide;
N-[5-(2,2-difluoroethoxy)-4,6-dimethoxy-pyrimidin-2-yl]-7-(4-methylpyridazin-3-yl)-1H-indole-3-sulfonamide;
6-chloro-N-[5-(2,2-difluoroethoxy)-4,6-dimethoxy-pyrimidin-2-yl]-7-oxazol-4-yl-1H-indole-3-sulfonamide;
6-chloro-N-[5-(2,2-difluoroethyl)-4,6-dimethoxy-pyrimidin-2-yl]-7-pyrazin-2-yl-1H-indole-3-sulfonamide;
6-chloro-N-[5-(2,2-difluoroethoxy)-4,6-dimethoxy-pyrimidin-2-yl]-7-pyrazin-2-yl-1H-indole-3-sulfonamide;
N-[5-(2,2-difluoroethyl)-4,6-dimethoxy-pyrimidin-2-yl]-7-(2-pyrimidyl)-1H-indole-3-sulfonamide;
6-chloro-N-[5-(2,2-difluoroethyl)-4,6-dimethoxy-pyrimidin-2-yl]-7-(2-pyridyl)-1H-indole-3-sulfonamide;
6-chloro-N-[5-(2,2-difluoroethoxy)-4,6-dimethoxy-pyrimidin-2-yl]-7-(2-pyridyl)-1H-indole-3-sulfonamide;
6-chloro-N-[5-(difluoromethoxy)-4,6-dimethoxy-pyrimidin-2-yl]-7-pyrazin-2-yl-1H-indole-3-sulfonamide;
6-chloro-N-[5-(2-fluoroethoxy)-4,6-dimethoxy-pyrimidin-2-yl]-7-pyrazin-2-yl-1H-indole-3-sulfonamide;
6-chloro-N-[5-(2-fluoroethoxy)-4,6-dimethoxy-pyrimidin-2-yl]-7-(2-pyrimidyl)-1H-indole-3-sulfonamide;
6-chloro-N-[5-(difluoromethoxy)-4,6-dimethoxy-pyrimidin-2-yl]-7-(2-pyrimidyl)-1H-indole-3-sulfonamide;
6-chloro-N-[5-(2-cyanocyclopropyl)-4,6-dimethoxy-pyrimidin-2-yl]-7-(2-pyrimidyl)-1H-indole-3-sulfonamide;
N-[5-(2,2-difluoroethyl)-4,6-dimethoxy-pyrimidin-2-yl]-7-pyrazin-2-yl-1H-indole-3-sulfonamide;
N-[5-(2,2-difluoroethoxy)-4,6-dimethoxy-pyrimidin-2-yl]-6-fluoro-7-(2-pyrimidyl)-1H-indole-3-sulfonamide;
N-[5-(2,2-difluoroethyl)-4,6-dimethoxy-pyrimidin-2-yl]-6-fluoro-7-(2-pyrimidyl)-1H-indole-3-sulfonamide;
6-fluoro-N-[5-(2-fluoroethoxy)-4,6-dimethoxy-pyrimidin-2-yl]-7-(2-pyrimidyl)-1H-indole-3-sulfonamide;
N-[5-(difluoromethoxy)-4,6-dimethoxy-pyrimidin-2-yl]-6-fluoro-7-(2-pyrimidyl)-1H-indole-3-sulfonamide;
N-[5-(2,2-difluoroethoxy)-4,6-dimethoxy-pyrimidin-2-yl]-7-pyrazin-2-yl-1H-indole-3-sulfonamide;
N-[5-(2,2-difluoroethyl)-4,6-dimethoxy-pyrimidin-2-yl]-6-fluoro-7-pyrazin-2-yl-1H-indole-3-sulfonamide;
6-fluoro-N-[5-(2-fluoroethoxy)-4,6-dimethoxy-pyrimidin-2-yl]-7-pyrazin-2-yl-1H-indole-3-sulfonamide;
N-[5-(2,2-difluoroethoxy)-4,6-dimethoxy-pyrimidin-2-yl]-6-fluoro-7-pyrazin-2-yl-1H-indole-3-sulfonamide;
N-[5-(difluoromethoxy)-4,6-dimethoxy-pyrimidin-2-yl]-6-fluoro-7-pyrazin-2-yl-1H-indole-3-sulfonamide;
6-bromo-N-[5-(2,2-difluoroethyl)-4,6-dimethoxy-pyrimidin-2-yl]-7-(2-pyrimidyl)-1H-indole-3-sulfonamide;
6-bromo-N-[5-(difluoromethoxy)-4,6-dimethoxy-pyrimidin-2-yl]-7-(2-pyrimidyl)-1H-indole-3-sulfonamide;
6-bromo-N-[5-(2-fluoroethoxy)-4,6-dimethoxy-pyrimidin-2-yl]-7-(2-pyrimidyl)-1H-indole-3-sulfonamide;
6-bromo-N-[5-(2,2-difluoroethoxy)-4,6-dimethoxy-pyrimidin-2-yl]-7-(2-pyrimidyl)-1H-indole-3-sulfonamide;
6-bromo-N-[5-(2,2-difluoroethyl)-4,6-dimethoxy-pyrimidin-2-yl]-7-pyrazin-2-yl-1H-indole-3-sulfonamide;
6-bromo-N-[5-(difluoromethoxy)-4,6-dimethoxy-pyrimidin-2-yl]-7-pyrazin-2-yl-1H-indole-3-sulfonamide;
6-bromo-N-[5-(2,2-difluoroethoxy)-4,6-dimethoxy-pyrimidin-2-yl]-7-pyrazin-2-yl-1H-indole-3-sulfonamide;
6-chloro-N-[5-(2,2-difluoroethoxy)-4,6-dimethoxy-pyrimidin-2-yl]-7-(5-fluoropyrazin-2-yl)-1H-indole-3-sulfonamide;
6-bromo-N-[5-(2,2-difluoroethyl)-4,6-dimethoxy-pyrimidin-2-yl]-7-pyridazin-3-yl-1H-indole-3-sulfonamide;
6-(difluoromethyl)-N-[5-(2-fluoroethoxy)-4,6-dimethoxy-pyrimidin-2-yl]-7-(2-pyrimidyl)-1H-indole-3-sulfonamide;
N-[5-(2,2-difluoroethoxy)-4,6-dimethoxy-pyrimidin-2-yl]-6-fluoro-7-(1H-imidazol-2-yl)-1H-indole-3-sulfonamide; and
pharmaceutically acceptable salts thereof.

10. A compound according to any one of claims 1 to 8 selected from
6-chloro-N-[5-(2,2-difluoroethoxy)-4,6-dimethoxy-pyrimidin-2-yl]-7-(1,5-dimethylimidazol-4-yl)-1H-indole-3-sulfonamide;
6-chloro-N-[5-(2,2-difluoroethoxy)-4,6-dimethoxy-pyrimidin-2-yl]-7-(1-methylimidazol-4-yl)-1H-indole-3-sulfonamide;
N-[5-(2,2-difluoroethoxy)-4,6-dimethoxy-pyrimidin-2-yl]-6-methyl-7-(2-pyrimidyl)-1H-indole-3-sulfonamide;
N-[5-(2,2-difluoroethoxy)-4,6-dimethoxy-pyrimidin-2-yl]-6-(difluoromethyl)-7-(2-pyrimidyl)-1H-indole-3-sulfonamide;
N-[5-(difluoromethoxy)-4,6-dimethoxy-pyrimidin-2-yl]-6-(difluoromethyl)-7-(2-pyrimidyl)-1H-indole-3-sulfonamide;
N-[5-(2,2-difluoroethyl)-4,6-dimethoxy-pyrimidin-2-yl]-6-(difluoromethyl)-7-(2-pyrimidyl)-1H-indole-3-sulfonamide;
N-[5-(2,2-difluoroethyl)-4,6-dimethoxy-pyrimidin-2-yl]-6-methyl-7-(2-pyrimidyl)-1H-indole-3-sulfonamide;
N-[5-(2,2-difluoroethyl)-4,6-dimethoxy-pyrimidin-2-yl]-7-(3-methylpyrazin-2-yl)-1H-indole-3-sulfonamide;
N-[5-(2,2-difluoroethoxy)-4,6-dimethoxy-pyrimidin-2-yl]-7-(3-methylpyrazin-2-yl)-1H-indole-3-sulfonamide;
N-[5-(difluoromethoxy)-4,6-dimethoxy-pyrimidin-2-yl]-6-methyl-7-(2-pyrimidyl)-1H-indole-3-sulfonamide;
N-[5-(2-fluoroethoxy)-4,6-dimethoxy-pyrimidin-2-yl]-6-methyl-7-(2-pyrimidyl)-1H-indole-3-sulfonamide;
N-[5-(2,2-difluoroethoxy)-4,6-dimethoxy-pyrimidin-2-yl]-6-methyl-7-pyridazin-3-yl-1H-indole-3-sulfonamide;
N-[5-(2,2-difluoroethoxy)-4,6-dimethoxy-pyrimidin-2-yl]-6-methyl-7-pyrazin-2-yl-1H-indole-3-sulfonamide;
N-[5-(difluoromethoxy)-4,6-dimethoxy-pyrimidin-2-yl]-6-methyl-7-pyrazin-2-yl-1H-indole-3-sulfonamide;
6-chloro-N-[5-(2,2-difluoroethoxy)-4,6-dimethoxy-pyrimidin-2-yl]-5-fluoro-7-(2-pyrimidyl)-1H-indole-3-sulfonamide;
6-chloro-N-[5-(2,2-difluoroethoxy)-4,6-dimethoxy-pyrimidin-2-yl]-7-(1-methylpyrazol-3-yl)-1H-indole-3-sulfonamide;
6-chloro-N-[5-(2,2-difluoroethoxy)-4,6-dimethoxy-pyrimidin-2-yl]-7-(2-methylpyrimidin-4-yl)-1H-indole-3-sulfonamide;
6-chloro-N-[5-(2,2-difluoroethoxy)-4,6-dimethoxy-pyrimidin-2-yl]-7-(6-methylpyridazin-3-yl)-1H-indole-3-sulfonamide;
6-chloro-7-(3-chloropyrazin-2-yl)-N-[5-(difluoromethoxy)-4,6-dimethoxy-pyrimidin-2-yl]-1H-indole-3-sulfonamide;
6-chloro-7-(3-chloropyrazin-2-yl)-N-[5-(2,2-difluoroethyl)-4,6-dimethoxy-pyrimidin-2-yl]-1H-indole-3-sulfonamide;
6-chloro-7-(3-chloropyrazin-2-yl)-N-[5-(2,2-difluoroethoxy)-4,6-dimethoxy-pyrimidin-2-yl]-1H-indole-3-sulfonamide;
6-chloro-N-[5-(difluoromethoxy)-4,6-dimethoxy-pyrimidin-2-yl]-7-(1-methylpyrazol-3-yl)-1H-indole-3-sulfonamide;
6-chloro-N-[5-(2-fluoroethoxy)-4,6-dimethoxy-pyrimidin-2-yl]-7-(1-methylpyrazol-3-yl)-1H-indole-3-sulfonamide;
6-bromo-N-[5-(2,2-difluoroethoxy)-4,6-dimethoxy-pyrimidin-2-yl]-7-(1-methylpyrazol-4-yl)-1H-indole-3-sulfonamide;
6-chloro-N-[5-(2,2-difluoroethoxy)-4,6-dimethoxy-pyrimidin-2-yl]-7-phenyl-1H-indole-3-sulfonamide;
6-chloro-N-[5-(2,2-difluoroethyl)-4,6-dimethoxy-pyrimidin-2-yl]-7-phenyl-1H-indole-3-sulfonamide;
N-[5-(2,2-difluoroethoxy)-4,6-dimethoxy-pyrimidin-2-yl]-7-(2-pyrimidyl)-1H-indole-3-sulfonamide;
6-chloro-N-[5-(2,2-difluoroethoxy)-4,6-dimethoxy-pyrimidin-2-yl]-7-(3-methylpyrazin-2-yl)-1H-indole-3-sulfonamide;
6-chloro-N-[5-(2,2-difluoroethyl)-4,6-dimethoxy-pyrimidin-2-yl]-7-(3-methylpyrazin-2-yl)-1H-indole-3-sulfonamide;
N-(5-bromo-4,6-dimethoxy-pyrimidin-2-yl)-6-chloro-7-(2-pyrimidyl)-1H-indole-3-sulfonamide;
6-chloro-N-[5-(2,2-difluoroethyl)-4,6-dimethoxy-pyrimidin-2-yl]-7-(4-pyrimidyl)-1H-indole-3-sulfonamide;
6-chloro-N-[5-(2,2-difluoroethoxy)-4,6-dimethoxy-pyrimidin-2-yl]-7-(4-pyrimidyl)-1H-indole-3-sulfonamide;
6-chloro-N-[5-(cyanomethoxy)-4,6-dimethoxy-pyrimidin-2-yl]-7-(2-pyrimidyl)-1H-indole-3-sulfonamide;
6-chloro-N-[5-(2-cyanoethyl)-4,6-dimethoxy-pyrimidin-2-yl]-7-(2-pyrimidyl)-1H-indole-3-sulfonamide;
6-chloro-N-[5-(2-fluoroethoxy)-4-methoxy-pyrimidin-2-yl]-7-(2-pyrimidyl)-1H-indole-3-sulfonamide;
6-chloro-N-[5-(difluoromethoxy)-4,6-dimethoxy-pyrimidin-2-yl]-7-(3-methylpyrazin-2-yl)-1H-indole-3-sulfonamide;
6-chloro-N-[5-(2,2-difluoroethyl)-4,6-dimethoxy-pyrimidin-2-yl]-7-(5-methylpyrazin-2-yl)-1H-indole-3-sulfonamide;
6-chloro-N-[5-(difluoromethoxy)-4,6-dimethoxy-pyrimidin-2-yl]-7-(5-methylpyrazin-2-yl)-1H-indole-3-sulfonamide;
6-chloro-N-[5-(2,2-difluoroethoxy)-4,6-dimethoxy-pyrimidin-2-yl]-7-(5-methylpyrazin-2-yl)-1H-indole-3-sulfonamide;
6-chloro-N-(4,6-dimethoxy-5-methyl-pyrimidin-2-yl)-7-(2-pyrimidyl)-1H-indole-3-sulfonamide;
6-chloro-N-[5-(2,2-difluoroethyl)-4,6-dimethoxy-pyrimidin-2-yl]-7-(6-methylpyrazin-2-yl)-1H-indole-3-sulfonamide;
6-chloro-N-[5-(difluoromethoxy)-4,6-dimethoxy-pyrimidin-2-yl]-7-(6-methylpyrazin-2-yl)-1H-indole-3-sulfonamide;
6-chloro-N-[5-(2,2-difluoroethoxy)-4,6-dimethoxy-pyrimidin-2-yl]-7-(6-methylpyrazin-2-yl)-1H-indole-3-sulfonamide;
6-chloro-N-[5-(2,2-difluoroethoxy)-4,6-dimethoxy-pyrimidin-2-yl]-7-(5-pyrimidyl)-1H-indole-3-sulfonamide;
6-chloro-7-(5-chloropyrazin-2-yl)-N-[5-(2,2-difluoroethoxy)-4,6-dimethoxy-pyrimidin-2-yl]-1H-indole-3-sulfonamide;
6-chloro-7-(5-chloropyrazin-2-yl)-N-[5-(2,2-difluoroethyl)-4,6-dimethoxy-pyrimidin-2-yl]-1H-indole-3-sulfonamide;
6-chloro-N-[5-(2,2-difluoroethoxy)-4,6-dimethoxy-pyrimidin-2-yl]-7-(6-methyl-2-pyridyl)-1H-indole-3-sulfonamide;
6-chloro-N-[5-(2,2-difluoroethyl)-4,6-dimethoxy-pyrimidin-2-yl]-7-(6-methyl-2-pyridyl)-1H-indole-3-sulfonamide;
6-chloro-N-[5-(difluoromethoxy)-4,6-dimethoxy-pyrimidin-2-yl]-7-(6-methyl-2-pyridyl)-1H-indole-3-sulfonamide;
6-chloro-N-[5-(2,2-difluoroethoxy)-4,6-dimethoxy-pyrimidin-2-yl]-7-pyridazin-4-yl-1H-indole-3-sulfonamide;
6-chloro-N-[5-(2,2-difluoroethoxy)-4,6-dimethoxy-pyrimidin-2-yl]-7-(6-methylpyrimidin-4-yl)-1H-indole-3-sulfonamide;
6-chloro-N-[5-(2,2-difluoroethyl)-4,6-dimethoxy-pyrimidin-2-yl]-7-(3-fluoropyrazin-2-yl)-1H-indole-3-sulfonamide;
6-chloro-N-[5-(2,2-difluoroethoxy)-4,6-dimethoxy-pyrimidin-2-yl]-7-(3-fluoropyrazin-2-yl)-1H-indole-3-sulfonamide;
6-chloro-N-[5-(difluoromethoxy)-4,6-dimethoxy-pyrimidin-2-yl]-7-(3-fluoropyrazin-2-yl)-1H-indole-3-sulfonamide;
6-chloro-N-[5-(2,2-difluoroethoxy)-4,6-dimethoxy-pyrimidin-2-yl]-7-(3-methyl-2-pyridyl)-1H-indole-3-sulfonamide;
6-chloro-N-[5-(2,2-difluoroethoxy)-4,6-dimethoxy-pyrimidin-2-yl]-7-(1-methyltriazol-4-yl)-1H-indole-3-sulfonamide;
6-chloro-N-[5-(2,2-difluoroethyl)-4,6-dimethoxy-pyrimidin-2-yl]-7-(3-methyl-2-pyridyl)-1H-indole-3-sulfonamide;
6-chloro-N-[5-(difluoromethoxy)-4,6-dimethoxy-pyrimidin-2-yl]-7-(3-methyl-2-pyridyl)-1H-indole-3-sulfonamide;
6-chloro-N-[5-(2,2-difluoroethoxy)-4,6-dimethoxy-pyrimidin-2-yl]-7-(1-methyl-1,2,4-triazol-3-yl)-1H-indole-3-sulfonamide;
6-chloro-N-[5-(2,2-difluoroethoxy)-4,6-dimethoxy-pyrimidin-2-yl]-7-(5-fluoropyrimidin-2-yl)-1H-indole-3-sulfonamide;
6-chloro-N-[5-(2,2-difluoroethoxy)-4,6-dimethoxy-pyrimidin-2-yl]-7-(2-methyltriazol-4-yl)-1H-indole-3-sulfonamide;
6-chloro-N-[5-(difluoromethoxy)-4,6-dimethoxy-pyrimidin-2-yl]-7-(2-methyltriazol-4-yl)-1H-indole-3-sulfonamide;
6-chloro-N-[5-(2,2-difluoroethyl)-4,6-dimethoxy-pyrimidin-2-yl]-7-(2-methyltriazol-4-yl)-1H-indole-3-sulfonamide;
6-chloro-N-[5-(2-fluoroethoxy)-4,6-dimethoxy-pyrimidin-2-yl]-7-(2-methyltriazol-4-yl)-1H-indole-3-sulfonamide;
6-chloro-N-[5-(2,2-difluoroethoxy)-4,6-dimethoxy-pyrimidin-2-yl]-7-(1,3,4-oxadiazol-2-yl)-1H-indole-3-sulfonamide;
6-chloro-N-[5-(2,2-difluoroethoxy)-4,6-dimethoxy-pyrimidin-2-yl]-7-(1-methylpyrazol-4-yl)-1H-indole-3-sulfonamide;
6-chloro-N-[5-(2-fluoroethoxy)-4,6-dimethoxy-pyrimidin-2-yl]-7-(1-methylpyrazol-4-yl)-1H-indole-3-sulfonamide;
6-chloro-N-[5-(difluoromethoxy)-4,6-dimethoxy-pyrimidin-2-yl]-7-(1-methylpyrazol-4-yl)-1H-indole-3-sulfonamide;
6-chloro-N-[5-(2,2-difluoroethyl)-4,6-dimethoxy-pyrimidin-2-yl]-7-(1-methylpyrazol-4-yl)-1H-indole-3-sulfonamide;
6-chloro-N-[5-(2,2-difluoroethoxy)-4,6-dimethoxy-pyrimidin-2-yl]-7-thiazol-4-yl-1H-indole-3-sulfonamide;
6-chloro-N-[5-(2,2-difluoroethoxy)-4,6-dimethoxy-pyrimidin-2-yl]-7-oxazol-5-yl-1H-indole-3-sulfonamide;
6-chloro-N-[5-(difluoromethoxy)-4,6-dimethoxy-pyrimidin-2-yl]-7-oxazol-5-yl-1H-indole-3-sulfonamide;
6-chloro-N-[5-(2,2-difluoroethoxy)-4,6-dimethoxy-pyrimidin-2-yl]-7-isoxazol-4-yl-1H-indole-3-sulfonamide;
6-chloro-N-[5-(difluoromethoxy)-4,6-dimethoxy-pyrimidin-2-yl]-7-isoxazol-4-yl-1H-indole-3-sulfonamide;
6-chloro-N-[5-(difluoromethoxy)-4,6-dimethoxy-pyrimidin-2-yl]-7-thiazol-4-yl-1H-indole-3-sulfonamide;
N-[5-(2,2-difluoroethyl)-4,6-dimethoxy-pyrimidin-2-yl]-7-(6,7-dihydro-5H-pyrrol[1,2-c]imidazol-3-yl)-1H-indole-3-sulfonamide; and
pharmaceutically acceptable salts thereof.

11. A process to prepare a compound according to any one of claims 1 to 10 comprising the reacting a compound of formula III with a compound of formula II in the presence of a base selected from N-ethyldiisopropylamine, pyridine, potassium phosphate or sodium hydride, to provide a compound of formula I, 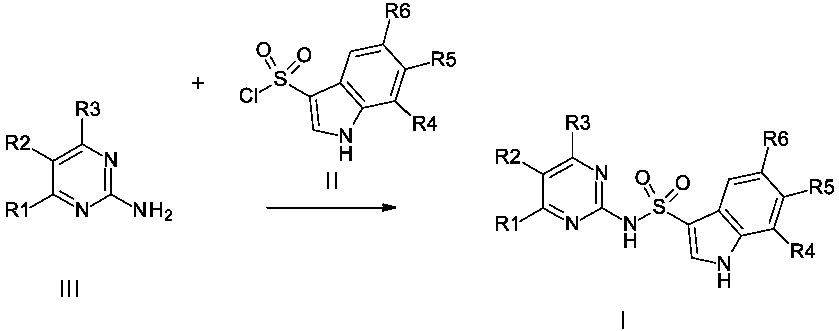 wherein R¹, R², R³, R³, R⁴, R⁵ and R⁶ are as described above.

12. A compound according to any one of claims 1 to 10 for use as therapeutically active substance.

13. A pharmaceutical composition comprising a compound according to any one of claims 1 to 10 and a therapeutically inert carrier.

14. A compound according to any one of claims 1 to 10 for use in the treatment or prophylaxis of conditions resulting from direct damage to myelin sheaths (including but not limited central pontine and extra-pontine myelinolysis, carbon monoxide poisoning, nutritional deficiency, and virus-induced demyelination), demyelinating disorders (including but not limited to multiple sclerosis, acute and multiphasic disseminated encephalomyelitis, neuromyelitis optica spectrum disorders, and leukodystrophies), CNS disorders associated with myelin loss (including but not limited to Alzheimer's disease, schizophrenia, Parkinson's disease, Huntington's disease, Amyotrophic lateral sclerosis, and Ischemia due to stroke), and inflammation in the CNS for instance following encephalitis, primary angiitis, meningitis and obesity.

15. A compound according to any one of claims 1 to 10 for use in the treatment or prophylaxis of multiple sclerosis.

## Patentansprüche

1. Verbindungen der Formel I 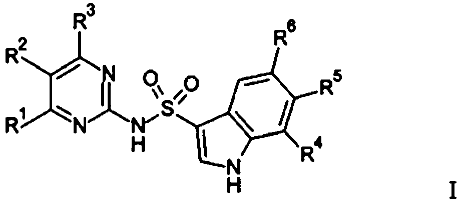 wobei
R¹ Alkoxy oder Halogenalkoxy ist;
R² Halogen, Alkyl, Alkoxy, Halogenalkyl, Halogenalkoxy, Cyanoalkyl, Cyanoalkoxy oder Cyclopropyl, gegebenenfalls substituiert mit bis zu zwei Substituenten, unabhängig voneinander ausgewählt aus Cyano und Halogen, ist;
R³ H, Alkoxy oder Halogenalkoxy ist;
R⁵ H, Halogen, Alkyl oder Halogenalkyl ist;
R⁶ H oder Halogen ist;
R⁴ ein C-verknüpftes Aryl oder ein C-verknüpftes Heteroaryl ist, wobei das C-verknüpfte Aryl oder C-verknüpfte Heteroaryl gegebenenfalls mit einem oder mehreren Substituenten, unabhängig voneinander ausgewählt aus Alkyl, Halogen und Halogenalkyl, substituiert ist;
und pharmazeutisch verträgliche Salze.

2. Verbindung nach Anspruch 1, wobei R¹ Alkoxy ist.

3. Verbindung nach Anspruch 1 oder Anspruch 2, wobei R² Halogen, Alkyl, Halogenalkyl, Halogenalkoxy, Cyanoalkyl, Cyanoalkoxy oder Cyclopropyl, substituiert mit Cyano, ist.

4. Verbindung nach einem der Ansprüche 1 bis 3, wobei R³ H oder Alkoxy ist.

5. Verbindung nach einem der Ansprüche 1 bis 4, wobei R⁴ ein C-verknüpftes Aryl oder ein C-verknüpftes Heteroaryl ist, wobei das C-verknüpfte Aryl oder C-verknüpfte Heteroaryl gegebenenfalls mit einem oder mehreren Substituenten, unabhängig voneinander ausgewählt aus Alkyl und Halogen, substituiert ist.

6. Verbindung nach einem der Ansprüche 1 bis 5, wobei R⁴ ausgewählt ist aus
i. einem C-verknüpften 6-gliedrigen Aryl;
ii. einem C-verknüpften 6-gliedrigen Heteroaryl, umfassend 1 bis 2 Stickstoffheteroatome, gegebenenfalls substituiert mit Alkyl oder Halogen;
iii. einem C-verknüpften 5-gliedrigen Heteroaryl, umfassend 1-3 Heteroatome, unabhängig voneinander ausgewählt aus N, S und O, gegebenenfalls substituiert mit 1 bis 2 Substituenten, unabhängig voneinander ausgewählt aus Alkyl und Halogen; und
iv. einem C-verknüpften 8-gliedrigen bicyclischen Heteroarylsystem, umfassend 2 N-Heteroatome.

7. Verbindung nach Anspruch 1, wobei
R¹ Alkoxy ist;
R² Halogen, Alkyl, Halogenalkyl, Halogenalkoxy, Cyanoalkyl, Cyanoalkoxy oder Cyclopropyl, substituiert mit Cyano, ist;
R³ H oder Alkoxy ist;
R⁵ H, Halogen, Alkyl oder Halogenalkyl ist;
R⁶ H oder Halogen ist;
R⁴ ein C-verknüpftes Aryl oder ein C-verknüpftes Heteroaryl ist, wobei das C-verknüpfte Aryl oder C-verknüpfte Heteroaryl gegebenenfalls mit einem oder mehreren Substituenten, unabhängig voneinander ausgewählt aus Alkyl und Halogen, substituiert ist;
und pharmazeutisch verträgliche Salze.

8. Verbindung nach Anspruch 1, wobei
R¹ Alkoxy ist;
R² Halogen, Alkyl, Halogenalkyl, Halogenalkoxy, Cyanoalkyl, Cyanoalkoxy oder Cyclopropyl, substituiert mit Cyano, ist;
R³ H oder Alkoxy ist;
R⁵ H, Halogen, Alkyl oder Halogenalkyl ist;
R⁶ H oder Halogen ist;
R⁴ ausgewählt ist aus
i. einem C-verknüpften 6-gliedrigen Aryl;
ii. einem C-verknüpften 6-gliedrigen Heteroaryl, umfassend 1 bis 2 Stickstoffheteroatome, gegebenenfalls substituiert mit Alkyl oder Halogen;
iii. einem C-verknüpften 5-gliedrigen Heteroaryl, umfassend 1-3 Heteroatome, unabhängig voneinander ausgewählt aus N, S und O, gegebenenfalls substituiert mit 1 bis 2 Substituenten, unabhängig voneinander ausgewählt aus Alkyl und Halogen; und
iv. einem C-verknüpften 8-gliedrigen bicyclischen Heteroarylsystem, umfassend 2 N-Heteroatome;
und pharmazeutisch verträgliche Salze.

9. Verbindung nach einem der Ansprüche 1 bis 8, ausgewählt aus
6-Chlor-N-[5-(2,2-difluorethoxy)-4,6-dimethoxypyrimidin-2-yl]-7-(2-pyrimidyl)-1H-indol-3-sulfonamid;
6-Chlor-N-[5-(2,2-difluorethoxy)-4,6-dimethoxypyrimidin-2-yl]-7-oxazol-2-yl-1H-indol-3-sulfonamid;
6-Chlor-N-[5-(2,2-difluorethyl)-4,6-dimethoxypyrimidin-2-yl]-7-(2-pyrimidyl)-1H-indol-3-sulfonamid;
6-Chlor-N-[5-(2,2-difluorethoxy)-4,6-dimethoxypyrimidin-2-yl]-7-pyridazin-3-yl-1H-indol-3-sulfonamid;
N-[5-(2,2-Difluorethyl)-4,6-dimethoxypyrimidin-2-yl]-7-(1-methylimidazol-2-yl)-1H-indol-3-sulfonamid;
6-Chlor-N-[5-(2,2-difluorethyl)-4,6-dimethoxypyrimidin-2-yl]-7-pyridazin-3-yl-1H-indol-3-sulfonamid;
N-[5-(2,2-Difluorethoxy)-4,6-dimethoxypyrimidin-2-yl]-7-(1-methylimidazol-2-yl)-1H-indol-3-sulfonamid;
6-Chlor-N-[5-(2,2-difluorethoxy)-4,6-dimethoxypyrimidin-2-yl]-7-(1-methylimidazol-2-yl)-1H-indol-3-sulfonamid;
N-[5-(2,2-Difluorethoxy)-4,6-dimethoxypyrimidin-2-yl]-6-fluor-7-(1-methylimidazol-2-yl)-1H-indol-3-sulfonamid;
N-[5-(2,2-Difluorethyl)-4,6-dimethoxypyrimidin-2-yl]-6-fluor-7-(1-methylimidazol-2-yl)-1H-indol-3-sulfonamid;
N-[5-(2,2-Difluorethyl)-4,6-dimethoxypyrimidin-2-yl]-7-pyridazin-3-yl-1H-indol-3-sulfonamid;
N-[5-(2,2-Difluorethoxy)-4,6-dimethoxypyrimidin-2-yl]-7-pyridazin-3-yl-1H-indol-3-sulfonamid;
N-[5-(2,2-Difluorethoxy)-4,6-dimethoxypyrimidin-2-yl]-7-(4-methylpyridazin-3-yl)-1H-indol-3-sulfonamid;
6-Chlor-N-[5-(2,2-difluorethoxy)-4,6-dimethoxypyrimidin-2-yl]-7-oxazol-4-yl-1H-indol-3-sulfonamid;
6-Chlor-N-[5-(2,2-difluorethyl)-4,6-dimethoxypyrimidin-2-yl]-7-pyrazin-2-yl-1H-indol-3-sulfonamid;
6-Chlor-N-[5-(2,2-difluorethoxy)-4,6-dimethoxypyrimidin-2-yl]-7-pyrazin-2-yl-1H-indol-3-sulfonamid;
N-[5-(2,2-Difluorethyl)-4,6-dimethoxypyrimidin-2-yl]-7-(2-pyrimidyl)-1H-indol-3-sulfonamid;
6-Chlor-N-[5-(2,2-difluorethyl)-4,6-dimethoxypyrimidin-2-yl]-7-(2-pyridyl)-1H-indol-3-sulfonamid;
6-Chlor-N-[5-(2,2-difluorethoxy)-4,6-dimethoxypyrimidin-2-yl]-7-(2-pyridyl)-1H-indol-3-sulfonamid;
6-Chlor-N-[5-(difluormethoxy)-4,6-dimethoxypyrimidin-2-yl]-7-pyrazin-2-yl-1H-indol-3-sulfonamid;
6-Chlor-N-[5-(2-fluorethoxy)-4,6-dimethoxypyrimidin-2-yl]-7-pyrazin-2-yl-1H-indol-3-sulfonamid;
6-Chlor-N-[5-(2-fluorethoxy)-4,6-dimethoxypyrimidin-2-yl]-7-(2-pyrimidyl)-1H-indol-3-sulfonamid;
6-Chlor-N-[5-(difluormethoxy)-4,6-dimethoxypyrimidin-2-yl]-7-(2-pyrimidyl)-1H-indol-3-sulfonamid;
6-Chlor-N-[5-(2-cyanocyclopropyl)-4,6-dimethoxypyrimidin-2-yl]-7-(2-pyrimidyl)-1H-indol-3-sulfonamid;
N-[5-(2,2-Difluorethyl)-4,6-dimethoxypyrimidin-2-yl]-7-pyrazin-2-yl-1H-indol-3-sulfonamid;
N-[5-(2,2-Difluorethoxy)-4,6-dimethoxypyrimidin-2-yl]-6-fluor-7-(2-pyrimidyl)-1H-indol-3-sulfonamid;
N-[5-(2,2-Difluorethyl)-4,6-dimethoxypyrimidin-2-yl]-6-fluor-7-(2-pyrimidyl)-1H-indol-3-sulfonamid;
6-Fluor-N-[5-(2-fluorethoxy)-4,6-dimethoxypyrimidin-2-yl]-7-(2-pyrimidyl)-1H-indol-3-sulfonamid;
N-[5-(Difluormethoxy)-4,6-dimethoxypyrimidin-2-yl]-6-fluor-7-(2-pyrimidyl)-1H-indol-3-sulfonamid;
N-[5-(2,2-Difluorethoxy)-4,6-dimethoxypyrimidin-2-yl]-7-pyrazin-2-yl-1H-indol-3-sulfonamid;
N-[5-(2,2-Difluorethyl)-4,6-dimethoxypyrimidin-2-yl]-6-fluor-7-pyrazin-2-yl-1H-indol-3-sulfonamid;
6-Fluor-N-[5-(2-fluorethoxy)-4,6-dimethoxypyrimidin-2-yl]-7-pyrazin-2-yl-1H-indol-3-sulfonamid;
N-[5-(2,2-Difluorethoxy)-4,6-dimethoxypyrimidin-2-yl]-6-fluor-7-pyrazin-2-yl-1H-indol-3-sulfonamid;
N-[5-(Difluormethoxy)-4,6-dimethoxypyrimidin-2-yl]-6-fluor-7-pyrazin-2-yl-1H-indol-3-sulfonamid;
6-Brom-N-[5-(2,2-difluorethyl)-4,6-dimethoxypyrimidin-2-yl]-7-(2-pyrimidyl)-1H-indol-3-sulfonamid;
6-Brom-N-[5-(difluormethoxy)-4,6-dimethoxypyrimidin-2-yl]-7-(2-pyrimidyl)-1H-indol-3-sulfonamid;
6-Brom-N-[5-(2-fluorethoxy)-4,6-dimethoxypyrimidin-2-yl]-7-(2-pyrimidyl)-1H-indol-3-sulfonamid;
6-Brom-N-[5-(2,2-difluorethoxy)-4,6-dimethoxypyrimidin-2-yl]-7-(2-pyrimidyl)-1H-indol-3-sulfonamid;
6-Brom-N-[5-(2,2-difluorethyl)-4,6-dimethoxypyrimidin-2-yl]-7-pyrazin-2-yl-1H-indol-3-sulfonamid;
6-Brom-N-[5-(difluormethoxy)-4,6-dimethoxypyrimidin-2-yl]-7-pyrazin-2-yl-1H-indol-3-sulfonamid;
6-Brom-N-[5-(2,2-difluorethoxy)-4,6-dimethoxypyrimidin-2-yl]-7-pyrazin-2-yl-1H-indol-3-sulfonamid;
6-Chlor-N-[5-(2,2-difluorethoxy)-4,6-dimethoxypyrimidin-2-yl]-7-(5-fluorpyrazin-2-yl)-1H-indol-3-sulfonamid;
6-Brom-N-[5-(2,2-difluorethyl)-4,6-dimethoxypyrimidin-2-yl]-7-pyridazin-3-yl-1H-indol-3-sulfonamid;
6-(Difluormethyl)-N-[5-(2-fluorethoxy)-4,6-dimethoxypyrimidin-2-yl]-7-(2-pyrimidyl)-1H-indol-3-sulfonamid;
N-[5-(2,2-Difluorethoxy)-4,6-dimethoxypyrimidin-2-yl]-6-fluor-7-(1H-imidazol-2-yl)-1H-indol-3-sulfonamid; und
pharmazeutisch verträgliche Salze davon.

10. Verbindung nach einem der Ansprüche 1 bis 8, ausgewählt aus
6-Chlor-N-[5-(2,2-difluorethoxy)-4,6-dimethoxypyrimidin-2-yl]-7-(1,5-dimethylimidazol-4-yl)-1H-indol-3-sulfonamid;
6-Chlor-N-[5-(2,2-difluorethoxy)-4,6-dimethoxypyrimidin-2-yl]-7-(1-methylimidazol-4-yl)-1H-indol-3-sulfonamid;
N-[5-(2,2-Difluorethoxy)-4,6-dimethoxypyrimidin-2-yl]-6-methyl-7-(2-pyrimidyl)-1H-indol-3-sulfonamid;
N-[5-(2,2-Difluorethoxy)-4,6-dimethoxypyrimidin-2-yl]-6-(difluormethyl)-7-(2-pyrimidyl)-1H-indol-3-sulfonamid;
N-[5-(Difluormethoxy)-4,6-dimethoxypyrimidin-2-yl]-6-(difluormethyl)-7-(2-pyrimidyl)-1H-indol-3-sulfonamid;
N-[5-(2,2-Difluorethyl)-4,6-dimethoxypyrimidin-2-yl]-6-(difluormethyl)-7-(2-pyrimidyl)-1H-indol-3-sulfonamid;
N-[5-(2,2-Difluorethyl)-4,6-dimethoxypyrimidin-2-yl]-6-methyl-7-(2-pyrimidyl)-1H-indol-3-sulfonamid;
N-[5-(2,2-Difluorethyl)-4,6-dimethoxypyrimidin-2-yl]-7-(3-methylpyrazin-2-yl)-1H-indol-3-sulfonamid;
N-[5-(2,2-Difluorethoxy)-4,6-dimethoxypyrimidin-2-yl]-7-(3-methylpyrazin-2-yl)-1H-indol-3-sulfonamid;
N-[5-(Difluormethoxy)-4,6-dimethoxypyrimidin-2-yl]-6-methyl-7-(2-pyrimidyl)-1H-indol-3-sulfonamid;
N-[5-(2-Fluorethoxy)-4,6-dimethoxypyrimidin-2-yl]-6-methyl-7-(2-pyrimidyl)-1H-indol-3-sulfonamid;
N-[5-(2,2-Difluorethoxy)-4,6-dimethoxypyrimidin-2-yl]-6-methyl-7-pyridazin-3-yl-1H-indol-3-sulfonamid;
N-[5-(2,2-Difluorethoxy)-4,6-dimethoxypyrimidin-2-yl]-6-methyl-7-pyrazin-2-yl-1H-indol-3-sulfonamid;
N-[5-(Difluormethoxy)-4,6-dimethoxypyrimidin-2-yl]-6-methyl-7-pyrazin-2-yl-1H-indol-3-sulfonamid;
6-Chlor-N-[5-(2,2-difluorethoxy)-4,6-dimethoxypyrimidin-2-yl]-5-fluor-7-(2-pyrimidyl)-1H-indol-3-sulfonamid;
6-Chlor-N-[5-(2,2-difluorethoxy)-4,6-dimethoxypyrimidin-2-yl]-7-(1-methylpyrazol-3-yl)-1H-indol-3-sulfonamid;
6-Chlor-N-[5-(2,2-difluorethoxy)-4,6-dimethoxypyrimidin-2-yl]-7-(2-methylpyrimidin-4-yl)-1H-indol-3-sulfonamid;
6-Chlor-N-[5-(2,2-difluorethoxy)-4,6-dimethoxypyrimidin-2-yl]-7-(6-methylpyridazin-3-yl)-1H-indol-3-sulfonamid;
6-Chlor-7-(3-chlorpyrazin-2-yl)-N-[5-(difluormethoxy)-4,6-dimethoxypyrimidin-2-yl]-1H-indol-3-sulfonamid;
6-Chlor-7-(3-chlorpyrazin-2-yl)-N-[5-(2,2-difluorethyl)-4,6-dimethoxypyrimidin-2-yl]-1H-indol-3-sulfonamid;
6-Chlor-7-(3-chlorpyrazin-2-yl)-N-[5-(2,2-difluorethoxy)-4,6-dimethoxypyrimidin-2-yl]-1H-indol-3-sulfonamid;
6-Chlor-N-[5-(difluormethoxy)-4,6-dimethoxypyrimidin-2-yl]-7-(1-methylpyrazol-3-yl)-1H-indol-3-sulfonamid;
6-Chlor-N-[5-(2-fluorethoxy)-4,6-dimethoxypyrimidin-2-yl]-7-(1-methylpyrazol-3-yl)-1H-indol-3-sulfonamid;
6-Brom-N-[5-(2,2-difluorethoxy)-4,6-dimethoxypyrimidin-2-yl]-7-(1-methylpyrazol-4-yl)-1H-indol-3-sulfonamid;
6-Chlor-N-[5-(2,2-difluorethoxy)-4,6-dimethoxypyrimidin-2-yl]-7-phenyl-1H-indol-3-sulfonamid;
6-Chlor-N-[5-(2,2-difluorethyl)-4,6-dimethoxypyrimidin-2-yl]-7-phenyl-1H-indol-3-sulfonamid;
N-[5-(2,2-Difluorethoxy)-4,6-dimethoxypyrimidin-2-yl]-7-(2-pyrimidyl)-1H-indol-3-sulfonamid;
6-Chlor-N-[5-(2,2-difluorethoxy)-4,6-dimethoxypyrimidin-2-yl]-7-(3-methylpyrazin-2-yl)-1H-indol-3-sulfonamid;
6-Chlor-N-[5-(2,2-difluorethyl)-4,6-dimethoxypyrimidin-2-yl]-7-(3-methylpyrazin-2-yl)-1H-indol-3-sulfonamid;
N-(5-Brom-4,6-dimethoxypyrimidin-2-yl)-6-chlor-7-(2-pyrimidyl)-1H-indol-3-sulfonamid;
6-Chlor-N-[5-(2,2-difluorethyl)-4,6-dimethoxypyrimidin-2-yl]-7-(4-pyrimidyl)-1H-indol-3-sulfonamid;
6-Chlor-N-[5-(2,2-difluorethoxy)-4,6-dimethoxypyrimidin-2-yl]-7-(4-pyrimidyl)-1H-indol-3-sulfonamid;
6-Chlor-N-[5-(cyanomethoxy)-4,6-dimethoxypyrimidin-2-yl]-7-(2-pyrimidyl)-1H-indol-3-sulfonamid;
6-Chlor-N-[5-(2-cyanoethyl)-4,6-dimethoxypyrimidin-2-yl]-7-(2-pyrimidyl)-1H-indol-3-sulfonamid;
6-Chlor-N-[5-(2-fluorethoxy)-4-methoxypyrimidin-2-yl]-7-(2-pyrimidyl)-1H-indol-3-sulfonamid;
6-Chlor-N-[5-(difluormethoxy)-4,6-dimethoxypyrimidin-2-yl]-7-(3-methylpyrazin-2-yl)-1H-indol-3-sulfonamid;
6-Chlor-N-[5-(2,2-difluorethyl)-4,6-dimethoxypyrimidin-2-yl]-7-(5-methylpyrazin-2-yl)-1H-indol-3-sulfonamid;
6-Chlor-N-[5-(difluormethoxy)-4,6-dimethoxypyrimidin-2-yl]-7-(5-methylpyrazin-2-yl)-1H-indol-3-sulfonamid;
6-Chlor-N-[5-(2,2-difluorethoxy)-4,6-dimethoxypyrimidin-2-yl]-7-(5-methylpyrazin-2-yl)-1H-indol-3-sulfonamid;
6-Chlor-N-(4,6-dimethoxy-5-methylpyrimidin-2-yl)-7-(2-pyrimidyl)-1H-indol-3-sulfonamid;
6-Chlor-N-[5-(2,2-difluorethyl)-4,6-dimethoxypyrimidin-2-yl]-7-(6-methylpyrazin-2-yl)-1H-indol-3-sulfonamid;
6-Chlor-N-[5-(difluormethoxy)-4,6-dimethoxypyrimidin-2-yl]-7-(6-methylpyrazin-2-yl)-1H-indol-3-sulfonamid;
6-Chlor-N-[5-(2,2-difluorethoxy)-4,6-dimethoxypyrimidin-2-yl]-7-(6-methylpyrazin-2-yl)-1H-indol-3-sulfonamid;
6-Chlor-N-[5-(2,2-difluorethoxy)-4,6-dimethoxypyrimidin-2-yl]-7-(5-pyrimidyl)-1H-indol-3-sulfonamid;
6-Chlor-7-(5-chlorpyrazin-2-yl)-N-[5-(2,2-difluorethoxy)-4,6-dimethoxypyrimidin-2-yl]-1H-indol-3-sulfonamid;
6-Chlor-7-(5-chlorpyrazin-2-yl)-N-[5-(2,2-difluorethyl)-4,6-dimethoxypyrimidin-2-yl]-1H-indol-3-sulfonamid;
6-Chlor-N-[5-(2,2-difluorethoxy)-4,6-dimethoxypyrimidin-2-yl]-7-(6-methyl-2-pyridyl)-1H-indol-3-sulfonamid;
6-Chlor-N-[5-(2,2-difluorethyl)-4,6-dimethoxypyrimidin-2-yl]-7-(6-methyl-2-pyridyl)-1H-indol-3-sulfonamid;
6-Chlor-N-[5-(difluormethoxy)-4,6-dimethoxypyrimidin-2-yl]-7-(6-methyl-2-pyridyl)-1H-indol-3-sulfonamid;
6-Chlor-N-[5-(2,2-difluorethoxy)-4,6-dimethoxypyrimidin-2-yl]-7-pyridazin-4-yl-1H-indol-3-sulfonamid;
6-Chlor-N-[5-(2,2-difluorethoxy)-4,6-dimethoxypyrimidin-2-yl]-7-(6-methylpyrimidin-4-yl)-1H-indol-3-sulfonamid;
6-Chlor-N-[5-(2,2-difluorethyl)-4,6-dimethoxypyrimidin-2-yl]-7-(3-fluorpyrazin-2-yl)-1H-indol-3-sulfonamid;
6-Chlor-N-[5-(2,2-difluorethoxy)-4,6-dimethoxypyrimidin-2-yl]-7-(3-fluorpyrazin-2-yl)-1H-indol-3-sulfonamid;
6-Chlor-N-[5-(difluormethoxy)-4,6-dimethoxypyrimidin-2-yl]-7-(3-fluorpyrazin-2-yl)-1H-indol-3-sulfonamid;
6-Chlor-N-[5-(2,2-difluorethoxy)-4,6-dimethoxypyrimidin-2-yl]-7-(3-methyl-2-pyridyl)-1H-indol-3-sulfonamid;
6-Chlor-N-[5-(2,2-difluorethoxy)-4,6-dimethoxypyrimidin-2-yl]-7-(1-methyltriazol-4-yl)-1H-indol-3-sulfonamid;
6-Chlor-N-[5-(2,2-difluorethyl)-4,6-dimethoxypyrimidin-2-yl]-7-(3-methyl-2-pyridyl)-1H-indol-3-sulfonamid;
6-Chlor-N-[5-(difluormethoxy)-4,6-dimethoxypyrimidin-2-yl]-7-(3-methyl-2-pyridyl)-1H-indol-3-sulfonamid;
6-Chlor-N-[5-(2,2-difluorethoxy)-4,6-dimethoxypyrimidin-2-yl]-7-(1-methyl-1,2,4-triazol-3-yl)-1H-indol-3-sulfonamid;
6-Chlor-N-[5-(2,2-difluorethoxy)-4,6-dimethoxypyrimidin-2-yl]-7-(5-fluorpyrimidin-2-yl)-1H-indol-3-sulfonamid;
6-Chlor-N-[5-(2,2-difluorethoxy)-4,6-dimethoxypyrimidin-2-yl]-7-(2-methyltriazol-4-yl)-1H-indol-3-sulfonamid;
6-Chlor-N-[5-(difluormethoxy)-4,6-dimethoxypyrimidin-2-yl]-7-(2-methyltriazol-4-yl)-1H-indol-3-sulfonamid;
6-Chlor-N-[5-(2,2-difluorethyl)-4,6-dimethoxypyrimidin-2-yl]-7-(2-methyltriazol-4-yl)-1H-indol-3-sulfonamid;
6-Chlor-N-[5-(2-fluorethoxy)-4,6-dimethoxypyrimidin-2-yl]-7-(2-methyltriazol-4-yl)-1H-indol-3-sulfonamid;
6-Chlor-N-[5-(2,2-difluorethoxy)-4,6-dimethoxypyrimidin-2-yl]-7-(1,3,4-oxadiazol-2-yl)-1H-indol-3-sulfonamid;
6-Chlor-N-[5-(2,2-difluorethoxy)-4,6-dimethoxypyrimidin-2-yl]-7-(1-methylpyrazol-4-yl)-1H-indol-3-sulfonamid;
6-Chlor-N-[5-(2-fluorethoxy)-4,6-dimethoxypyrimidin-2-yl]-7-(1-methylpyrazol-4-yl)-1H-indol-3-sulfonamid;
6-Chlor-N-[5-(difluormethoxy)-4,6-dimethoxypyrimidin-2-yl]-7-(1-methylpyrazol-4-yl)-1H-indol-3-sulfonamid;
6-Chlor-N-[5-(2,2-difluorethyl)-4,6-dimethoxypyrimidin-2-yl]-7-(1-methylpyrazol-4-yl)-1H-indol-3-sulfonamid;
6-Chlor-N-[5-(2,2-difluorethoxy)-4,6-dimethoxypyrimidin-2-yl]-7-thiazol-4-yl-1H-indol-3-sulfonamid;
6-Chlor-N-[5-(2,2-difluorethoxy)-4,6-dimethoxypyrimidin-2-yl]-7-oxazol-5-yl-1H-indol-3-sulfonamid;
6-Chlor-N-[5-(difluormethoxy)-4,6-dimethoxypyrimidin-2-yl]-7-oxazol-5-yl-1H-indol-3-sulfonamid;
6-Chlor-N-[5-(2,2-difluorethoxy)-4,6-dimethoxypyrimidin-2-yl]-7-isoxazol-4-yl-1H-indol-3-sulfonamid;
6-Chlor-N-[5-(difluormethoxy)-4,6-dimethoxypyrimidin-2-yl]-7-isoxazol-4-yl-1H-indol-3-sulfonamid;
6-Chlor-N-[5-(difluormethoxy)-4,6-dimethoxypyrimidin-2-yl]-7-thiazol-4-yl-1H-indol-3-sulfonamid;
N-[5-(2,2-Difluorethyl)-4,6-dimethoxypyrimidin-2-yl]-7-(6,7-dihydro-5H-pyrrol[1,2-c]imidazol-3-yl)-1H-indol-3-sulfonamid; und
pharmazeutisch verträgliche Salze davon.

11. Verfahren zur Herstellung einer Verbindung nach einem der Ansprüche 1 bis 10, umfassend das Umsetzen einer Verbindung der Formel III mit einer Verbindung der Formel II in Gegenwart einer Base, ausgewählt aus N-Ethyldiisopropylamin, Pyridin, Kaliumphosphat oder Natriumhydrid, um eine Verbindung der Formel I bereitzustellen, 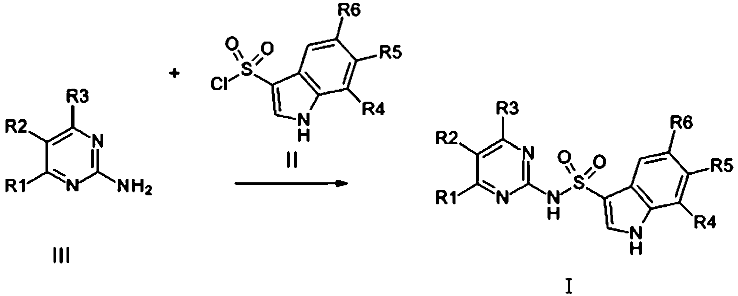 wobei R¹, R², R³, R³, R⁴, R⁵ und R⁶ wie oben beschrieben sind.

12. Verbindung nach einem der Ansprüche 1 bis 10 zur Verwendung als therapeutisch wirksame Substanz.

13. Pharmazeutische Zusammensetzung, umfassend eine Verbindung nach einem der Ansprüche 1 bis 10 und einen therapeutisch inerten Träger.

14. Verbindung nach einem der Ansprüche 1 bis 10 zur Verwendung bei der Behandlung oder Prophylaxe von Erkrankungen, die aus direkter Schädigung von Myelinscheiden resultieren (einschließlich, aber nicht beschränkt auf zentrale pontine und extrapontine Myelinolyse, Kohlenmonoxidvergiftung, Mangelernährung und virusinduzierte Demyelinisierung), demyelinisierenden Störungen (einschließlich, aber nicht beschränkt auf Multiple Sklerose, akute und mehrphasige disseminierte Enzephalomyelitis, Neuromyelitis-optica-Spektrum-Störungen und Leukodystrophien), Störungen des ZNS, die mit Myelinverlust assoziiert sind (einschließlich, aber nicht beschränkt auf Alzheimer-Krankheit, Schizophrenie, Parkinson-Krankheit, Huntington-Krankheit, amyotrophe Lateralsklerose und Ischämie aufgrund von Schlaganfall), und Entzündungen im ZNS, zum Beispiel nach Enzephalitis, primärer Angiitis, Meningitis und Fettleibigkeit.

15. Verbindung nach einem der Ansprüche 1 bis 10 zur Verwendung bei der Behandlung oder Prophylaxe von Multipler Sklerose.

## Revendications

1. Composés de formule I 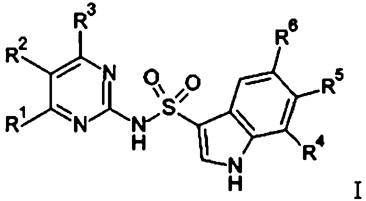 dans lesquels
R¹ représente un alcoxy ou un halogénoalcoxy ;
R² représente un halogène, un alkyle, un alcoxy, un halogénoalkyle, un halogénoalcoxy, un cyanoalkyle, un cyanoalcoxy ou un cyclopropyle éventuellement substitué par jusqu'à deux substituants indépendamment choisis parmi un cyano et un halogène ;
R³ représente H, un alcoxy ou un halogénoalcoxy ;
R⁵ représente H, un halogène, un alkyle ou un halogénoalkyle ;
R⁶ représente H ou un halogène ;
R⁴ représente un aryle lié par C ou un hétéroaryle lié par C, l'aryle lié par C ou l'hétéroaryle lié par C étant éventuellement substitué par un ou plusieurs substituants indépendamment choisis parmi un alkyle, un halogène et un halogénoalkyle ;
et des sels pharmaceutiquement acceptables.

2. Composé selon la revendication 1, dans lequel R¹ représente un alcoxy.

3. Composé selon la revendication 1 ou la revendication 2, dans lequel R² représente un halogène, un alkyle, un halogénoalkyle, un halogénoalcoxy, un cyanoalkyle, un cyanoalcoxy ou un cyclopropyle substitué par un cyano.

4. Composé selon l'une quelconque des revendications 1 à 3, dans lequel R³ représente H ou un alcoxy.

5. Composé selon l'une quelconque des revendications 1 à 4, dans lequel R⁴ représente un aryle lié par C ou un hétéroaryle lié par C, l'aryle lié par C ou l'hétéroaryle lié par C étant éventuellement substitué par un ou plusieurs substituants indépendamment choisis parmi un alkyle et un halogène.

6. Composé selon l'une quelconque des revendications 1 à 5, dans lequel R⁴ est choisi parmi
i. un aryle à 6 chaînons lié par C ;
ii. un hétéroaryle à 6 chaînons lié par C comprenant 1 à 2 hétéroatomes d'azote éventuellement substitué par un alkyle ou un halogène ;
iii. un hétéroaryle à 5 chaînons lié par C comprenant 1 à 3 hétéroatomes indépendamment choisis parmi N, S et O, éventuellement substitué par 1 à 2 substituants indépendamment choisis parmi un alkyle et un halogène ; et
iv. un système hétéroaryle bicyclique à 8 chaînons lié par C comprenant 2 hétéroatomes N.

7. Composé selon la revendication 1, dans lequel
R¹ représente un alcoxy ;
R² représente un halogène, un alkyle, un halogénoalkyle, un halogénoalcoxy, un cyanoalkyle, un cyanoalcoxy ou un cyclopropyle substitué par un cyano ;
R³ représente H ou un alcoxy ;
R⁵ représente H, un halogène, un alkyle ou un halogénoalkyle ;
R⁶ représente H ou un halogène ;
R⁴ représente un aryle lié par C ou un hétéroaryle lié par C, l'aryle lié par C ou l'hétéroaryle lié par C étant éventuellement substitué par un ou plusieurs substituants indépendamment choisis parmi un alkyle et un halogène ;
et des sels pharmaceutiquement acceptables.

8. Composé selon la revendication 1, dans lequel
R¹ représente un alcoxy ;
R² représente un halogène, un alkyle, un halogénoalkyle, un halogénoalcoxy, un cyanoalkyle, un cyanoalcoxy ou un cyclopropyle substitué par un cyano ;
R³ représente H ou un alcoxy ;
R⁵ représente H, un halogène, un alkyle ou un halogénoalkyle ;
R⁶ représente H ou un halogène ;
R⁴ est choisi parmi
i. un aryle à 6 chaînons lié par C ;
ii. un hétéroaryle à 6 chaînons lié par C comprenant 1 à 2 hétéroatomes d'azote éventuellement substitué par un alkyle ou un halogène ;
iii. un hétéroaryle à 5 chaînons lié par C comprenant 1 à 3 hétéroatomes indépendamment choisis parmi N, S et O, éventuellement substitué par 1 à 2 substituants indépendamment choisis parmi un alkyle et un halogène ; et
iv. un système hétéroaryle bicyclique à 8 chaînons lié par C comprenant 2 hétéroatomes N ;
et des sels pharmaceutiquement acceptables.

9. Composé selon l'une quelconque des revendications 1 à 8, choisi parmi
le 6-chloro-N-[5-(2,2-difluoroéthoxy)-4,6-diméthoxy-pyrimidin-2-yl]-7-(2-pyrimidyl)-1H-indole-3-sulfonamide ;
le 6-chloro-N-[5-(2,2-difluoroéthoxy)-4,6-diméthoxy-pyrimidin-2-yl]-7-oxazol-2-yl-1H-indole-3-sulfonamide ;
le 6-chloro-N-[5-(2,2-difluoroéthyl)-4,6-diméthoxy-pyrimidin-2-yl]-7-(2-pyrimidyl)-1H-indole-3-sulfonamide ;
le 6-chloro-N-[5-(2,2-difluoroéthoxy)-4,6-diméthoxy-pyrimidin-2-yl]-7-pyridazin-3-yl-1H-indole-3-sulfonamide ;
le N-[5-(2,2-difluoroéthyl)-4,6-diméthoxy-pyrimidin-2-yl]-7-(1-méthylimidazol-2-yl)-1H-indole-3-sulfonamide ;
le 6-chloro-N-[5-(2,2-difluoroéthyl)-4,6-diméthoxy-pyrimidin-2-yl]-7-pyridazin-3-yl-1H-indole-3-sulfonamide ;
le N-[5-(2,2-difluoroéthoxy)-4,6-diméthoxy-pyrimidin-2-yl]-7-(1-méthylimidazol-2-yl)-1H-indole-3-sulfonamide ;
le 6-chloro-N-[5-(2,2-difluoroéthoxy)-4,6-diméthoxy-pyrimidin-2-yl]-7-(1-méthylimidazol-2-yl)-1H-indole-3-sulfonamide ;
le N-[5-(2,2-difluoroéthoxy)-4,6-diméthoxy-pyrimidin-2-yl]-6-fluoro-7-(1-méthylimidazol-2-yl)-1H-indole-3-sulfonamide ;
le N-[5-(2,2-difluoroéthyl)-4,6-diméthoxy-pyrimidin-2-yl]-6-fluoro-7-(1-méthylimidazol-2-yl)-1H-indole-3-sulfonamide ;
le N-[5-(2,2-difluoroéthyl)-4,6-diméthoxy-pyrimidin-2-yl]-7-pyridazin-3-yl-1H-indole-3-sulfonamide ;
le N-[5-(2,2-difluoroéthoxy)-4,6-diméthoxy-pyrimidin-2-yl]-7-pyridazin-3-yl-1H-indole-3-sulfonamide ;
le N-[5-(2,2-difluoroéthoxy)-4,6-diméthoxy-pyrimidin-2-yl]-7-(4-méthylpyridazin-3-yl)-1H-indole-3-sulfonamide ;
le 6-chloro-N-[5-(2,2-difluoroéthoxy)-4,6-diméthoxy-pyrimidin-2-yl]-7-oxazol-4-yl-1H-indole-3-sulfonamide ;
le 6-chloro-N-[5-(2,2-difluoroéthyl)-4,6-diméthoxy-pyrimidin-2-yl]-7-pyrazin-2-yl-1H-indole-3-sulfonamide ;
le 6-chloro-N-[5-(2,2-difluoroéthoxy)-4,6-diméthoxy-pyrimidin-2-yl]-7-pyrazin-2-yl-1H-indole-3-sulfonamide ;
le N-[5-(2,2-difluoroéthyl)-4,6-diméthoxy-pyrimidin-2-yl]-7-(2-pyrimidyl)-1H-indole-3-sulfonamide ;
le 6-chloro-N-[5-(2,2-difluoroéthyl)-4,6-diméthoxy-pyrimidin-2-yl]-7-(2-pyridyl)-1H-indole-3-sulfonamide ;
le 6-chloro-N-[5-(2,2-difluoroéthoxy)-4,6-diméthoxy-pyrimidin-2-yl]-7-(2-pyridyl)-1H-indole-3-sulfonamide ;
le 6-chloro-N-[5-(difluorométhoxy)-4,6-diméthoxy-pyrimidin-2-yl]-7-pyrazin-2-yl-1H-indole-3-sulfonamide ;
le 6-chloro-N-[5-(2-fluoroéthoxy)-4,6-diméthoxy-pyrimidin-2-yl]-7-pyrazin-2-yl-1H-indole-3-sulfonamide ;
le 6-chloro-N-[5-(2-fluoroéthoxy)-4,6-diméthoxy-pyrimidin-2-yl]-7-(2-pyrimidyl)-1H-indole-3-sulfonamide ;
le 6-chloro-N-[5-(difluorométhoxy)-4,6-diméthoxy-pyrimidin-2-yl]-7-(2-pyrimidyl)-1H-indole-3-sulfonamide ;
le 6-chloro-N-[5-(2-cyanocyclopropyl)-4,6-diméthoxy-pyrimidin-2-yl]-7-(2-pyrimidyl)-1H-indole-3-sulfonamide ;
le N-[5-(2,2-difluoroéthyl)-4,6-diméthoxy-pyrimidin-2-yl]-7-pyrazin-2-yl-1H-indole-3-sulfonamide ;
le N-[5-(2,2-difluoroéthoxy)-4,6-diméthoxy-pyrimidin-2-yl]-6-fluoro-7-(2-pyrimidyl)-1H-indole-3-sulfonamide ;
le N-[5-(2,2-difluoroéthyl)-4,6-diméthoxy-pyrimidin-2-yl]-6-fluoro-7-(2-pyrimidyl)-1H-indole-3-sulfonamide ;
le 6-fluoro-N-[5-(2-fluoroéthoxy)-4,6-diméthoxy-pyrimidin-2-yl]-7-(2-pyrimidyl)-1H-indole-3-sulfonamide ;
le N-[5-(difluorométhoxy)-4,6-diméthoxy-pyrimidin-2-yl]-6-fluoro-7-(2-pyrimidyl)-1H-indole-3-sulfonamide ;
le N-[5-(2,2-difluoroéthoxy)-4,6-diméthoxy-pyrimidin-2-yl]-7-pyrazin-2-yl-1H-indole-3-sulfonamide ;
le N-[5-(2,2-difluoroéthyl)-4,6-diméthoxy-pyrimidin-2-yl]-6-fluoro-7-pyrazin-2-yl-1H-indole-3-sulfonamide ;
le 6-fluoro-N-[5-(2-fluoroéthoxy)-4,6-diméthoxy-pyrimidin-2-yl]-7-pyrazin-2-yl-1H-indole-3-sulfonamide ;
le N-[5-(2,2-difluoroéthoxy)-4,6-diméthoxy-pyrimidin-2-yl]-6-fluoro-7-pyrazin-2-yl-1H-indole-3-sulfonamide ;
le N-[5-(difluorométhoxy)-4,6-diméthoxy-pyrimidin-2-yl]-6-fluoro-7-pyrazin-2-yl-1H-indole-3-sulfonamide ;
le 6-bromo-N-[5-(2,2-difluoroéthyl)-4,6-diméthoxy-pyrimidin-2-yl]-7-(2-pyrimidyl)-1H-indole-3-sulfonamide ;
le 6-bromo-N-[5-(difluorométhoxy)-4,6-diméthoxy-pyrimidin-2-yl]-7-(2-pyrimidyl)-1H-indole-3-sulfonamide ;
le 6-bromo-N-[5-(2-fluoroéthoxy)-4,6-diméthoxy-pyrimidin-2-yl]-7-(2-pyrimidyl)-1H-indole-3-sulfonamide ;
le 6-bromo-N-[5-(2,2-difluoroéthoxy)-4,6-diméthoxy-pyrimidin-2-yl]-7-(2-pyrimidyl)-1H-indole-3-sulfonamide ;
le 6-bromo-N-[5-(2,2-difluoroéthyl)-4,6-diméthoxy-pyrimidin-2-yl]-7-pyrazin-2-yl-1H-indole-3-sulfonamide ;
le 6-bromo-N-[5-(difluorométhoxy)-4,6-diméthoxy-pyrimidin-2-yl]-7-pyrazin-2-yl-1H-indole-3-sulfonamide ;
le 6-bromo-N-[5-(2,2-difluoroéthoxy)-4,6-diméthoxy-pyrimidin-2-yl]-7-pyrazin-2-yl-1H-indole-3-sulfonamide ;
le 6-chloro-N-[5-(2,2-difluoroéthoxy)-4,6-diméthoxy-pyrimidin-2-yl]-7-(5-fluoropyrazin-2-yl)-1H-indole-3-sulfonamide ;
le 6-bromo-N-[5-(2,2-difluoroéthyl)-4,6-diméthoxy-pyrimidin-2-yl]-7-pyridazin-3-yl-1H-indole-3-sulfonamide ;
le 6-(difluorométhyl)-N-[5-(2-fluoroéthoxy)-4,6-diméthoxy-pyrimidin-2-yl]-7-(2-pyrimidyl)-1H-indole-3-sulfonamide ;
le N-[5-(2,2-difluoroéthoxy)-4,6-diméthoxy-pyrimidin-2-yl]-6-fluoro-7-(1H-imidazol-2-yl)-1H-indole-3-sulfonamide ; et
des sels pharmaceutiquement acceptables de ceux-ci.

10. Composé selon l'une quelconque des revendications 1 à 8 choisi parmi
le 6-chloro-N-[5-(2,2-difluoroéthoxy)-4,6-diméthoxy-pyrimidin-2-yl]-7-(1,5-diméthylimidazol-4-yl)-1H-indole-3-sulfonamide ;
le 6-chloro-N-[5-(2,2-difluoroéthoxy)-4,6-diméthoxy-pyrimidin-2-yl]-7-(1-méthylimidazol-4-yl)-1H-indole-3-sulfonamide ;
le N-[5-(2,2-difluoroéthoxy)-4,6-diméthoxy-pyrimidin-2-yl]-6-méthyl-7-(2-pyrimidyl)-1H-indole-3-sulfonamide ;
le N-[5-(2,2-difluoroéthoxy)-4,6-diméthoxy-pyrimidin-2-yl]-6-(difluorométhyl)-7-(2-pyrimidyl)-1H-indole-3-sulfonamide ;
le N-[5-(difluorométhoxy)-4,6-diméthoxy-pyrimidin-2-yl]-6-(difluorométhyl)-7-(2-pyrimidyl)-1H-indole-3-sulfonamide ;
le N-[5-(2,2-difluoroéthyl)-4,6-diméthoxy-pyrimidin-2-yl]-6-(difluorométhyl)-7-(2-pyrimidyl)-1H-indole-3-sulfonamide ;
le N-[5-(2,2-difluoroéthyl)-4,6-diméthoxy-pyrimidin-2-yl]-6-méthyl-7-(2-pyrimidyl)-1H-indole-3-sulfonamide ;
le N-[5-(2,2-difluoroéthyl)-4,6-diméthoxy-pyrimidin-2-yl]-7-(3-méthylpyrazin-2-yl)-1H-indole-3-sulfonamide ;
le N-[5-(2,2-difluoroéthoxy)-4,6-diméthoxy-pyrimidin-2-yl]-7-(3-méthylpyrazin-2-yl)-1H-indole-3-sulfonamide ;
le N-[5-(difluorométhoxy)-4,6-diméthoxy-pyrimidin-2-yl]-6-méthyl-7-(2-pyrimidyl)-1H-indole-3-sulfonamide ;
le N-[5-(2-fluoroéthoxy)-4,6-diméthoxy-pyrimidin-2-yl]-6-méthyl-7-(2-pyrimidyl)-1H-indole-3-sulfonamide ;
le N-[5-(2,2-difluoroéthoxy)-4,6-diméthoxy-pyrimidin-2-yl]-6-méthyl-7-pyridazin-3-yl-1H-indole-3-sulfonamide ;
le N-[5-(2,2-difluoroéthoxy)-4,6-diméthoxy-pyrimidin-2-yl]-6-méthyl-7-pyrazin-2-yl-1H-indole-3-sulfonamide ;
le N-[5-(difluorométhoxy)-4,6-diméthoxy-pyrimidin-2-yl]-6-méthyl-7-pyrazin-2-yl-1H-indole-3-sulfonamide ;
le 6-chloro-N-[5-(2,2-difluoroéthoxy)-4,6-diméthoxy-pyrimidin-2-yl]-5-fluoro-7-(2-pyrimidyl)-1H-indole-3-sulfonamide ;
le 6-chloro-N-[5-(2,2-difluoroéthoxy)-4,6-diméthoxy-pyrimidin-2-yl]-7-(1-méthylpyrazol-3-yl)-1H-indole-3-sulfonamide ;
le 6-chloro-N-[5-(2,2-difluoroéthoxy)-4,6-diméthoxy-pyrimidin-2-yl]-7-(2-méthylpyrimidin-4-yl)-1H-indole-3-sulfonamide ;
le 6-chloro-N-[5-(2,2-difluoroéthoxy)-4,6-diméthoxy-pyrimidin-2-yl]-7-(6-méthylpyridazin-3-yl)-1H-indole-3-sulfonamide ;
le 6-chloro-7-(3-chloropyrazin-2-yl)-N-[5-(difluorométhoxy)-4,6-diméthoxy-pyrimidin-2-yl]-1H-indole-3-sulfonamide ;
le 6-chloro-7-(3-chloropyrazin-2-yl)-N-[5-(2,2-difluoroéthyl)-4,6-diméthoxy-pyrimidin-2-yl]-1H-indole-3-sulfonamide ;
le 6-chloro-7-(3-chloropyrazin-2-yl)-N-[5-(2,2-difluoroéthoxy)-4,6-diméthoxy-pyrimidin-2-yl]-1H-indole-3-sulfonamide ;
le 6-chloro-N-[5-(difluorométhoxy)-4,6-diméthoxy-pyrimidin-2-yl]-7-(1-méthylpyrazol-3-yl)-1H-indole-3-sulfonamide ;
le 6-chloro-N-[5-(2-fluoroéthoxy)-4,6-diméthoxy-pyrimidin-2-yl]-7-(1-méthylpyrazol-3-yl)-1H-indole-3-sulfonamide ;
le 6-bromo-N-[5-(2,2-difluoroéthoxy)-4,6-diméthoxy-pyrimidin-2-yl]-7-(1-méthylpyrazol-4-yl)-1H-indole-3-sulfonamide ;
le 6-chloro-N-[5-(2,2-difluoroéthoxy)-4,6-diméthoxy-pyrimidin-2-yl]-7-phényl-1H-indole-3-sulfonamide ;
le 6-chloro-N-[5-(2,2-difluoroéthyl)-4,6-diméthoxy-pyrimidin-2-yl]-7-phényl-1H-indole-3-sulfonamide ;
le N-[5-(2,2-difluoroéthoxy)-4,6-diméthoxy-pyrimidin-2-yl]-7-(2-pyrimidyl)-1H-indole-3-sulfonamide ;
le 6-chloro-N-[5-(2,2-difluoroéthoxy)-4,6-diméthoxy-pyrimidin-2-yl]-7-(3-méthylpyrazin-2-yl)-1H-indole-3-sulfonamide ;
le 6-chloro-N-[5-(2,2-difluoroéthyl)-4,6-diméthoxy-pyrimidin-2-yl]-7-(3-méthylpyrazin-2-yl)-1H-indole-3-sulfonamide ;
le N-(5-bromo-4,6-diméthoxy-pyrimidin-2-yl)-6-chloro-7-(2-pyrimidyl)-1H-indole-3-sulfonamide ;
le 6-chloro-N-[5-(2,2-difluoroéthyl)-4,6-diméthoxy-pyrimidin-2-yl]-7-(4-pyrimidyl)-1H-indole-3-sulfonamide ;
le 6-chloro-N-[5-(2,2-difluoroéthoxy)-4,6-diméthoxy-pyrimidin-2-yl]-7-(4-pyrimidyl)-1H-indole-3-sulfonamide ;
le 6-chloro-N-[5-(cyanométhoxy)-4,6-diméthoxy-pyrimidin-2-yl]-7-(2-pyrimidyl)-1H-indole-3-sulfonamide ;
le 6-chloro-N-[5-(2-cyanoéthyl)-4,6-diméthoxy-pyrimidin-2-yl]-7-(2-pyrimidyl)-1H-indole-3-sulfonamide ;
le 6-chloro-N-[5-(2-fluoroéthoxy)-4-méthoxy-pyrimidin-2-yl]-7-(2-pyrimidyl)-1H-indole-3-sulfonamide ;
le 6-chloro-N-[5-(difluorométhoxy)-4,6-diméthoxy-pyrimidin-2-yl]-7-(3-méthylpyrazin-2-yl)-1H-indole-3-sulfonamide ;
le 6-chloro-N-[5-(2,2-difluoroéthyl)-4,6-diméthoxy-pyrimidin-2-yl]-7-(5-méthylpyrazin-2-yl)-1H-indole-3-sulfonamide ;
le 6-chloro-N-[5-(difluorométhoxy)-4,6-diméthoxy-pyrimidin-2-yl]-7-(5-méthylpyrazin-2-yl)-1H-indole-3-sulfonamide ;
le 6-chloro-N-[5-(2,2-difluoroéthoxy)-4,6-diméthoxy-pyrimidin-2-yl]-7-(5-méthylpyrazin-2-yl)-1H-indole-3-sulfonamide ;
le 6-chloro-N-(4,6-diméthoxy-5-méthyl-pyrimidin-2-yl)-7-(2-pyrimidyl)-1H-indole-3-sulfonamide ;
le 6-chloro-N-[5-(2,2-difluoroéthyl)-4,6-diméthoxy-pyrimidin-2-yl]-7-(6-méthylpyrazin-2-yl)-1H-indole-3-sulfonamide ;
le 6-chloro-N-[5-(difluorométhoxy)-4,6-diméthoxy-pyrimidin-2-yl]-7-(6-méthylpyrazin-2-yl)-1H-indole-3-sulfonamide ;
le 6-chloro-N-[5-(2,2-difluoroéthoxy)-4,6-diméthoxy-pyrimidin-2-yl]-7-(6-méthylpyrazin-2-yl)-1H-indole-3-sulfonamide ;
le 6-chloro-N-[5-(2,2-difluoroéthoxy)-4,6-diméthoxy-pyrimidin-2-yl]-7-(5-pyrimidyl)-1H-indole-3-sulfonamide ;
le 6-chloro-7-(5-chloropyrazin-2-yl)-N-[5-(2,2-difluoroéthoxy)-4,6-diméthoxy-pyrimidin-2-yl]-1H-indole-3-sulfonamide ;
le 6-chloro-7-(5-chloropyrazin-2-yl)-N-[5-(2,2-difluoroéthyl)-4,6-diméthoxy-pyrimidin-2-yl]-1H-indole-3-sulfonamide ;
le 6-chloro-N-[5-(2,2-difluoroéthoxy)-4,6-diméthoxy-pyrimidin-2-yl]-7-(6-méthyl-2-pyridyl)-1H-indole-3-sulfonamide ;
le 6-chloro-N-[5-(2,2-difluoroéthyl)-4,6-diméthoxy-pyrimidin-2-yl]-7-(6-méthyl-2-pyridyl)-1H-indole-3-sulfonamide ;
le 6-chloro-N-[5-(difluorométhoxy)-4,6-diméthoxy-pyrimidin-2-yl]-7-(6-méthyl-2-pyridyl)-1H-indole-3-sulfonamide ;
le 6-chloro-N-[5-(2,2-difluoroéthoxy)-4,6-diméthoxy-pyrimidin-2-yl]-7-pyridazin-4-yl-1H-indole-3-sulfonamide ;
le 6-chloro-N-[5-(2,2-difluoroéthoxy)-4,6-diméthoxy-pyrimidin-2-yl]-7-(6-méthylpyrimidin-4-yl)-1H-indole-3-sulfonamide ;
le 6-chloro-N-[5-(2,2-difluoroéthyl)-4,6-diméthoxy-pyrimidin-2-yl]-7-(3-fluoropyrazin-2-yl)-1H-indole-3-sulfonamide ;
le 6-chloro-N-[5-(2,2-difluoroéthoxy)-4,6-diméthoxy-pyrimidin-2-yl]-7-(3-fluoropyrazin-2-yl)-1H-indole-3-sulfonamide ;
le 6-chloro-N-[5-(difluorométhoxy)-4,6-diméthoxy-pyrimidin-2-yl]-7-(3-fluoropyrazin-2-yl)-1H-indole-3-sulfonamide ;
le 6-chloro-N-[5-(2,2-difluoroéthoxy)-4,6-diméthoxy-pyrimidin-2-yl]-7-(3-méthyl-2-pyridyl)-1H-indole-3-sulfonamide ;
le 6-chloro-N-[5-(2,2-difluoroéthoxy)-4,6-diméthoxy-pyrimidin-2-yl]-7-(1-méthyltriazol-4-yl)-1H-indole-3-sulfonamide ;
le 6-chloro-N-[5-(2,2-difluoroéthyl)-4,6-diméthoxy-pyrimidin-2-yl]-7-(3-méthyl-2-pyridyl)-1H-indole-3-sulfonamide ;
le 6-chloro-N-[5-(difluorométhoxy)-4,6-diméthoxy-pyrimidin-2-yl]-7-(3-méthyl-2-pyridyl)-1H-indole-3-sulfonamide ;
le 6-chloro-N-[5-(2,2-difluoroéthoxy)-4,6-diméthoxy-pyrimidin-2-yl]-7-(1-méthyl-1,2,4-triazol-3-yl)-1H-indole-3-sulfonamide ;
le 6-chloro-N-[5-(2,2-difluoroéthoxy)-4,6-diméthoxy-pyrimidin-2-yl]-7-(5-fluoropyrimidin-2-yl)-1H-indole-3-sulfonamide ;
le 6-chloro-N-[5-(2,2-difluoroéthoxy)-4,6-diméthoxy-pyrimidin-2-yl]-7-(2-méthyltriazol-4-yl)-1H-indole-3-sulfonamide ;
le 6-chloro-N-[5-(difluorométhoxy)-4,6-diméthoxy-pyrimidin-2-yl]-7-(2-méthyltriazol-4-yl)-1H-indole-3-sulfonamide ;
le 6-chloro-N-[5-(2,2-difluoroéthyl)-4,6-diméthoxy-pyrimidin-2-yl]-7-(2-méthyltriazol-4-yl)-1H-indole-3-sulfonamide ;
le 6-chloro-N-[5-(2-fluoroéthoxy)-4,6-diméthoxy-pyrimidin-2-yl]-7-(2-méthyltriazol-4-yl)-1H-indole-3-sulfonamide ;
le 6-chloro-N-[5-(2,2-difluoroéthoxy)-4,6-diméthoxy-pyrimidin-2-yl]-7-(1,3,4-oxadiazol-2-yl)-1H-indole-3-sulfonamide ;
le 6-chloro-N-[5-(2,2-difluoroéthoxy)-4,6-diméthoxy-pyrimidin-2-yl]-7-(1-méthylpyrazol-4-yl)-1H-indole-3-sulfonamide ;
le 6-chloro-N-[5-(2-fluoroéthoxy)-4,6-diméthoxy-pyrimidin-2-yl]-7-(1-méthylpyrazol-4-yl)-1H-indole-3-sulfonamide ;
le 6-chloro-N-[5-(difluorométhoxy)-4,6-diméthoxy-pyrimidin-2-yl]-7-(1-méthylpyrazol-4-yl)-1H-indole-3-sulfonamide ;
le 6-chloro-N-[5-(2,2-difluoroéthyl)-4,6-diméthoxy-pyrimidin-2-yl]-7-(1-méthylpyrazol-4-yl)-1H-indole-3-sulfonamide ;
le 6-chloro-N-[5-(2,2-difluoroéthoxy)-4,6-diméthoxy-pyrimidin-2-yl]-7-thiazol-4-yl-1H-indole-3-sulfonamide ;
le 6-chloro-N-[5-(2,2-difluoroéthoxy)-4,6-diméthoxy-pyrimidin-2-yl]-7-oxazol-5-yl-1H-indole-3-sulfonamide ;
le 6-chloro-N-[5-(difluorométhoxy)-4,6-diméthoxy-pyrimidin-2-yl]-7-oxazol-5-yl-1H-indole-3-sulfonamide ;
le 6-chloro-N-[5-(2,2-difluoroéthoxy)-4,6-diméthoxy-pyrimidin-2-yl]-7-isoxazol-4-yl-1H-indole-3-sulfonamide ;
le 6-chloro-N-[5-(difluorométhoxy)-4,6-diméthoxy-pyrimidin-2-yl]-7-isoxazol-4-yl-1H-indole-3-sulfonamide ;
le 6-chloro-N-[5-(difluorométhoxy)-4,6-diméthoxy-pyrimidin-2-yl]-7-thiazol-4-yl-1H-indole-3-sulfonamide ;
le N-[5-(2,2-difluoroéthyl)-4,6-diméthoxy-pyrimidin-2-yl]-7-(6,7-dihydro-5H-pyrrol[1,2-c]imidazol-3-yl)-1H-indole-3-sulfonamide ; et
des sels pharmaceutiquement acceptables de ceux-ci.

11. Procédé de préparation d'un composé selon l'une quelconque des revendications 1 à 10 comprenant la réaction d'un composé de formule III avec un composé de formule II en présence d'une base choisie parmi la N-éthyldiisopropylamine, la pyridine, le phosphate de potassium ou l'hydrure de sodium, pour fournir un composé de formule I, 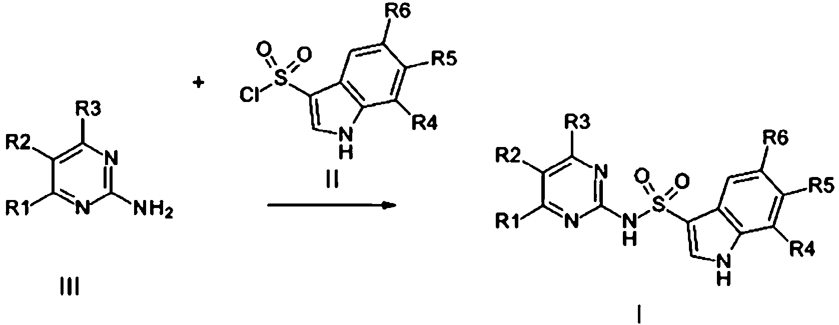 dans lequel R¹, R², R³, R³, R⁴, R⁵ et R⁶ sont tels que décrits ci-dessus.

12. Composé selon l'une quelconque des revendications 1 à 10 pour une utilisation comme substance thérapeutiquement active.

13. Composition pharmaceutique comprenant un composé selon l'une quelconque des revendications 1 à 10 et un véhicule thérapeutiquement inerte.

14. Composé selon l'une quelconque des revendications 1 à 10 pour une utilisation dans le traitement ou la prophylaxie d'affections résultant d'une lésion directe de gaines de myéline (notamment mais sans s'y limiter une myélinolyse centropontine et extrapontine, un empoisonnement au monoxyde de carbone, une carence nutritionnelle et une démyélinisation induite par un virus), des troubles de démyélinisation (notamment mais sans s'y limiter une sclérose en plaques, une encéphalomyélite disséminée aigüe et multiphasique, des troubles du spectre de la neuromyélite optique et des leucodystrophies), des troubles du SNC associés à une perte de myéline (notamment mais sans s'y limiter la maladie d'Alzheimer, une schizophrénie, la maladie de Parkinson, la maladie d'Huntington, une sclérose latérale amyotrophique et une ischémie due à un accident vasculaire cérébral) et une inflammation dans le SNC, par exemple suite à une encéphalite, une angéite primaire, une méningite et une obésité.

15. Composé selon l'une quelconque des revendications 1 à 10 pour une utilisation dans le traitement ou la prophylaxie d'une sclérose en plaques.
